(19) Europäisches Patentamt
European Patent Office
Office européen des brevets

(11) Veröffentlichungsnummer: **0 330 959**
**A2**

(12) # EUROPÄISCHE PATENTANMELDUNG

(21) Anmeldenummer: **89102926.6**

(22) Anmeldetag: **20.02.89**

(51) Int. Cl.[4]: **C07D 403/04 , C07D 401/04 , C07D 403/14 , C07D 409/14 , C07D 417/14 , A01N 43/48 , A01N 43/64**

(30) Priorität: **04.03.88 DE 3807034**
**29.07.88 DE 3825867**

(43) Veröffentlichungstag der Anmeldung:
**06.09.89 Patentblatt 89/36**

(84) Benannte Vertragsstaaten:
**BE CH DE ES FR GB IT LI NL**

(71) Anmelder: **BAYER AG**

**D-5090 Leverkusen 1 Bayerwerk(DE)**

(72) Erfinder: **Kirsten, Rolf, Dr.**
**Carl-Langhans-Strasse 27**
**D-4019 Monheim(DE)**
Erfinder: **Kluth, Joachim, Dr.**
**Kurt-Schumacher-Strasse 9**
**D-4018 Langenfeld(DE)**
Erfinder: **Fest, Christa, Dr.**
**Im Johannistal 20**
**D-5600 Wuppertal 1(DE)**
Erfinder: **Gesing, Ernst, Dr.**
**Trillser Graben 4**
**D-4006 Erkrath-Hochdahl(DE)**
Erfinder: **Müller, Klaus-Helmut, Dr.**
**Bockhackstrasse 55**
**D-4000 Düsseldorf 13(DE)**
Erfinder: **Riebel, Hans-Jochem, Dr.**
**In der Beek 92**
**D-5600 Wuppertal 1(DE)**
Erfinder: **Babczinski, Peter, Dr.**
**In der Lohrenbeck 11**
**D-5600 Wuppertal 1(DE)**
Erfinder: **Schallner, Otto, Dr.**
**Noldeweg 22**
**D-4019 Monheim(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6A**
**D-4000 Düsseldorf 31(DE)**

(54) **Substituierte Sulfonylaminoazole.**

(57) Die Erfindung betrifft neuartige substituierte Sulfonylaminoazole der allgemeinen Formel (I)

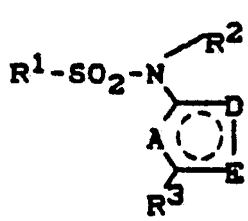

(I)

ein Verfahren zu ihrer Herstellung und ihre Verwendung als Herbizide.
Die allgemeine Formel (I) repräsentiert die möglichen Isomeren der Formeln (IA), (IB) und (IC).

EP 0 330 959 A2

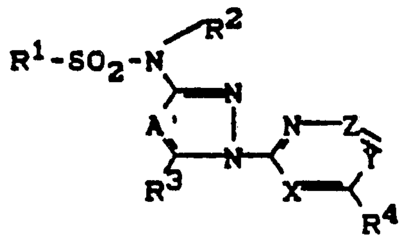

(IA)

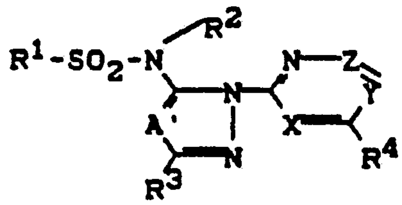

(IB)

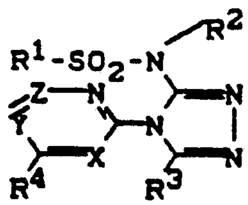

(IC)

sowie Gemische dieser Isomeren.

(In den obigen Formeln haben die variablen Reste $R^1$, $R^2$, $R^3$, $R^4$, A bzw. A', D, E, X, Y und Z die in der Beschreibung angegebenen Bedeutungen).

## Substituierte Sulfonylaminoazole

Die Erfindung betrifft neuartige substituierte Sulfonylaminoazole, Verfahren und neue Zwischenprodukte zu ihrer Herstellung sowie ihre Verwendung als Herbizide.

Es ist bekannt, daß 3-Amino-1,2,4-triazol (Amitrol) als Herbizid verwendet werden kann (vgl. Science 145 (1964), 97). Die Wirkung dieser Verbindung ist jedoch nicht in allen Belangen zufriedenstellend.

Es wurden nun die neuen substituierten Sulfonylaminoazole der allgemeinen Formel (I)

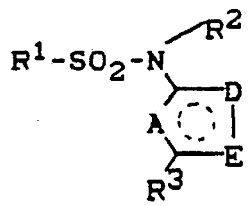

(I)

in welcher

$R^1$ für einen gegebenenfalls substituierten Rest aus der Reihe Alkyl, Aralkyl, Aryl und Heteroaryl steht,

$R^2$ für Wasserstoff oder die Gruppierung $-SO_2-R^1$ steht, worin

$R^1$ die oben angegebene Bedeutung hat,

$R^3$ für Wasserstoff, Halogen, Hydroxy, Mercapto, Amino oder einen gegebenenfalls substituierten Rest aus der Reihe Alkyl, Alkoxy, Alkylthio, Alkylamino und Dialkylamino steht,

A für eine CH-Gruppierung, für Stickstoff oder die Gruppierung

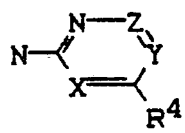

steht,

D für Stickstoff oder die Gruppierung

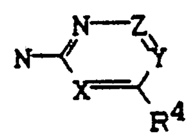

steht,

E für Stickstoff oder die Gruppierung

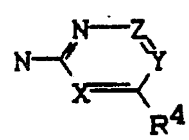

steht,

wobei jeweils immer einer der Reste A, D oder E für die Gruppierung

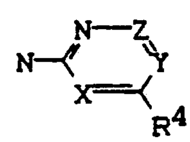

steht, worin
$R^4$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^5$-Gruppierung steht, worin
$R^5$ für Wasserstoff, Halogen, Cyano, Alkyl, Formyl, Alkylcarbonyl oder Alkoxycarbonyl steht, und
Z für Stickstoff oder eine $CR^6$-Gruppierung steht, worin
$R^6$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
sowie Salze von Verbindungen der Formel (I) gefunden.

Die allgemeine Formel (I) steht für die möglichen Isomeren der Formeln (IA), (IB) und (IC)

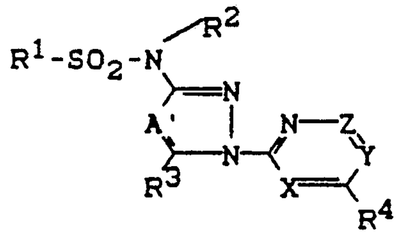

(IA)

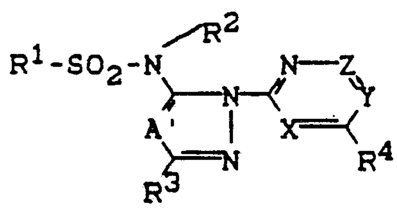

(IB)

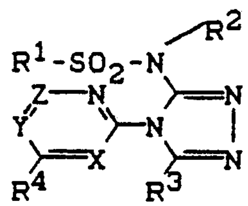

(IC)

in welchen
$R^1$, $R^2$, $R^3$, $R^4$, X, Y und Z die oben angegebenen Bedeutungen haben und
A′ für Stickstoff oder eine CH-Gruppierung steht,
sowie für Gemische dieser Isomeren.

Man erhält die neuen substituierten Sulfonylaminoazole der allgemeinen Formel (I), wenn man
(a) substituierte Aminoazole der allgemeinen Formel (II)

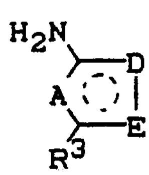

(II)

in welcher
A, D, E und $R^3$ die oben angegebenen Bedeutungen haben,
mit Sulfonsäurehalogeniden oder Sulfonsäureanhydriden der allgemeinen Formel (III)
$R^1 - SO_2 - Q$ (III)
in welcher
$R^1$ die oben angegebene Bedeutung hat und
Q für Fluor, Chlor, Brom oder die Gruppierung $-O-SO_2-R^1$ steht, worin
$R^1$ die oben angegebene Bedeutung hat,
gegebenenfalls in Gegenwart eines Säureakzeptors, gegebenenfalls in Gegenwart eines Katalysators und

gegebenenfalls in Gegenwart eines Verdünnungsmittels umsetzt, und
gegebenenfalls die so erhältlichen Verbindungen der Formel (I), in welcher
$R^2$ für die Gruppierung $-SO_2-R^1$ steht, mit Desulfonylierungsmitteln,
gegebenenfalls in Gegenwart von Verdünnungsmitteln, zu Verbindungen der Formel (I), in welcher
$R^2$ für Wasserstoff steht,
umsetzt, oder wenn man
(b) sulfonylierte Aminoguanidine der allgemeinen Formeln (IVA) oder (IVB)

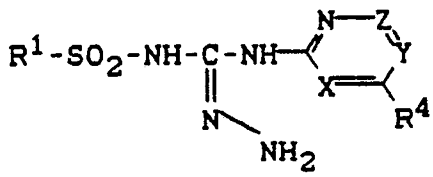

(IVA)

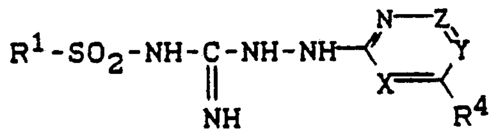

(IVB)

in welchen
$R^1$, $R^4$, X, Y und Z die oben angegebenen Bedeutungen haben,
oder Tautomere zu den Verbindungen der Formeln (IVA) und (IVB) oder Gemische aus den Verbindungen der Formeln (IVA) bzw. (IVB) und den jeweils möglichen Tautomeren mit Ester(amide)n der allgemeinen Formel (V) (d.h. Orthoestern bzw. Amid-acetalen)

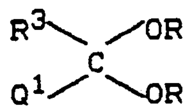

(V)

in welcher
$R^3$ die oben angegebene Bedeutung hat,
R für Alkyl steht und
$Q^1$ für Alkoxy oder Dialkylamino steht,
gegebenenfalls in Gegenwart eines Verdünnungsmittels umsetzt, oder wenn man
(c) substituierte Aminopyrazoline der allgemeinen Formel (VI)

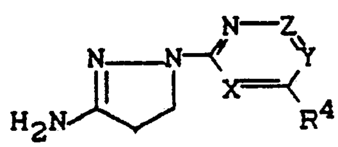

(VI)

in welcher
$R^4$, X, Y und Z die oben angegebenn Bedeutungen haben,
mit Sulfonsäurehalogeniden oder Sulfonsäureanhydriden der allgemeinen Formel (III)
$R^1 - SO_2 - Q$ (III)
in welcher
$R^1$ die oben angegebene Bedeutung hat und
Q für Fluor, Chlor, Brom oder die Gruppierung $-O-SO_2-R^1$ steht, worin
$R^1$ die oben angegebene Bedeutung hat,
in Gegenwart von (Luft)Sauerstoff, gegebenenfalls in Gegenwart eines Säureakzeptors und gegebenenfalls in Gegenwart eines Verdünnungsmittels umsetzt, und
gegebenenfalls die so erhältlichen Verbindungen der Formel (I), in welcher
$R^2$ für die Gruppierung $-SO_2-R^1$ steht,

mit Desulfonylierungsmitteln, gegebenenfalls in Gegenwart von Verdünnungsmitteln, zu Verbindungen der Formel (I), in welcher
$R^2$ für Wasserstoff steht,
umsetzt und gegebenenfalls die nach den Verfahren (a), (b) oder (c) erhaltenen Produkte nach üblichen Methoden in Salze überführt.

Ein Teil der erfindungsgemäßen Verbindungen der Formel (I) kann auch wie nachstehend skizziert erhalten werden ($R^1$, $R^4$, X, Y, Z wie oben definiert; $Q^2$ z. B. $SCH_3$ oder $OC_6H_5$):

(d)

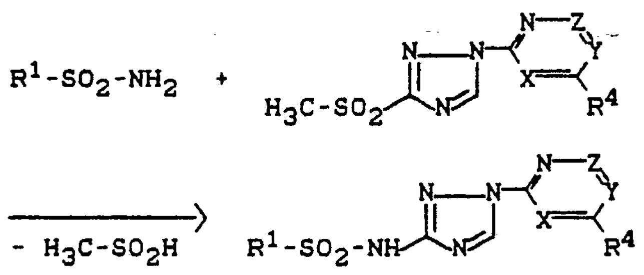


(e)

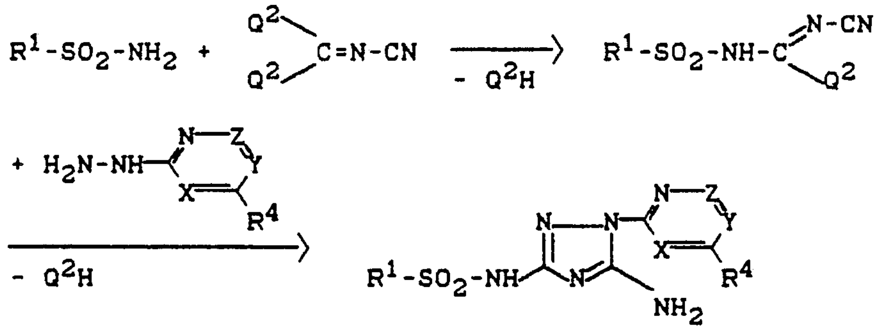


Die neuen substituierten Sulfonylaminoazole der allgemeinen Formel (I) zeichnen sich durch starke herbizide Wirksamkeit aus. Diese Verbindungen stellen eine chemisch neuartige Klasse von Herbiziden dar. Überraschenderweise zeigen die neuen Verbindungen der Formel (I) erheblich bessere herbizide Wirkung als das strukturell ähnliche bekannte Herbizid Aminotriazol (Amitrol).

Gegenstand der Erfindung sind vorzugsweise Verbindungen der Formel (I), in welcher
$R^1$ für den Rest

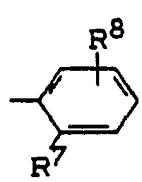

steht, worin
$R^7$ und $R^8$ gleich oder verschieden sind und für Wasserstoff, Fluor, Chlor, Brom, Iod, Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)aminocarbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$--alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$- $C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom,

Cyano oder $C_1$-$C_4$-Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxy-carbonyl substituiert ist], $C_3$-$C_6$-Alkinyloxy, $C_3$-$C_6$-Alkinylthio oder für den Rest -$S(O)_p$-$R^9$ stehen, wobei

p für die Zahlen 1 oder 2 steht und

$R^9$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest -NHOR$^{10}$ steht, wobei

$R^{10}$ für $C_1$-$C_6$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^7$ und $R^8$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest -CO-$R^{11}$ stehen, wobei

$R^{11}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$ Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^7$ und $R^8$ weiterhin für $C_1$-$C_4$-Alkylsulfonyloxy, Di-($C_1$-$C_4$-alkyl)-aminosulfonylamino oder für den Rest -CH = N-$R^{12}$ stehen, wobei

$R^{12}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht, worin weiter

$R^1$ für den Rest

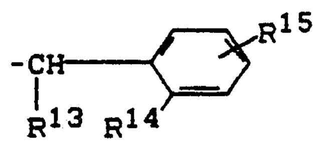

steht, worin

$R^{13}$ für Wasserstoff oder $C_1$-$C_4$-Alkyl steht,

$R^{14}$ und $R^{15}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^1$ für den Rest

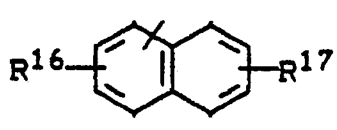

steht, worin

$R^{16}$ und $R^{17}$ gleich oder verschieden sind und für Wasserstoff, Fluor, Chlor, Brom, Ni tro, Cyano, $C_1$-$C_4$-Alkyl [welches gegebenenfalls durch Fluor und/oder Chlor substituiert ist] oder $C_1$-$C_4$-Alkoxy [welches gegebenenfalls durch fluor und/oder Chlor substituiert ist], stehen; worin weiter

$R^1$ für den Rest

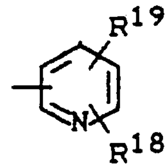

steht, worin
$R^{18}$ und $R^{19}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_2$-$C_4$-Alkenyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl, $C_1$-$C_4$-Alkoxy-carbonyl, Dimethylaminocarbonyl oder Dioxolanyl stehen; worin weiter
$R^1$ für den Rest

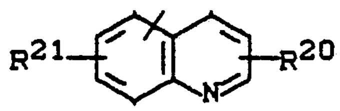

steht, worin
$R^{20}$ und $R^{21}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; worin weiter
$R^1$ für den Rest

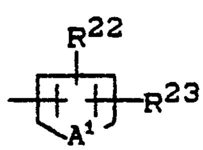

steht, worin
$R^{22}$ und $R^{23}$ gleich oder verschieden sind und für Wasserstoff, Fluor, Chlor, Brom, Cyano, Nitro, $C_1$-$C_4$-Alkyl [welches gegebenenfalls durch Fluor, Chlor, $C_1$-$C_4$-Alkoxy und/oder $C_1$-$C_4$-Halogenalkoxy substituiert ist], $C_1$-$C_4$-Alkoxy [welches gegebenenfalls durch Fluor und/ oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-amino-sulfonyl oder $C_1$-$C_4$-Alkoxy-carbonyl stehen, und $A^1$ für Sauerstoff, Schwefel oder die Gruppierung N-$Z^1$ steht, wobei
$Z^1$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter
$R^1$ für den Rest

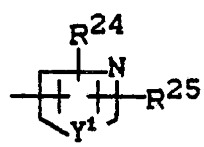

steht, worin
$R^{24}$ und $R^{25}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,
$Y^1$ für Schwefel oder die Gruppierung N-$R^{26}$ steht, wobei
$R^{26}$ für Wasserstoff oder $C_1$-$C_4$-Alkyl steht; worin weiter

$R^1$ für den Rest

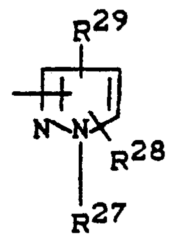

steht, worin
$R^{27}$ für Wasserstoff, $C_1$-$C_4$-Alkyl, Phenyl oder (Iso)Chinolinyl steht,
$R^{28}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Dioxolanyl oder $C_1$-$C_4$-Alkoxy-carbonyl steht und
$R^{29}$ für Wasserstoff, Halogen oder $C_1$-$C_4$-Alkyl steht; worin weiter
$R^1$ für den Rest

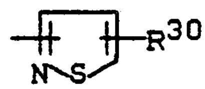

steht, worin
$R^{30}$ für Wasserstoff, Halogen, $C_1$-$C_4$-Alkyl oder $C_1$-$C_4$-Alkoxy-carbonyl steht; worin weiter
$R^1$ für den Rest

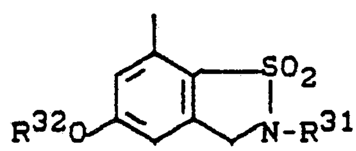

steht, worin
$R^{31}$ für $C_1$-$C_4$-Alkyl steht und
$R^{32}$ für $C_1$-$C_4$-Alkyl steht, worin weiter
$R^1$ für den Rest

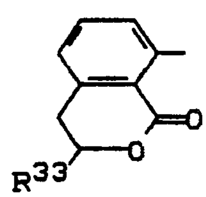

steht, worin
$R^{33}$ für Wasserstoff oder Methyl steht;
in welcher weiter
$R^2$ für Wasserstoff oder für die Gruppierung $-SO_2-R^1$ steht, worin
$R^1$ die oben vorzugsweise angegebene Bedeutung hat;
in welcher weiter
$R^3$ für Wasserstoff, Fluor, Chlor, Brom, Iod, Hydroxy, Mercapto, Amino oder einen gegebenenfalls durch Fluor und/oder Chlor substituierten Rest aus der Reihe $C_1$-$C_4$-Alkyl, $C_1$-$C_4$-Alkoxy, $C_1$-$C_4$-Alkylthio, $C_1$-$C_4$-Alkylamino und Di-($C_1$-$C_4$-alkyl)-amino steht,
A für Stickstoff, eine -CH-Gruppierung oder die Gruppierung

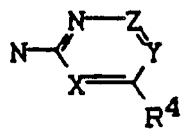

steht,
D für Stickstoff oder die Gruppierung

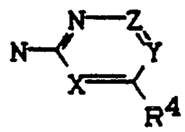

steht,
E für Stickstoff oder die Gruppierung

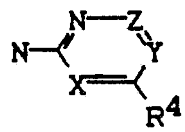

steht,
wobei jeweils immer einer der Reste A, D oder E für die Gruppierung

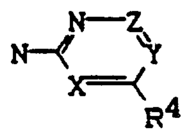

steht, worin
$R^4$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,
X für Stickstoff oder eine CH-Gruppierung steht,
Y für Stickstoff oder eine $CR^5$-Gruppierung steht, worin
$R^5$ für Wasserstoff, Fluor, Chlor, Brom, Cyano, Methyl, Formyl, Acetyl, Methoxycarbonyl oder Ethoxycarbonyl steht und
Z für Stickstoff oder eine $CR^6$-Gruppierung steht, worin
$R^6$ für Wasserstoff, Fluor, Chlor, Brom, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$- Alkylamino, Dimethylamino oder Diethylamino steht.

Gegenstand der Erfindung sind insbesondere Verbindungen der Formel (I), in welcher
$R^1$ für den Rest

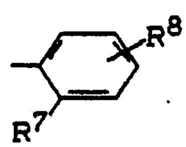

steht worin
$R^7$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und
$R^8$ für Wasserstoff, Fluor oder Chlor steht; worin weiter
$R^1$ für den Rest

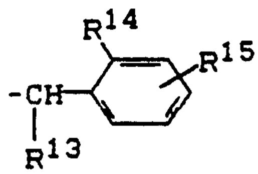

steht, worin
$R^{13}$ für Wasserstoff steht,
$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
$R^{15}$ für Wasserstoff, Fluor oder Chlor steht; worin weiter
$R^1$ für den Rest

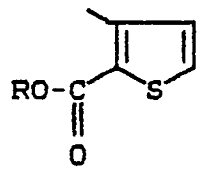

steht, worin
R für $C_1$-$C_4$-Alkyl steht, oder
$R^1$ für den Rest

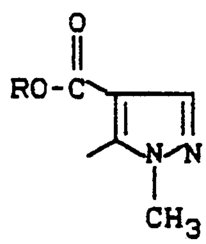

steht, worin
R für $C_1$-$C_4$-Alkyl steht, oder
$R^1$ für den Rest

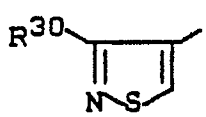

steht, worin
$R^{30}$ für Wasserstoff, Chlor, Methyl, Ethyl, Methoxycarbonyl oder Ethoxycarbonyl steht;
in welcher weiter
$R^2$ für Wasserstoff oder für die Gruppierung $-SO_2-R^1$ steht, worin
$R^1$ die oben als insbesondere bevorzugt angegebene Bedeutung hat,
$R^3$ für Wasserstoff, Methyl, Methoxy oder Methylthio steht,
A für Stickstoff, eine -CH-Gruppierung oder die Gruppierung

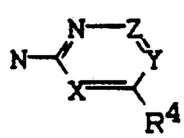

steht,
D für Stickstoff oder die Gruppierung

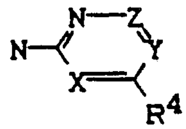

steht,
E für Stickstoff oder die Gruppierung

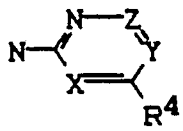

steht,
wobei jeweils immer einer der Reste A, D oder E für die Gruppierung

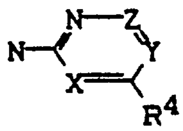

steht, worin
$R^4$ für Wasserstoff, Fluor, Chlor, Brom, Methyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine CH-Gruppierung steht,
Y für Stickstoff oder eine $CR^5$-Gruppierung steht, worin
$R^5$ für Wasserstoff, Fluor, Chlor oder Methyl steht, und
Z für Stickstoff oder eine $CR^6$-Gruppierung steht, worin
$R^6$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

Ganz besonders bevorzugt werden die Verbindungen der allgemeinen Formel (IA) - oben - in welcher $R^1$, $R^2$, $R^3$, $R^4$, X, Y und Z diejenigen Bedeutungen haben, die im Zusammenhang mit der Beschreibung der erfindungsgemäßen Verbindungen der Formel (I) als insbesondere bevorzugt für $R^1$, $R^2$, $R^3$, $R^4$, X, Y und Z angegeben wurden. $A'$ steht insbesondere bevorzugt für Stickstoff oder eine CH-Gruppierung.

Weiterhin werden besonders bevorzugt die Verbindungen der allgemeinen Formel (IB) - oben - in welcher $R^1$, $R^2$, $R^3$, $R^4$, X, Y und Z diejenigen Bedeutungen haben, die im Zusammenhang mit der Beschreibung der erfindungsgemäßen Verbindungen der Formel (I) als insbesondere bevorzugt für $R^1$, $R^2$, $R^3$, $R^4$, X, Y und Z angegeben wurden. $A'$ steht insbesondere bevorzugt für Stickstoff oder eine CH-Gruppierung.

Außerdem werden besonders bevorzugt die Verbindungen der allgemeinen Formel (IC) - oben - in welcher $R^1$, $R^2$, $R^3$, $R^4$, X, Y und Z diejenigen Bedeutungen haben, die im Zusammenhang mit der Beschreibung der erfindungsgemäßen Verbindungen der Formel (I) als insbesondere bevorzugt für $R^1$, $R^2$, $R^3$, $R^4$, X, Y und Z angegeben wurden.

Die Erfindung betrifft weiter vorzugsweise Salze von Verbindungen der Formel (I)

α) mit Protonensäuren, wie z. B. Salzsäure, Schwefelsäure, Phosphorsäure, Methansulfonsäure, Benzol-oder p-Toluolsulfonsäure, oder Naphthalin-mono-oder -di-sulfonsäuren, oder

β) mit Basen, wie z. B. Natrium-, Kalium- oder Calcium-hydroxid, -hydrid, -amid oder carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

Verwendet man beispielsweise 5-Amino-3-methyl-1-(4,6-dimethoxy-1,3,5-triazin-2-yl)-1,2,4-triazol und 2-Fluorbenzol-sulfonsäurechlorid (2 Moläquivalente) als Aus gangsstoffe, so kann der Reaktionsablauf beim erfindungsgemäßen Verfahren (a) durch folgendes Formelschema skizziert werden:

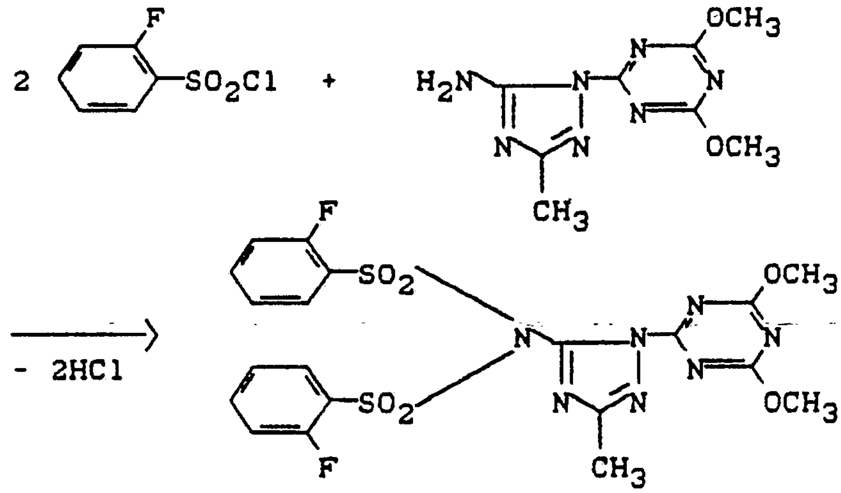


Verwendet man beispielsweise N′-(4,6-Dimethoxy-pyrimidin-2-yl-amino)-N″-(2-methoxycarbonyl-benzylsulfonyl)-guanidin und Orthoameisensäuretrimethylester als Ausgangsstoffe, so kann der Reaktionsablauf beim erfindungsgemäßen Verfahren (b) durch folgendes Formelschema skizziert werden:

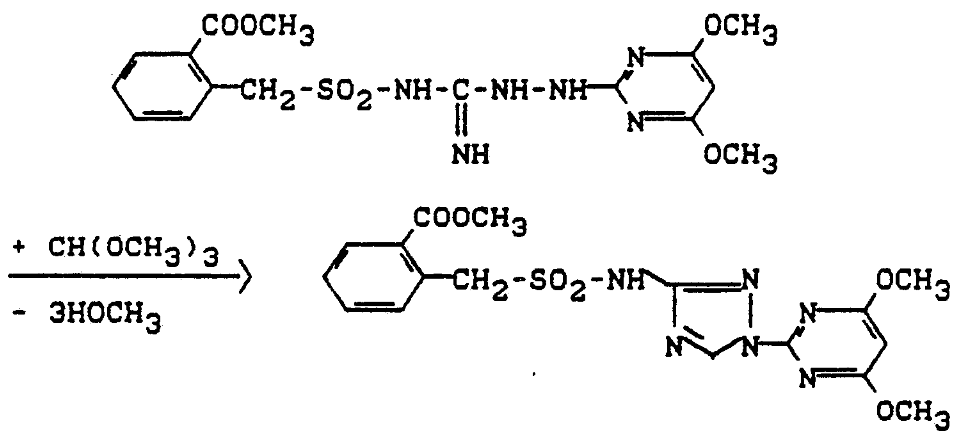


Verwendet man beispielsweise 3-Amino-1-(4,6-dimethylpyrimidin-2-yl)-2-pyrazolin und 2-Methylsulfonylbenzolsulfonsäurechlorid als Ausgangsstoffe, so kann bei Durchführung unter (Luft)Sauerstoff-Atmosphäre der Reaktionsverlauf beim erfindungsgemäßen Verfahren (c) durch folgendes Formelschema skizziert werden:

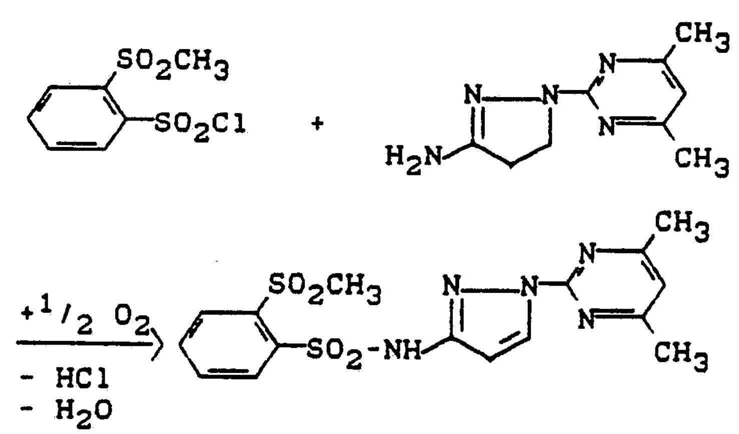


Die beim erfindungsgemäßen Verfahren (a) zur Herstellung von Verbindungen der Formel (I) als

Ausgangsstoffe zu verwendenden substituierten Aminoazole sind durch die Formel (II) allgemein definiert.

In Formel (II) haben A, D, E und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, D, E und $R^3$ angegeben wurden.

Die allgemeine Formel (II) steht für die möglichen Isomeren der Formeln (IIA), (IIB) und (IIC)

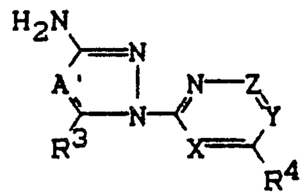

(IIA)

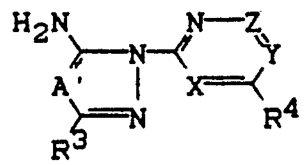

(IIB)

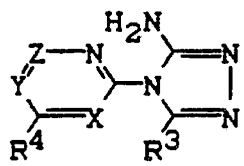

(IIC)

sowie für Gemische dieser Isomeren.

In den Formeln (IIA), (IIB) und (IIC) haben $A'$, $R^3$, $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A'$, $R^3$, $R^4$, X, Y und Z angegeben wurden.

Beispiele für die Ausgangsstoffe der Formeln (IIA), (IIB) und (IIC) - und damit auch für die Verbindungen der Formel (II) - sind in der nachstehenden Tabelle 1 aufgeführt. Die jeweils zeilenweise für einzelne Beispiele angegebenen Bedeutungen der Variablen A, $R^3$, $R^4$, X, Y und Z stehen also in jedem Einzelfall sowohl für die Isomeren der Formel (IIA), (IIB) als auch für die Isomeren der Formel (IIC).

Tabelle 1: Beispiele für die Ausgangsstoffe der Formeln (IIA), (IIB) und (IIC)

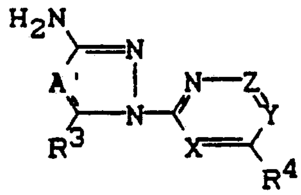

(IIA)

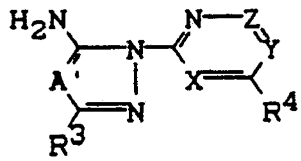

(IIB)

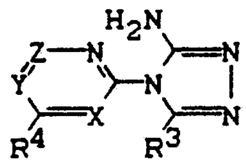

(IIC)

| A bzw. A' | $R^3$ | $R^4$ | X | Y | Z |
|---|---|---|---|---|---|
| N | H | $CH_3$ | N | CH | $C-CH_3$ |
| N | H | $CH_3$ | N | CH | $C-OCH_3$ |
| N | H | $CH_3$ | N | CH | $C-OC_2H_5$ |
| N | H | $OCH_3$ | N | CH | $C-OCH_3$ |
| N | H | $OCH_3$ | N | CH | C-Cl |
| N | H | H | N | CH | $C-CH_3$ |
| N | H | $CF_3$ | N | CH | $C-OCH_3$ |
| N | H | $OCH_3$ | N | CH | $C-OCHF_2$ |
| N | H | $CH_3$ | N | CH | $C-OCHF_2$ |
| N | H | $OCHF_2$ | N | CH | $C-OCHF_2$ |
| N | H | $CH_3$ | N | N | $C-CH_3$ |

Tabelle 1 - Fortsetzung

| A bzw. A' | $R^3$ | $R^4$ | X | Y | Z |
|---|---|---|---|---|---|
| N | H | $CH_3$ | N | N | $C-OCH_3$ |
| N | H | $OCH_3$ | N | N | $C-OCH_3$ |
| N | H | $OCH_3$ | N | N | C-Cl |
| N | H | $C_2H_5$ | N | CH | $C-OCH_3$ |
| N | H | $C_2H_5$ | N | N | $C-OCH_3$ |
| N | $CH_3$ | $CH_3$ | N | CH | $C-CH_3$ |
| N | $CH_3$ | $CH_3$ | N | CH | $C-OCH_3$ |
| N | $CH_3$ | $OCH_3$ | N | CH | $C-OCH_3$ |
| N | $CH_3$ | $OCH_3$ | N | CH | C-Cl |
| N | $CH_3$ | H | N | CH | $C-CH_3$ |
| N | $CH_3$ | $CF_3$ | N | CH | $C-OCH_3$ |
| N | $CH_3$ | $OCH_3$ | N | CH | $C-OCHF_2$ |
| N | $CH_3$ | $CH_3$ | N | CH | $C-OCHF_2$ |
| N | $CH_3$ | $CH_3$ | N | N | $C-CH_3$ |
| N | $CH_3$ | $CH_3$ | N | N | $C-OCH_3$ |
| N | $CH_3$ | $OCH_3$ | N | N | $C-OCH_3$ |
| N | $CH_3$ | $C_2H_5$ | N | CH | $C-OCH_3$ |
| N | $CH_3$ | $CH_3$ | N | N | $C-OC_2H_5$ |
| N | $CH_3$ | $C_2H_5$ | N | N | $C-OCH_3$ |
| N | $CH_3$ | $CH_3$ | N | N | C-Cl |
| N | $CH_3$ | $CH_3$ | CH | N | $C-CH_3$ |

Tabelle 1 - Fortsetzung

| A bzw. A' | $R^3$ | $R^4$ | X | Y | Z |
|---|---|---|---|---|---|
| N | $CH_3$ | $OCH_3$ | CH | N | $C-OCH_3$ |
| N | $CH_3$ | $CH_3$ | N | CH | $C-SCH_3$ |
| N | H | $CH_3$ | N | CH | $C-N(CH_3)_2$ |
| N | H | $OCH_3$ | N | CH | $C-SCH_3$ |
| N | H | $OCH_3$ | N | N | $C-NHC_2H_5$ |
| N | H | $OC_2H_5$ | N | N | $C-NHCH_3$ |
| N | H | $CH_3$ | CH | CH | $C-CH_3$ |
| N | $OCH_3$ | $CH_3$ | N | CH | $C-CH_3$ |
| N | $OCH_3$ | $CH_3$ | N | CH | $C-OCH_3$ |
| N | $OCH_3$ | $OCH_3$ | N | CH | $C-OCH_3$ |
| N | $OCH_3$ | $OCH_3$ | N | CH | C-Cl |
| N | $OCH_3$ | H | N | CH | $C-CH_3$ |
| N | $OCH_3$ | $CF_3$ | N | CH | $C-OCH_3$ |
| N | $OCHF_2$ | $OCH_3$ | N | CH | $C-OCHF_2$ |
| N | $OCH_3$ | $CH_3$ | N | CH | $C-OCHF_2$ |
| N | $OCH_3$ | $CH_3$ | N | N | $C-CH_3$ |
| N | $OCH_3$ | $CH_3$ | N | N | $C-OCH_3$ |
| N | $OCH_3$ | $OCH_3$ | N | N | $C-OCH_3$ |
| N | $OCH_3$ | $C_2H_5$ | N | CH | $C-OCH_3$ |
| N | $OCH_3$ | $C_2H_5$ | N | N | $C-OCH_3$ |
| N | $SCH_3$ | $CH_3$ | N | CH | $C-CH_3$ |
| N | $OCH_3$ | $CH_3$ | N | N | $C-OC_2H_5$ |

Tabelle 1 - Fortsetzung

| A bzw. A' | $R^3$ | $R^4$ | X | Y | Z |
|---|---|---|---|---|---|
| N | $SCH_3$ | $CH_3$ | N | CH | $C-OCH_3$ |
| N | $SCH_3$ | $CH_3$ | N | CH | $C-OC_2H_5$ |
| N | $SCH_3$ | $OCH_3$ | N | CH | $C-OCH_3$ |
| N | $SCH_3$ | $OCH_3$ | N | CH | C-Cl |
| N | $SCH_3$ | H | N | CH | $C-CH_3$ |
| N | $SCH_3$ | $CF_3$ | N | CH | $C-OCH_3$ |
| N | $SCH_3$ | $OCH_3$ | N | CH | $C-OCHF_2$ |
| N | $SCH_3$ | $CH_3$ | N | CH | $C-OCHF_2$ |
| N | $SCH_3$ | $OCHF_2$ | N | CH | $C-OCHF_2$ |
| N | $SCH_3$ | $CH_3$ | N | N | $C-CH_3$ |
| N | $SCH_3$ | $CH_3$ | N | N | $C-OCH_3$ |
| N | $SCH_3$ | $OCH_3$ | N | N | $C-OCH_3$ |
| N | $SCH_3$ | $OCH_3$ | N | N | C-Cl |
| N | $SCH_3$ | $C_2H_5$ | N | CH | $C-OCH_3$ |
| N | $SCH_3$ | $C_2H_5$ | N | N | $C-OCH_3$ |
| CH | H | $CH_3$ | N | CH | $C-CH_3$ |
| CH | H | $CH_3$ | N | CH | $C-OCH_3$ |
| CH | H | $OCH_3$ | N | CH | $C-OCH_3$ |
| CH | H | $OCH_3$ | N | CH | C-Cl |
| CH | H | $CF_3$ | N | CH | $C-OCH_3$ |
| CH | H | $OCH_3$ | N | CH | $C-OCHF_2$ |

Tabelle 1 - Fortsetzung

| A bzw. A' | $R^3$ | $R^4$ | X | Y | Z |
|---|---|---|---|---|---|
| CH | H | $CH_3$ | N | CH | $C-OCHF_2$ |
| CH | H | $OCHF_2$ | N | CH | $C-OCHF_2$ |
| CH | H | $CH_3$ | N | N | $C-CH_3$ |
| CH | H | $CH_3$ | N | N | $C-OCH_3$ |
| CH | H | $OCH_3$ | N | N | C-Cl |
| CH | H | $C_2H_5$ | N | CH | $C-OCH_3$ |
| CH | H | $C_2H_5$ | N | N | $C-OCH_3$ |

Die Ausgangsstoffe der Formel (II) sind - wie auch diejenigen der Formel (IIA), (IIB) und (IIC) - weitgehend noch nicht aus der Literatur bekannt. Neu sind insbesondere die Verbindungen der Formel (II) sowie der Formeln (IIA), (IIB) und (IIC) bei denen A bzw. A′ für Stickstoff steht.

Man erhält die Verbindungen der Formel (II) - im allgemeinen als Gemische unterschiedlicher Zusammensetzung von Verbindungen der Formeln (IIA), (IIB) und (IIC) -wenn man

α) Aminoazole der allgemeinen Formel (VII)

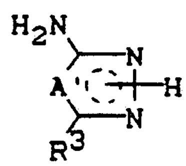

(VII)

in welcher

A′ und $R^3$ die oben angegebenen Bedeutungen haben,

mit Azinen der allgemeinen Formel (VIII)

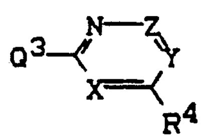

(VIII)

in welcher

$R^4$, X, Y und Z die oben angegebenen Bedeutungen haben und

$Q^3$ für Halogen oder Alkylsulfonyl steht,

gegebenenfalls in Gegenwart eines Säureakzeptors, wie z. B. Kaliumcarbonat, und gegebenenfalls in Gegenwart eines Verdünnungsmittels, wie z. B. Acetonitril oder Dimethylformamid, bei Temperaturen zwischen 0 °C und 150 °C umsetzt, oder wenn man

β) Hydrazinoazine der allgemeinen Formel (IX)

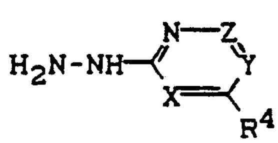

(IX)

in welcher

$R^4$, X, Y und Z die oben angegebenen Bedeutungen haben,
mit Cyaniminodithiokohlensäure-S,S-dimethylester der Formel (X)

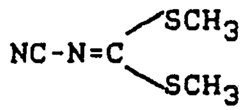

(X)

gegebenenfalls in Gegenwart eines Verdünnungsmittels, wie z. B. Methanol oder Ethanol, bei Temperaturen zwischen 0 °C und 100 °C umsetzt, oder wenn man

γ) Aminoguanidine der allgemeinen Formel (XIA) oder (XIB)

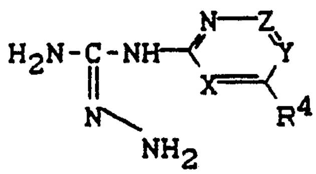

(XIA)

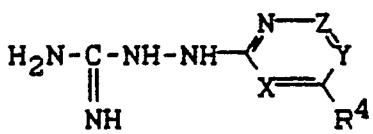

(XIB)

in welchen
$R^4$, X, Y und Z die oben angegebenen Bedeutungen haben,
oder saure Salze hiervon, wie z. B. die Hydrochloride,
mit Ester(amide)n der allgemeinen Formel (V)

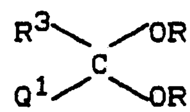

(V)

in welcher
$R^3$, R und $Q^1$ die oben angegebenen Bedeutungen haben,
gegebenenfalls in Gegenwart eines Verdünnungsmittels, wie z. B. Acetonitril oder Dimethylformamid, bei Temperaturen zwischen 0 °C und 150 °C umsetzt.

Die als Zwischenprodukte benötigten Aminoazole sind durch die Formel (VII) allgemein definiert. In Formel (VII) haben A′ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A′ und $R^3$ angegeben wurden.

Als Beispiele für die Verbindungen der Formel (VII) seien genannt:
3-Amino-1,2,4-triazol, 3-Amino-5-methyl-1,2,4-triazol, 3-Amino-5-methoxy-1,2,4-triazol, 3-Amino-5-methylthio-1,2,4-triazol, 3-Amino-pyrazol, 3-Amino-5-methyl-pyrazol, 3-Amino-5-methoxy-pyrazol und 3-Amino-5-methylthio-pyrazol.

Die Aminoazole der Formel (VII) sind bekannt und/oder können nach an sich bekannten Verfahren hergestellt werden (vgl. J. Org. Chem. 28 (1963), 1816 -1821; ibid. 39 (1974), 1522 - 1526).

Die weiter als Zwischenprodukte benötigten Azine sind durch die Formel (VIII) allgemein definiert. In Formel (VIII) haben $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$, X, Y und Z angegeben wurden, und $Q^1$ steht vorzugsweise für Fluor, Chlor, Brom oder $C_1$-$C_4$-Alkylsulfonyl, insbesondere für Chlor oder Methylsulfonyl.

Als Beispiele für die Verbindungen der Formel (VIII) seien genannt:
2-Chlor- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-

pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin sowie -4,6-bis-trifluormethyl-pyrimidin, ferner 2-Chlor-4,6-dimethyl-s-triazin 2-Chlor-4-methyl-6-methoxy-s-triazin, 2-Chlor-4,6-dimethoxy-s-triazin, 2,4-Dichlor-6-methoxy-s-triazin, 2-Chlor-4-ethyl-6-methoxy-s-triazin und 2-Chlor-4-methyl-6-ethoxy-s-triazin.

Die Azine der Formel (VIII) sind bekannt und/oder können nach an sich bekannten Verfahren hergestellt werden (vgl. J. Chem. Soc. 1957, 1830, 1833; J. Org. Chem. 26 (1961), 792; US-P 3 308 119 und US-P 4 711 959).

Die weiter als Zwischenprodukte benötigten Hydrazinoazine sind durch die Formel (IX) allgemein definiert. In Formel (IX) haben $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$, X, Y und Z angegeben wurden.

Als Beispiele für die Verbindungen der Formel (IX) seien genannt:

2-Hydrazino-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4,6-diethoxy-pyrimidin, 4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin sowie -4,6-bis-trifluormethyl-pyrimidin, ferner 2-Hydrazino-4,6-dimethyl-s-triazin, -4-methyl-6-methoxy-s-triazin, -4,6-dimethoxy-s-triazin, -4-ethyl-6-methoxy-s-triazin und -4-methyl-6-ethoxy-s-triazin.

Die Hydrazinoazine der Formel (IX) sind bekannt und/oder können nach an sich bekannten Verfahren hergestellt werden (vgl. Chem. Pharm. Bull. 11 (1963), 1382 - 1388).

Man erhält die Verbindungen der Formel (IX) im allgemeinen, wenn man Azine der Formel (VIII) - oben - mit Hydrazin oder Hydrazinhydrat, gegebenenfalls in Gegenwart eines Verdünnungsmittels, wie z. B. Ethanol, Aceton, Acetonitril oder Dimethylformamid, und gegebenenfalls in Gegenwart einer Base, wie z. B. Kaliumcarbonat oder Triethylamin, bei Temperaturen zwischen 0 °C und 100 °C umsetzt.

Der weiter als Zwischenprodukt benötigte Cyaniminodithiokohlensäure-S,S-dimethylester der Formel (X) ist bereits bekannt (vgl. J. Org. Chem. 32 (1967), 1566 -1572).

Die weiter als Zwischenprodukte benötigten Aminoguanidine sind durch die Formeln (XIA) und (XIB) allgemein definiert. In den Formeln (XIA) und (XIB) haben $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$, X, Y und Z angegeben wurden.

Beispiele für die Verbindungen der Formeln (XIA) und (XIB) sind in der nachstehenden Tabelle 2 aufgeführt. Die jeweils zeilenweise für einzelne Beispiele angegebenen Bedeutungen der Variablen $R^4$, X, Y und Z stehen in jedem Einzelfall für die Isomeren der Formel (XIA) und der Formel (XIB)

**Tabelle 2: Beispiele für die Verbindungen der Formeln (XIA) und (XIB)**

| $R^4$ | X | Y | Z |
|---|---|---|---|
| $CH_3$ | H | CH | C-$CH_3$ |
| $CH_3$ | N | CH | C-$OCH_3$ |
| $CH_3$ | N | CH | C-$OC_2H_5$ |
| $OCH_3$ | N | CH | C-$OCH_3$ |

Tabelle 2 - Fortsetzung

| $R^4$ | X | Y | Z |
|---|---|---|---|
| $OCH_3$ | N | CH | C-Cl |
| H | N | CH | $C-CH_3$ |
| $CF_3$ | N | CH | $C-OCH_3$ |
| $OCH_3$ | N | CH | $C-OCHF_2$ |
| $CH_3$ | N | CH | $C-OCHF_2$ |
| $OCHF_2$ | N | CH | $C-OCHF_2$ |
| $CH_3$ | N | N | $C-CH_3$ |
| $CH_3$ | N | N | $C-OCH_3$ |
| $OCH_3$ | N | N | $C-OCH_3$ |
| $C_2H_5$ | N | CH | $C-OCH_3$ |
| $C_2H_5$ | N | N | $C-OCH_3$ |

Die Aminoguanidine der Formeln (XIA) und (XIB) sind noch nicht aus der Literatur bekannt.

Man erhält die neuen Verbindungen der Formeln (XIA) und (XIB) im allgemeinen, wenn man Azine der Formel (VIII) - oben - mit Aminoguanidin oder einem sauren Salz hiervon, wie z. B. dem Hydrochlorid oder dem Hydrogencarbonat, gegebenenfalls in Gegenwart eines Verdünnungsmittels, wie z. B. Ethanol, Aceton, Acetonitril oder Dimethylformamid, und gegebenenfalls in Gegenwart einer Base, wie z. B. Kaliumcarbonat oder Triethylamin, bei Temperaturen zwischen 0 °C und 100 °C umsetzt.

Die Aminoguanidine der Formel (XIA) können auch aus bekannten S-Methyl-isothioureidoazinen der Formel (XII)

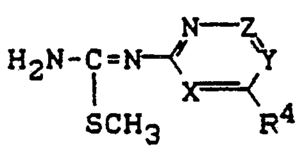

(XII)

in welcher

$R^4$, X, Y und Z die oben angegebenen Bedeutungen haben,

und Hydrazin oder Hydrazinhydrat, gegebenenfalls in Gegenwart eins Verdünnungsmittels, wie z. B. Ethanol oder Dioxan, bei Temperaturen zwischen 0 °C und 100 °C erhalten werden (vgl. EP-A 117 014/US-P 4 689 070).

Die Aminoguanidine der Formel (XIB) können auch durch Umsetzung von Hydrazinoazinen der Formel (IX) - oben - mit Cyanamid, gegebenenfalls in Gegenwart einer Säure, wie z. B. Salzsäure, und gegebenenfalls in Gegenwart eines Verdünnungsmittels, wie z. B. Ethanol und/oder Wasser, bei Temperaturen zwischen 0 °C und 100 °C erhalten werden.

Die weiter als Zwischenprodukte benötigten Ester(amide) sind durch die Formel (V) allgemein definiert. In Formel (V) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde, R steht vorzugsweise für $C_1$-$C_4$-Alkyl, insbesondere für Methyl oder Ethyl und $Q^1$ steht vorzugsweise für $C_1$-$C_4$-Alkoxy oder Di-($C_1$-$C_2$-alkyl)-amino, insbesondere für Methoxy, Ethoxy oder Dimethylamino.

Als Beispiele für die Verbindungen der Formel (V) seien genannt:
N,N-Dimethylformamid-dimethylacetal und -diethylacetal, Orthoameisensäure-trimethylester und -triethylester, Orthoessigsäure-trimethylester und -triethylester.

Die Verbindungen der Formel (V) sind bekannte Synthesechemikalien.

Die beim erfindungsgemäßen Verfahren (a) zur Herstellung der neuen Verbindungen der Formel (I) weiter als Ausgangsstoffe zu verwendenden Sulfonsäurehalogenide bzw. -anhydride sind durch die Formel (III) allgemein definiert. In Formel (III) hat $R^1$ vorzugsweise bzw. insbe sondere diejenige Bedeutung, die oben im Rahmen der Beschreibung der erfindungsgemäßen Verbindungen der Formel (I) vorzugsweise bzw. als insbesondere bevorzugt angegeben wurde und Q steht vorzugsweise für Chlor.

Als Ausgangsstoffe der Formel (III) seien beispielsweise genannt:
Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy-, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonylbenzolsulfonsäurechlorid sowie (2-Chlor-phenyl)-, (2-Cyano-phenyl)-, (2-Methoxycarbonyl-phenyl)- und (2-Trifluormethoxy-phenyl)-methansulfonsäurechlorid, 2-Chlor-6-methyl-benzolsulfonsäurechlorid und 2,6-Dichlor-benzolsulfonsäurechlorid.

Die Sulfonsäurehalogenide bzw. -anhydride der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem. 93 (1981), 151).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid und Pyridin.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert.-butylat, ferner aliphatische, aromatische oder heterocyclische Amine, beispielsweise Triethylamin, Trimethylamin, Dimethylanilin, Dimethylbenzylamin, Pyridin, Picolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Als Katalysatoren können beim erfindungsgemäßen Verfahren Metallhalogenide, wie z. B. Aluminiumchlorid oder Zinkchlorid, verwendet werden.

Die Reaktionstemperaturen können bei dem erfindungsgemäßen Verfahren (a) in einem größeren Bereich variiert werden. Im allgemeinen arbeitet man bei Temperaturen zwischen -50 °C und +50 °C, vorzugsweise bei Temperaturen zwischen -40 °C und +40 °C.

Zur Durchführung des erfindungsgemäßen Verfahrens (a) setzt man je Mol Aminoazol der Formel (II) im allgemeinen zwischen 1 und 5 Mol, vorzugsweise zwischen 1 und 4 Mol, Sulfonsäurehalogenid bzw. Sulfonsäureanhydrid der Formel (III) ein. Falls doppelt sulfonylierte Verbindungen der Formel (I, $R^2$ = $-SO_2-R^1$) in einer Eintopfreaktion hergestellt werden sollen, sind je Mol Aminoazol (II) mindestens 2 Mol Sulfonsäurehalogenid bzw. -anhydrid (III) einzusetzen.

Die Reaktionskomponenten können in beliebiger Reihenfolgen zusammengegeben werden.
In einer bevorzugten Ausführungsform des erfindungsgemäßen Verfahrens (a) werden die Ausgangsstoffe der Formeln (II) und (III) bei Raumtemperatur mit einem Verdünnungsmittel verrührt, und in diese Mischung wird - gegebenenfalls nach Abkühlen - der Säureakzeptor langsam eindosiert. Das Reaktionsgemisch wird dann bis zum Ende der Umsetzung gerührt.

Die Aufarbeitung kann auf übliche Weise durchgeführt werden, beispielsweise indem man - gegebenenfalls nach Einengen und/oder Verdünnen mit einem mit Wasser praktisch nicht mischbaren organischen Lösungsmittel, wie z. B. Methylenchlorid - mit Wasser wäscht, trocknet, filtriert und vom Filtrat das Lösungsmittel sorgfältig abdestilliert. Das im Rückstand verbleibende Rohprodukt kann auf übliche Weise,

z. B. durch Säulenchromatographie und/oder durch Umkristallisation, weiter gereinigt werden.

Die wie oben beschrieben erhältlichen Verbindungen der Formel (I), in welcher $R^2$ für die Gruppierung $-SO_2-R^1$ steht, können durch Umsetzung mit Desulfonylierungsmitteln, gegebenenfalls in Gegenwart von Verdünnungsmitteln, zu Verbindungen der Formel (I), in welcher $R^2$ für Wasserstoff steht, umgesetzt werden.

Unter Desulfonylierungsmitteln sind in diesem Zusammenhang Stoffe zu verstehen, die aus N,N-Bis-sulfonyl-aminoverbindungen eine Sulfonylgruppierung abspalten können. Geeignete Desulfonylierungsmittel sind vor allem Alkalimetall- oder Erdalkalimetall-hydroxide, wie Natrium-, Kalium- und Calcium-hydroxid, Alkalimetall-alkoholate, wie Natrium- und Kalium-methylat und -ethylat, ferner Ammoniak, Alkylamine, wie Methylamin, Ethylamin, Propylamin und Butylamin, sowie Dialkylamine, wie Dimethylamin und Diethylamin. Vorzugsweise wird Ammoniak als Desulfonylierungsmittel eingesetzt.

Die Desulfonylierung wird vorzugsweise in Gegenwart von Verdünnungsmitteln durchgeführt. Bevorzugte Verdünnungsmittel sind neben Wasser polare organische Solventien, wie Methanol, Ethanol, Propanol, Isopropanol, 2-Methoxy-ethanol, 2-Ethoxy-ethanol und Dioxan.

Die Desulfonylierung wird im allgemeinen bei Temperaturen zwischen 0 °C und 150 °C, vorzugsweise bei Temperaturen zwischen 20 °C und 100 °C durchgeführt.

Die Reaktionskomponenten zur Desulfonylierung werden im allgemeinen bei Raumtemperatur vermischt und, gegebenenfalls bei erhöhter Temperatur, bis zum Ende der Umsetzung gerührt. Zur Aufarbeitung wird gegebenenfalls eingeengt, mit Wasser verdünnt und mit einer starken Säure, wie z. B. Salzsäure, angesäuert. Das dabei kristallin anfallende Produkt (I, $R^2$ = H) kann durch Absaugen isoliert werden.

Die beim erfindungsgemäßen Verfahren (b) zur Herstellung von Verbindungen der Formel (I) als Ausgangsstoffe zu verwendenden sulfonylierten Aminoguanidine sind durch die Formeln (IVA) und (IVB) allgemein definiert.

In den Formeln (IVA) und (IVB) haben $R^1$, $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^4$, X, Y und Z angegeben wurden.

Beispiele für die Ausgangsstoffe der Formeln (IVA) und (IVB) sind die in der nachstehenden Tabelle 3 aufgeführt.

Die jeweils zeilenweise für einzelne Beispiele angegebenen Bedeutungen der Variablen $R^1$, $R^4$, X, Y und Z stehen in jedem Einzelfall für die Isomeren der Formeln (IVA) und (IVB).

<u>Tabelle 3</u>: Beispiele für die Verbindungen der Formeln (IVA) und (IVB)

| $R^1$ | $R^4$ | X | Y | Z |
|---|---|---|---|---|
| 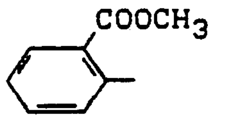 | $CH_3$ | N | CH | $C-CH_3$ |
| 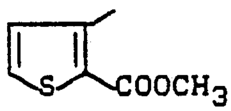 | $CH_3$ | N | CH | $C-CH_3$ |
| 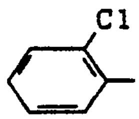 | $CH_3$ | N | CH | $C-CH_3$ |
| 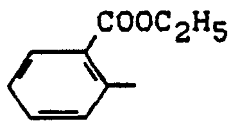 | $CH_3$ | N | CH | $C-CH_3$ |
| 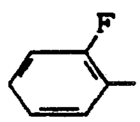 | $CH_3$ | N | CH | $C-CH_3$ |

Tabelle 3 - Fortsetzung

| $R^1$ | $R^4$ | X | Y | Z |
|---|---|---|---|---|
| 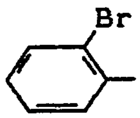 | $CH_3$ | N | CH | $C-CH_3$ |
| 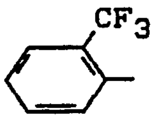 | $CH_3$ | N | CH | $C-CH_3$ |
| 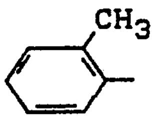 | $CH_3$ | N | CH | $C-CH_3$ |
| 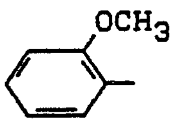 | $CH_3$ | N | CH | $C-CH_3$ |
| 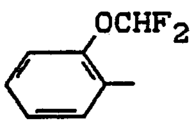 | $CH_3$ | N | CH | $C-CH_3$ |
| 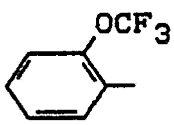 | $CH_3$ | N | CH | $C-CH_3$ |
| 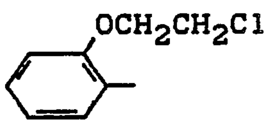 | $CH_3$ | N | CH | $C-CH_3$ |
| 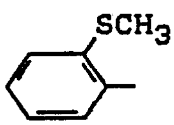 | $CH_3$ | N | CH | $C-CH_3$ |

Tabelle 3 - Fortsetzung

| $R^1$ | $R^4$ | X | Y | Z |
|---|---|---|---|---|
| 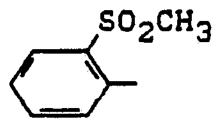 | $CH_3$ | N | CH | $C-CH_3$ |
| 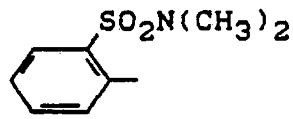 | $CH_3$ | N | CH | $C-CH_3$ |
| 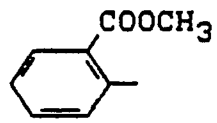 | $CH_3$ | N | CH | $C-OCH_3$ |
| 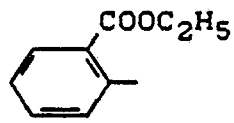 | $CH_3$ | N | CH | $C-OCH_3$ |
| 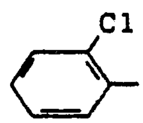 | $CH_3$ | N | CH | $C-OCH_3$ |
| 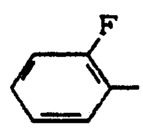 | $CH_3$ | N | CH | $C-OCH_3$ |
| 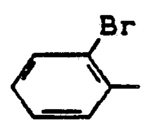 | $CH_3$ | N | CH | $C-OCH_3$ |
| 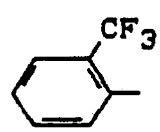 | $CH_3$ | N | CH | $C-OCH_3$ |

Tabelle 3 - Fortsetzung

| $R^1$ | $R^4$ | X | Y | Z |
|---|---|---|---|---|
| 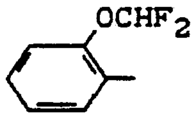 | $CH_3$ | N | CH | $C-OCH_3$ |
| 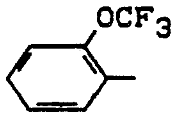 | $CH_3$ | N | CH | $C-OCH_3$ |
| 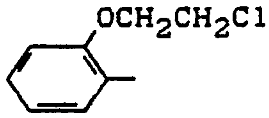 | $CH_3$ | N | CH | $C-OCH_3$ |
| 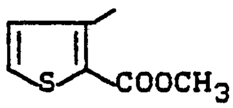 | $CH_3$ | N | CH | $C-OCH_3$ |
| 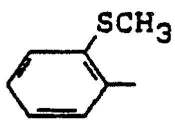 | $CH_3$ | N | CH | $C-OCH_3$ |
| 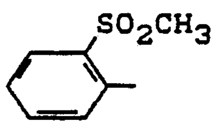 | $CH_3$ | N | CH | $C-OCH_3$ |
| 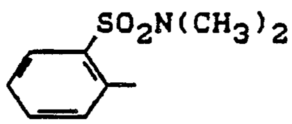 | $CH_3$ | N | CH | $C-OCH_3$ |
| 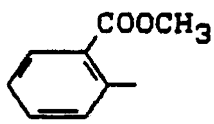 | $OCH_3$ | N | CH | $C-OCH_3$ |
| 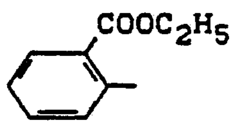 | $OCH_3$ | N | CH | $C-OCH_3$ |

Tabelle 3 - Fortsetzung

| $R^1$ | $R^4$ | X | Y | Z |
|---|---|---|---|---|
| 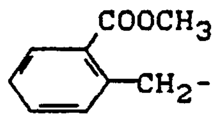 | $OCH_3$ | N | CH | $C-OCH_3$ |
| 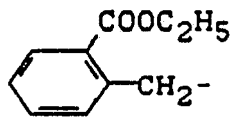 | $OCH_3$ | N | CH | $C-OCH_3$ |
| 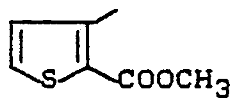 | $OCH_3$ | N | CH | $C-OCH_3$ |
| 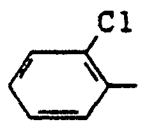 | $OCH_3$ | N | CH | $C-OCH_3$ |
| 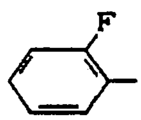 | $OCH_3$ | N | CH | $C-OCH_3$ |
| 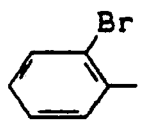 | $OCH_3$ | N | CH | $C-OCH_3$ |
| 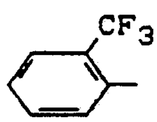 | $OCH_3$ | N | CH | $C-OCH_3$ |
| 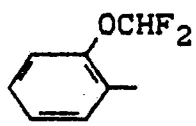 | $OCH_3$ | N | CH | $C-OCH_3$ |
| 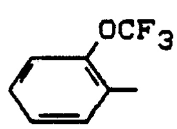 | $OCH_3$ | N | CH | $C-OCH_3$ |

Tabelle 3 - Fortsetzung

| $R^1$ | $R^4$ | X | Y | Z |
|---|---|---|---|---|
| 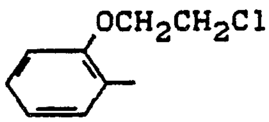 | $OCH_3$ | N | CH | $C-OCH_3$ |
| 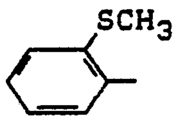 | $OCH_3$ | N | CH | $C-OCH_3$ |
| 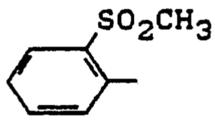 | $OCH_3$ | N | CH | $C-OCH_3$ |
| 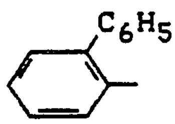 | $OCH_3$ | N | CH | $C-OCH_3$ |
| 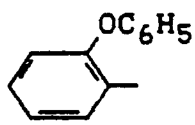 | $OCH_3$ | N | CH | $C-OCH_3$ |
| 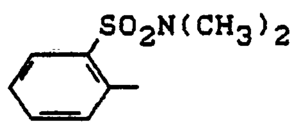 | $OCH_3$ | N | CH | $C-OCH_3$ |
| 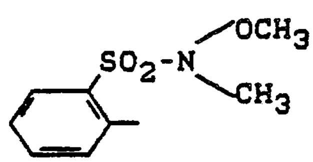 | $OCH_3$ | N | CH | $C-OCH_3$ |
| 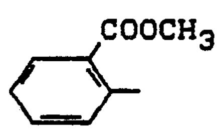 | $CF_3$ | N | CH | $C-OCH_3$ |

Tabelle 3 - Fortsetzung

| $R^1$ | $R^4$ | X | Y | Z |
|---|---|---|---|---|
| 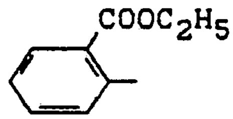 | $CF_3$ | N | CH | C-$OCH_3$ |
| 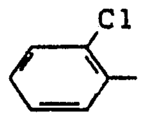 | $CF_3$ | N | CH | C-$OCH_3$ |
| 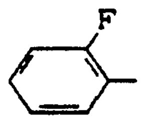 | $CF_3$ | N | CH | C-$OCH_3$ |
| 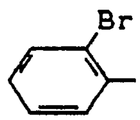 | $CF_3$ | N | CH | C-$OCH_3$ |
| 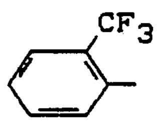 | $CF_3$ | N | CH | C-$OCH_3$ |
| 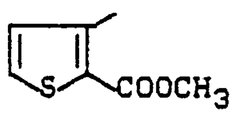 | $CF_3$ | N | CH | C-$OCH_3$ |
| 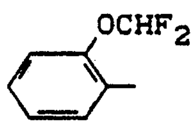 | $CF_3$ | N | CH | C-$OCH_3$ |
| 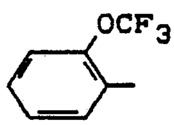 | $CF_3$ | N | CH | C-$OCH_3$ |
| 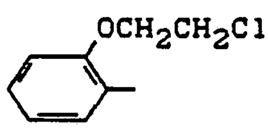 | $CF_3$ | N | CH | C-$OCH_3$ |

Tabelle 3 - Fortsetzung

| $R^1$ | $R^4$ | X | Y | Z |
|---|---|---|---|---|
| 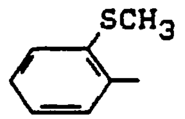 | $CF_3$ | N | CH | $C-OCH_3$ |
| 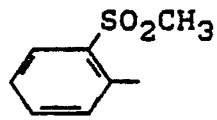 | $CF_3$ | N | CH | $C-OCH_3$ |
| 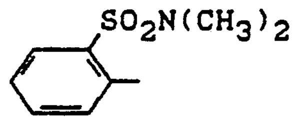 | $CF_3$ | N | CH | $C-OCH_3$ |
| 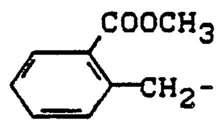 | $CF_3$ | N | CH | $C-OCH_3$ |
| 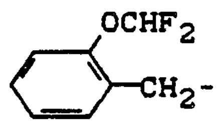 | $CF_3$ | N | CH | $C-OCH_3$ |
| 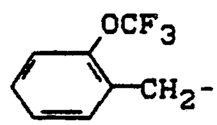 | $CF_3$ | N | CH | $C-OCH_3$ |
| 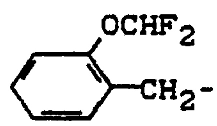 | $CH_3$ | N | CH | $C-OCH_3$ |
| 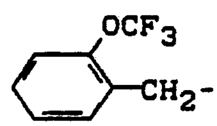 | $CH_3$ | N | CH | $C-OCH_3$ |

<u>Tabelle 3</u> - Fortsetzung

| $R^1$ | $R^4$ | X | Y | Z |
|---|---|---|---|---|
| 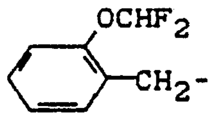 | $OCH_3$ | N | CH | $C-OCH_3$ |
| 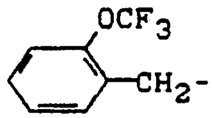 | $OCH_3$ | N | CH | $C-OCH_3$ |
| 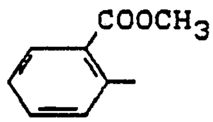 | $OCH_3$ | N | CH | C-Cl |
| 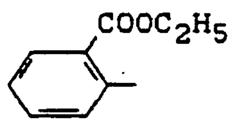 | $OCH_3$ | N | CH | C-Cl |
| 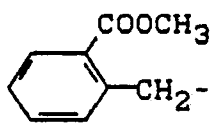 | $OCH_3$ | N | CH | C-Cl |
| 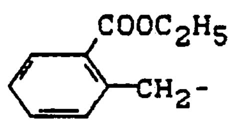 | $OCH_3$ | N | CH | C-Cl |
| 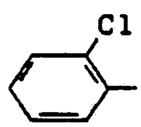 | $OCH_3$ | N | CH | C-Cl |
| 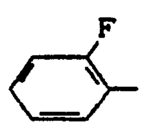 | $OCH_3$ | N | CH | C-Cl |

Tabelle 3 - Fortsetzung

| $R^1$ | $R^4$ | X | Y | Z |
|---|---|---|---|---|
| 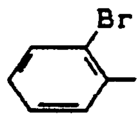 | $OCH_3$ | N | CH | C-Cl |
| 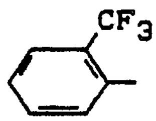 | $OCH_3$ | N | CH | C-Cl |
| 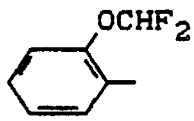 | $OCH_3$ | N | CH | C-Cl |
| 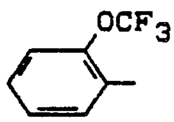 | $OCH_3$ | N | CH | C-Cl |
| 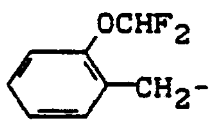 | $OCH_3$ | N | CH | C-Cl |
| 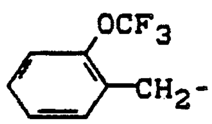 | $OCH_3$ | N | CH | C-Cl |
| 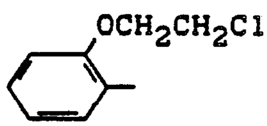 | $OCH_3$ | N | CH | C-Cl |
| 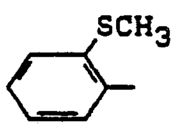 | $OCH_3$ | N | CH | C-Cl |

Tabelle 3 - Fortsetzung

| $R^1$ | $R^4$ | X | Y | Z |
|---|---|---|---|---|
| 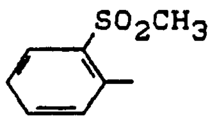 | $OCH_3$ | N | CH | C-Cl |
| 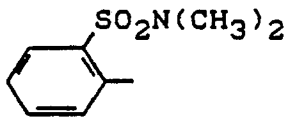 | $OCH_3$ | N | CH | C-Cl |
| 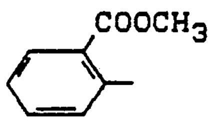 | $CH_3$ | N | CH | $C-OCHF_2$ |
| 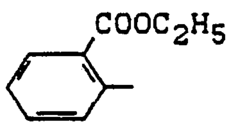 | $CH_3$ | N | CH | $C-OCHF_2$ |
| 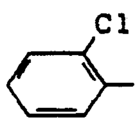 | $CH_3$ | N | CH | $C-OCHF_2$ |
| 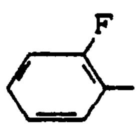 | $CH_3$ | N | CH | $C-OCHF_2$ |
| 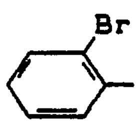 | $CH_3$ | N | CH | $C-OCHF_2$ |
| 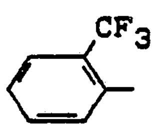 | $CH_3$ | N | CH | $C-OCHF_2$ |

Tabelle 3 - Fortsetzung

| $R^1$ | $R^4$ | X | Y | Z |
|---|---|---|---|---|
| 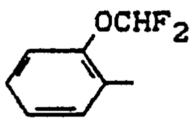 | $CH_3$ | N | CH | $C-OCHF_2$ |
| 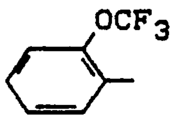 | $CH_3$ | N | CH | $C-OCHF_2$ |
| 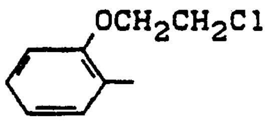 | $CH_3$ | N | CH | $C-OCHF_2$ |
| 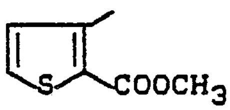 | $CH_3$ | N | CH | $C-OCHF_2$ |
| 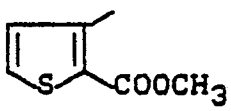 | $OCH_3$ | N | CH | C-Cl |
| 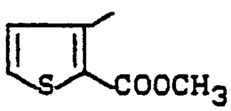 | $OCH_3$ | N | CH | $C-OCHF_2$ |
| 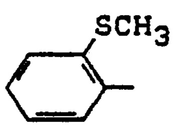 | $CH_3$ | N | CH | $C-OCHF_2$ |
| 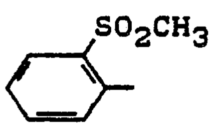 | $CH_3$ | N | CH | $C-OCHF_2$ |
| 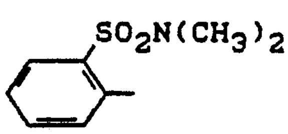 | $CH_3$ | N | CH | $C-OCHF_2$ |

Tabelle 3 - Fortsetzung

| $R^1$ | $R^4$ | X | Y | Z |
|---|---|---|---|---|
| 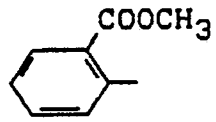 | $OCH_3$ | N | CH | $C-OCHF_2$ |
| 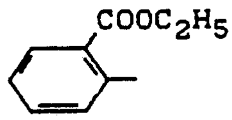 | $OCH_3$ | N | CH | $C-OCHF_2$ |
| 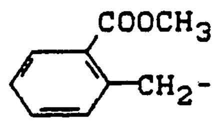 | $OCH_3$ | N | CH | $C-OCHF_2$ |
| 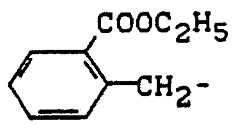 | $OCH_3$ | N | CH | $C-OCHF_2$ |
| 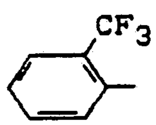 | $OCH_3$ | N | CH | $C-OCHF_2$ |
| 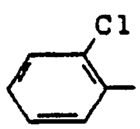 | $OCH_3$ | N | CH | $C-OCHF_2$ |
| 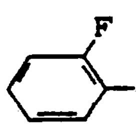 | $OCH_3$ | N | CH | $C-OCHF_2$ |
| 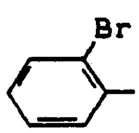 | $OCH_3$ | N | CH | $C-OCHF_2$ |

Tabelle 3 - Fortsetzung

| $R^1$ | $R^4$ | X | Y | Z |
|---|---|---|---|---|
| 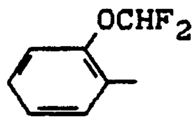 | $OCH_3$ | N | CH | $C\text{-}OCHF_2$ |
| 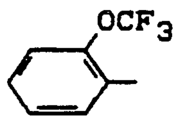 | $OCH_3$ | N | CH | $C\text{-}OCHF_2$ |
| 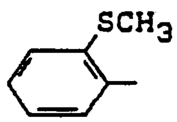 | $OCH_3$ | N | CH | $C\text{-}OCHF_2$ |
| 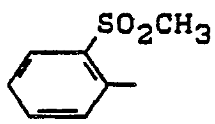 | $OCH_3$ | N | CH | $C\text{-}OCHF_2$ |
| 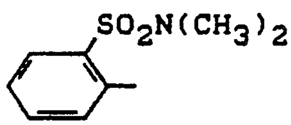 | $OCH_3$ | N | CH | $C\text{-}OCHF_2$ |
| 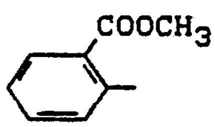 | $OCHF_2$ | N | CH | $C\text{-}OCHF_2$ |
| 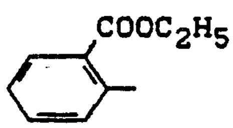 | $OCHF_2$ | N | CH | $C\text{-}OCHF_2$ |
| 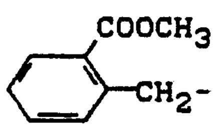 | $OCHF_2$ | N | CH | $C\text{-}OCHF_2$ |

<u>Tabelle 3</u> - Fortsetzung

| $R^1$ | $R^4$ | X | Y | Z |
|---|---|---|---|---|
| 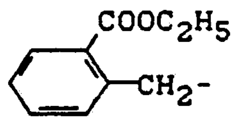 | $OCHF_2$ | N | CH | $C-OCHF_2$ |
| 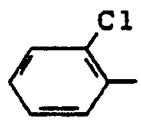 | $OCHF_2$ | N | CH | $C-OCHF_2$ |
| 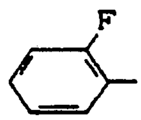 | $OCHF_2$ | N | CH | $C-OCHF_2$ |
| 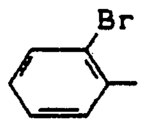 | $OCHF_2$ | N | CH | $C-OCHF_2$ |
| 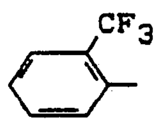 | $OCHF_2$ | N | CH | $C-OCHF_2$ |
| 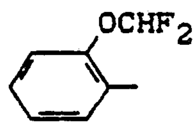 | $OCHF_2$ | N | CH | $C-OCHF_2$ |
| 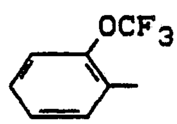 | $OCHF_2$ | N | CH | $C-OCHF_2$ |
| 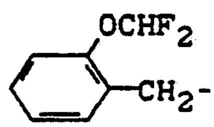 | $OCHF_2$ | N | CH | $C-OCHF_2$ |

Tabelle 3 - Fortsetzung

| $R^1$ | $R^4$ | X | Y | Z |
|---|---|---|---|---|
| 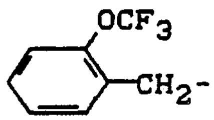 | $OCHF_2$ | N | CH | $C-OCHF_2$ |
| 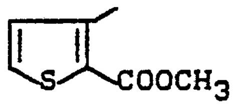 | $OCHF_2$ | N | CH | $C-OCHF_2$ |
| 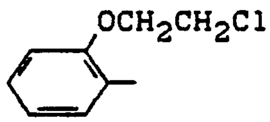 | $OCHF_2$ | N | CH | $C-OCHF_2$ |
| 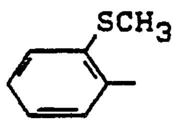 | $OCHF_2$ | N | CH | $C-OCHF_2$ |
| 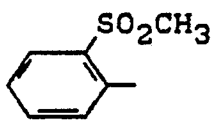 | $OCHF_2$ | N | CH | $C-OCHF_2$ |
| 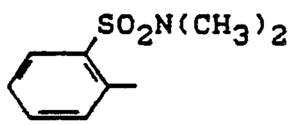 | $OCHF_2$ | N | CH | $C-OCHF_2$ |
| 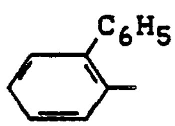 | $OCHF_2$ | N | CH | $C-OCHF_2$ |
| 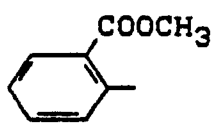 | $CH_3$ | N | N | $C-CH_3$ |
| 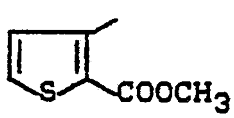 | $CH_3$ | N | N | $C-CH_3$ |

Tabelle 3 - Fortsetzung

| $R^1$ | $R^4$ | X | Y | Z |
|---|---|---|---|---|
| 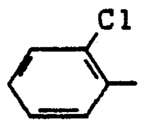 | $CH_3$ | N | N | $C-CH_3$ |
| 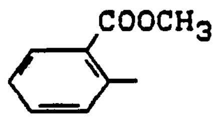 | $CH_3$ | N | N | $C-OCH_3$ |
| 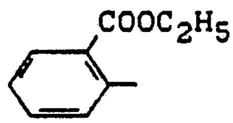 | $CH_3$ | N | N | $C-OCH_3$ |
| 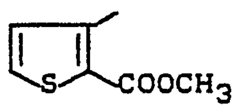 | $CH_3$ | N | N | $C-OCH_3$ |
| 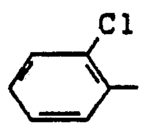 | $CH_3$ | N | N | $C-OCH_3$ |
| 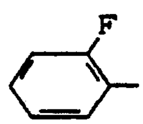 | $CH_3$ | N | N | $C-OCH_3$ |
| 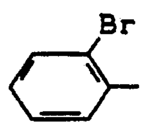 | $CH_3$ | N | N | $C-OCH_3$ |
| 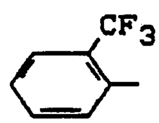 | $CH_3$ | N | N | $C-OCH_3$ |
| 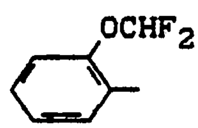 | $CH_3$ | N | N | $C-OCH_3$ |

<u>Tabelle 3</u> - Fortsetzung

| $R^1$ | $R^4$ | X | Y | Z |
|---|---|---|---|---|
| 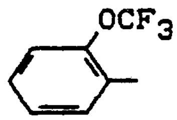 | $CH_3$ | N | N | $C-OCH_3$ |
| 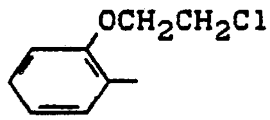 | $CH_3$ | N | N | $C-OCH_3$ |
| 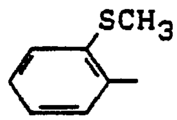 | $CH_3$ | N | N | $C-OCH_3$ |
| 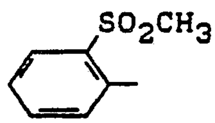 | $CH_3$ | N | N | $C-OCH_3$ |
| 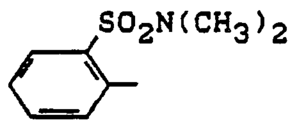 | $CH_3$ | N | N | $C-OCH_3$ |
| 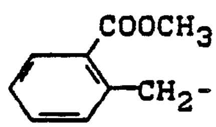 | $CH_3$ | N | N | $C-OCH_3$ |
| 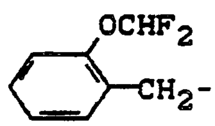 | $CH_3$ | N | N | $C-OCH_3$ |
| 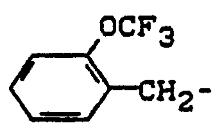 | $CH_3$ | N | N | $C-OCH_3$ |

Tabelle 3 - Fortsetzung

| $R^1$ | $R^4$ | X | Y | Z |
|---|---|---|---|---|
| 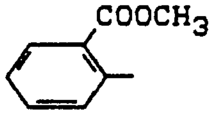 | $OCH_3$ | N | N | $C-OCH_3$ |
| 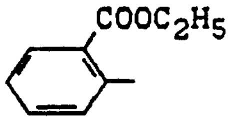 | $OCH_3$ | N | N | $C-OCH_3$ |
| 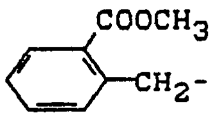 | $OCH_3$ | N | N | $C-OCH_3$ |
| 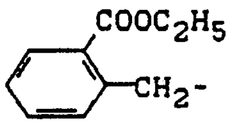 | $OCH_3$ | N | N | $C-OCH_3$ |
| 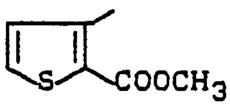 | $OCH_3$ | N | N | $C-OCH_3$ |
| 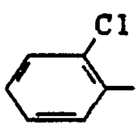 | $OCH_3$ | N | N | $C-OCH_3$ |
| 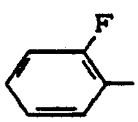 | $OCH_3$ | N | N | $C-OCH_3$ |
| 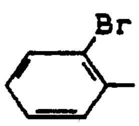 | $OCH_3$ | N | N | $C-OCH_3$ |
| 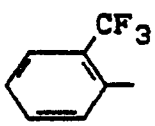 | $OCH_3$ | N | N | $C-OCH_3$ |

Tabelle 3 - Fortsetzung

| $R^1$ | $R^4$ | X | Y | Z |
|---|---|---|---|---|
| 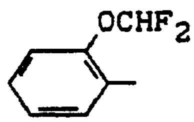 | $OCH_3$ | N | N | $C-OCH_3$ |
| 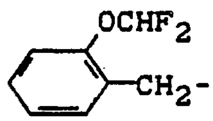 | $OCH_3$ | N | N | $C-OCH_3$ |
| 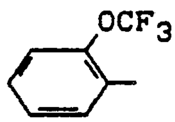 | $OCH_3$ | N | N | $C-OCH_3$ |
| 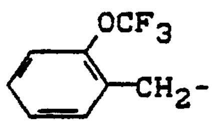 | $OCH_3$ | N | N | $C-OCH_3$ |
| 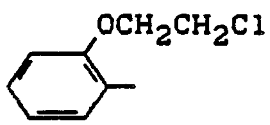 | $OCH_3$ | N | N | $C-OCH_3$ |
| 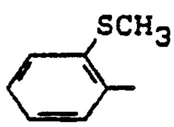 | $OCH_3$ | N | N | $C-OCH_3$ |
| 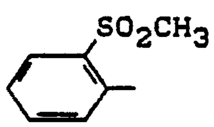 | $OCH_3$ | N | N | $C-OCH_3$ |
| 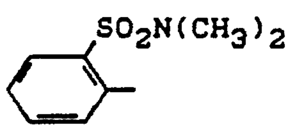 | $OCH_3$ | N | N | $C-OCH_3$ |

Tabelle 3 - Fortsetzung

| $R^1$ | $R^4$ | X | Y | Z |
|---|---|---|---|---|
| 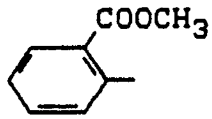 | $C_2H_5$ | N | N | $C-OCH_3$ |
| 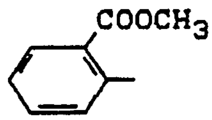 | $CH_3$ | N | N | $C-OC_2H_5$ |
| 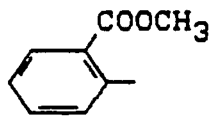 | $OCH_3$ | N | N | $C-NHC_2H_5$ |
| 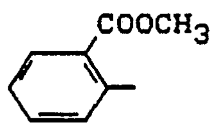 | $OC_2H_5$ | N | N | $C-NHCH_3$ |
| 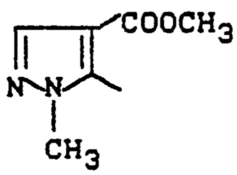 | $OCH_3$ | N | CH | $C-OCH_3$ |
| 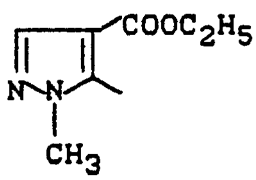 | $OCH_3$ | N | CH | $C-OCH_3$ |
| 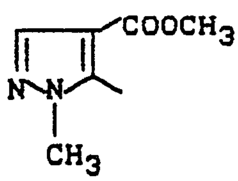 | $OCH_3$ | N | N | $C-OCH_3$ |
| 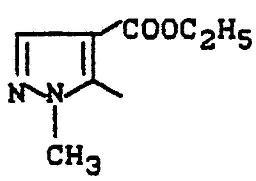 | $OCH_3$ | N | N | $C-OCH_3$ |

Tabelle 3 - Fortsetzung

| $R^1$ | $R^4$ | X | Y | Z |
|---|---|---|---|---|
| 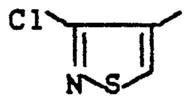 | $OCH_3$ | N | CH | $C-OCH_3$ |
| 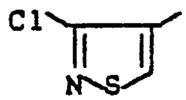 | $OCH_3$ | N | N | $C-OCH_3$ |
| 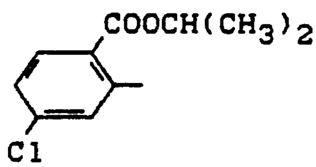 | $OCH_3$ | N | N | $C-OCH_3$ |
| 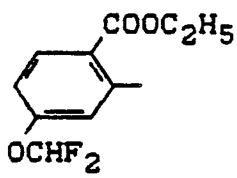 | $OCH_3$ | N | CH | $C-OCH_3$ |
| 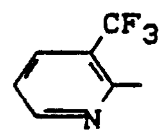 | $OCH_3$ | N | CH | $C-OCH_3$ |
| 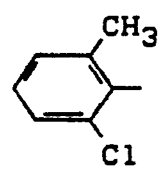 | $OCH_3$ | N | CH | $C-OCH_3$ |
| 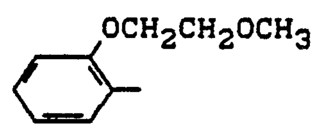 | $OCH_3$ | N | CH | $C-OCH_3$ |
| 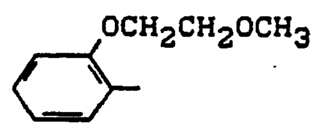 | $OCH_3$ | N | N | $C-OCH_3$ |

Die sulfonylierten Aminoguanidine der Formel (IVA) sind teilweise bekannt (vgl. EP-A 224 078, US-P 4 725 303) und teilweise Gegenstand einer nicht vorveröffentlichten DE-Patentanmeldung (P 38 18 040.5 vom 27.05.1988).

Man erhält die Verbindungen der allgemeinen Formel (IVA), wenn man Sulfonylverbindungen der allgemeinen Formel (XIII)

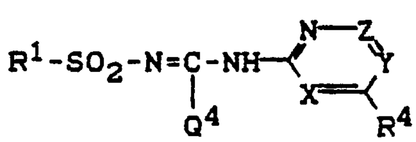 (XIII)

in welcher
$R^1$, $R^4$, X, Y und Z die oben angegebenen Bedeutungen haben und
$Q^4$ für Halogen oder eine der nachstehend angegebenen Abgangsgruppen $R^{34}-SO_2-\overset{|}{N}-O-R^{35}$ oder $-Q^5-R^{36}$ steht, worin
$R^{34}$ die oben für $R^1$ angegebene Bedeutung hat, aber nicht in jedem Einzelfall mit $R^1$ identisch sein muß,
$R^{35}$ für Alkyl, Alkenyl oder Aralkyl steht,
$R^{36}$ für jeweils gegebenenfalls substituiertes Alkyl, Aralkyl oder Aryl steht und
$Q^5$ für Sauerstoff oder Schwefel steht,
mit Hydrazin oder einem Hydrazin-Wasser- oder Hydrazin-Säure-Addukt,
gegebenenfalls in Gegenwart eines Säureakzeptors, wie z. B. Kaliumcarbonat, und gegebenenfalls in Gegenwart eines Verdünnungsmittels, wie z. B. Methanol, Ethanol und/oder Wasser, bei Temperaturen zwischen -20 °C und +100 °C umsetzt.

Ein Teil der Verbindungen der Formel (IVA) kann auch wie nachstehend skizziert erhalten werden (R wie oben für $R^{10}$ angegeben)

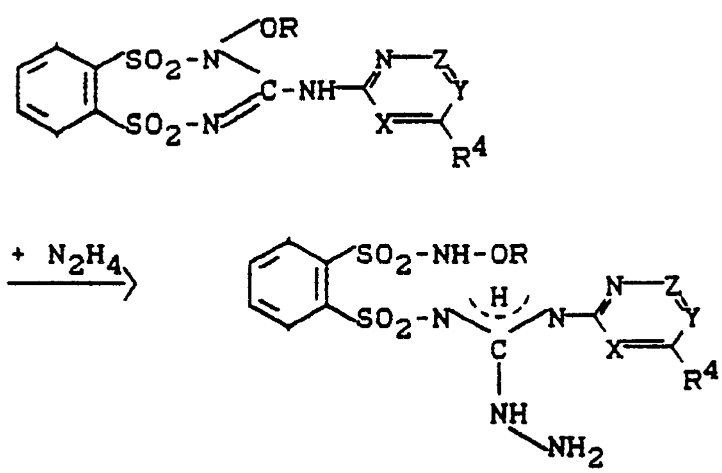


(zum Reaktionsprinzip vgl. US-P 4 659 364, EP-A 173 319).

Die als Zwischenprodukte benötigten Sulfonylverbindungen sind durch die Formel (XIII) allgemein definiert. In Formel (XIII) haben $R^1$, $R^4$, X, Y und Z vorzugsweise bzw. insbesondere diejenigen Bedeutungen, die bereis oben im Rahmen der Beschreibung der erfindungsgemäßen Verbindungen der Formel (I) als bevorzugt bzw. als insbesondere bevorzugt für $R^1$, $R^4$, X, Y und Z angegeben wurden und
$Q^4$ steht vorzugsweise für Chlor oder eine der nachstehend angegebenen Abgangsgruppen
$R^{34}-SO_2-\overset{|}{N}-OR^{35}$ oder $-Q^5-R^{36}$, worin
$R^{34}$ die oben für $R^1$ vorzugsweise angegebene Bedeutung hat, aber nicht in jedem Einzelfall mit $R^1$ identisch sein muß,
$R^{35}$ für $C_1$-$C_4$-Alkyl, $C_3$-$C_4$-Alkenyl oder Benzyl steht,
$R^{36}$ für gegebenenfalls durch Carboxy, $C_1$-$C_4$-Alkoxy-carbonyl oder $C_1$-$C_4$-Alkoxy substituiertes $C_1$-$C_4$-Alkyl oder für jeweils gegebenenfalls durch Fluor, Chlor, Brom, $C_1$-$C_4$-Alkyl oder $C_1$-$C_4$-Alkoxy substituiertes Benzyl oder Phenyl steht, und
$Q^5$ für Sauerstoff oder Schwefel steht.

Beispiele für die Verbindungen der Formel (XIII) sind in der nachstehenden Tabelle 4 aufgeführt.

**Tabelle 4**: Beispiele für die Verbindungen der Formel (XIII)

| $Q^4$ | $R^1$ | $R^4$ | X | Y | Z |
|---|---|---|---|---|---|
| 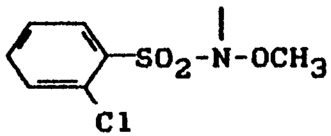 | 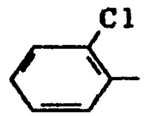 | $CH_3$ | N | CH | $C-OCH_3$ |
| 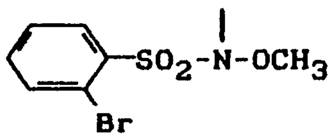 | 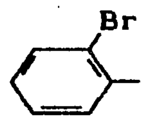 | $OCH_3$ | N | CH | $C-OCH_3$ |
| 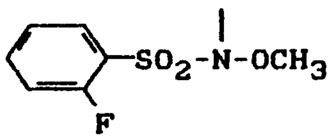 | 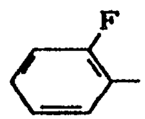 | $OCH_3$ | N | CH | $C-OCH_3$ |
| 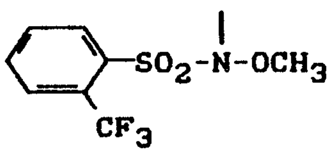 | 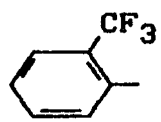 | $OCH_3$ | N | CH | $C-OCH_3$ |
| 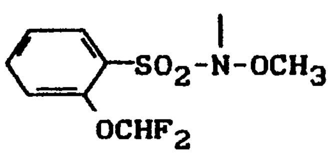 | 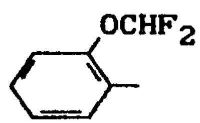 | $OCH_3$ | N | CH | $C-OCH_3$ |

**Tabelle 4** - Fortsetzung

| $Q^4$ | $R^1$ | $R^4$ | X | Y | Z |
|---|---|---|---|---|---|
| 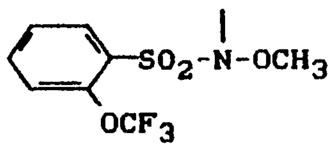 | 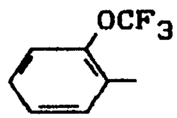 | $OCH_3$ | N | CH | $C-OCH_3$ |
| 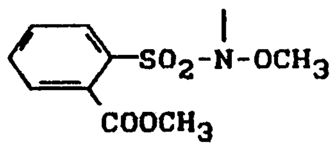 | 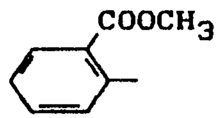 | $CH_3$ | N | CH | $C-CH_3$ |
| 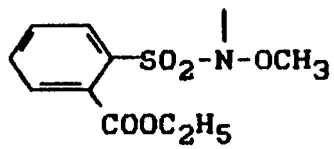 | 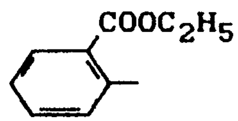 | $CH_3$ | N | CH | $C-CH_3$ |
| 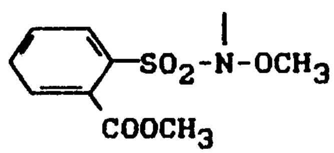 | 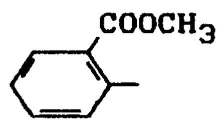 | $OCH_3$ | N | CH | $C-OCH_3$ |
| 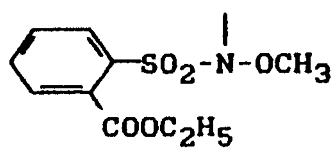 | 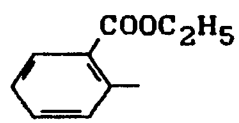 | $OCH_3$ | N | CH | $C-OCH_3$ |

**Tabelle 4** - Fortsetzung

| $Q^4$ | $R^1$ | $R^4$ | X | Y | Z |
|---|---|---|---|---|---|
| 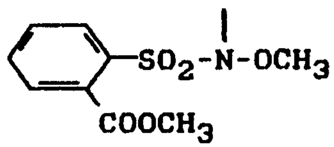 | 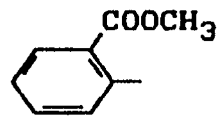 | $C_2H_5$ | N | CH | C-OCH$_3$ |
| 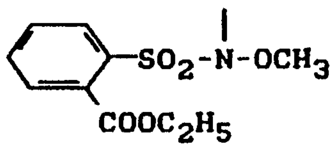 | 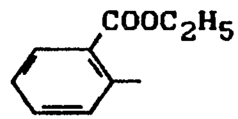 | $C_2H_5$ | N | CH | C-OCH$_3$ |
| 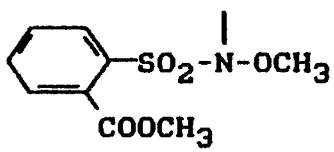 | 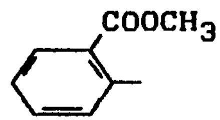 | $OCH_3$ | N | CH | C-Cl |
| 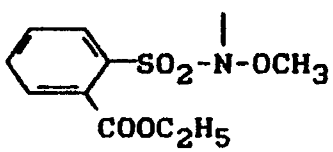 | 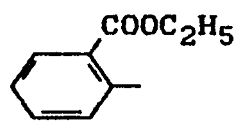 | $OCH_3$ | N | CH | C-Cl |
| 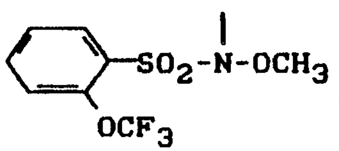 | 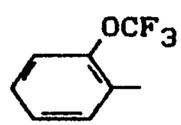 | $OCH_3$ | N | CH | C-Cl |

<u>Tabelle 4</u> - Fortsetzung

| $Q^4$ | $R^1$ | $R^4$ | X | Y | Z |
|---|---|---|---|---|---|
| 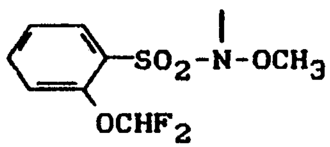 | 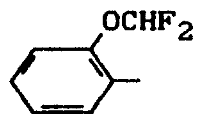 | $OCH_3$ | N | CH | C-Cl |
| 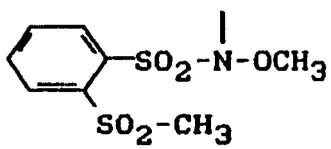 | 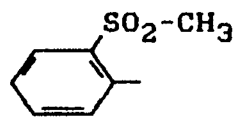 | H | N | CH | $C-CH_3$ |
| 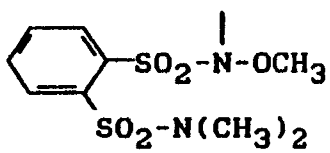 | 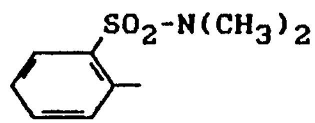 | $CH_3$ | N | CH | $C-OCH_3$ |
| 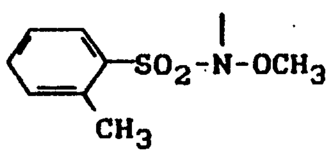 | 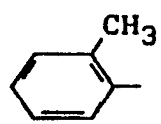 | $OCH_3$ | N | CH | $C-OCH_3$ |
| 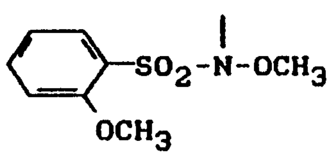 | 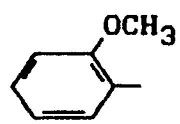 | $CH_3$ | N | CH | $C-OCH_3$ |

**Tabelle 4** - Fortsetzung

| $Q^4$ | $R^1$ | $R^4$ | X | Y | Z |
|---|---|---|---|---|---|
| 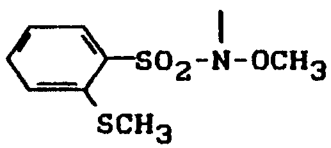 | 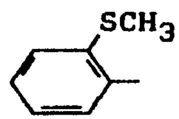 | $OCH_3$ | N | CH | $C-OCH_3$ |
| 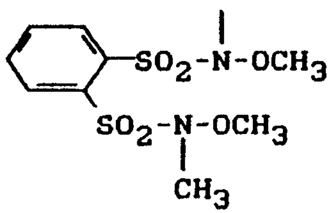 | 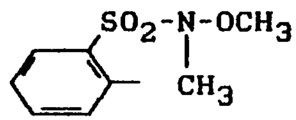 | $OCH_3$ | N | CH | $C-OCH_3$ |
| 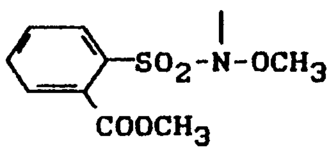 | 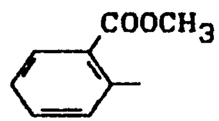 | $CH_3$ | N | CH | $C-OC_2H_5$ |
| 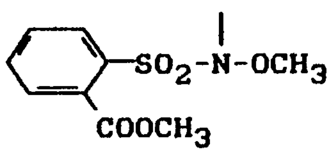 | 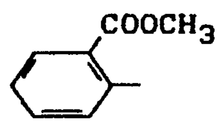 | $OCHF_2$ | N | CH | $C-CH_3$ |

Tabelle 4 - Fortsetzung

| $Q^4$ | $R^1$ | $R^4$ | X | Y | Z |
|---|---|---|---|---|---|
| 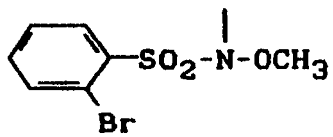 | 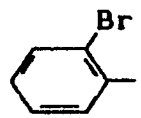 | $CH_3$ | N | CH | $C-SCH_3$ |
| 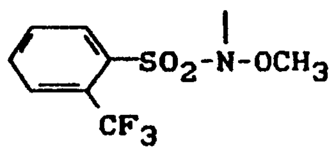 | 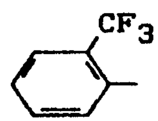 | $CH_3$ | N | CH | $C-N(CH_3)_2$ |
| 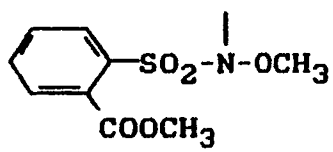 | 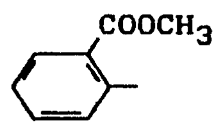 | $OCHF_2$ | N | CH | $C-OCHF_2$ |
| 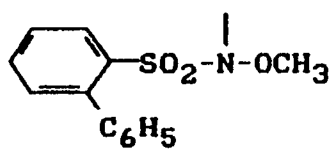 | 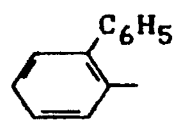 | $OCH_3$ | N | CH | $C-OCH_3$ |
| 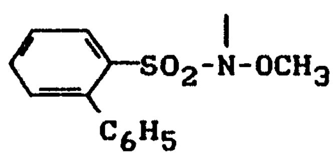 | 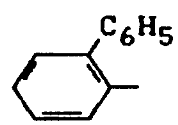 | $OCH_3$ | N | N | $C-OCH_3$ |

**Tabelle 4** - Fortsetzung

| $Q^4$ | $R^1$ | $R^4$ | X | 'Y | Z |
|---|---|---|---|---|---|
| 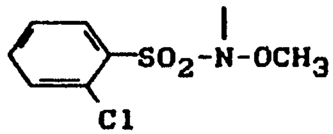 | 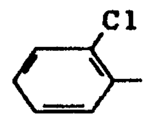 | $CH_3$ | N | N | C-$OCH_3$ |
| 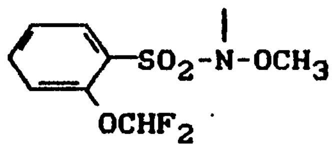 | 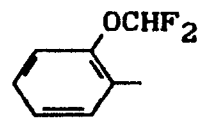 | $OCH_3$ | N | N | C-$OCH_3$ |
| 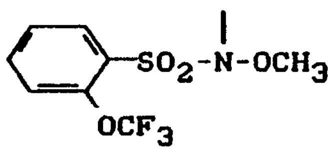 | 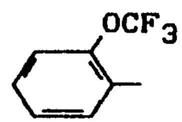 | $OCH_3$ | N | N | C-$OCH_3$ |
| 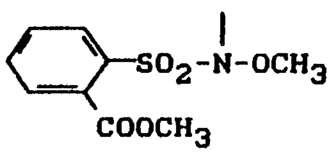 | 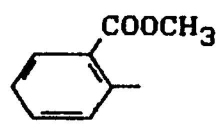 | $CH_3$ | N | N | C-$OCH_3$ |
| 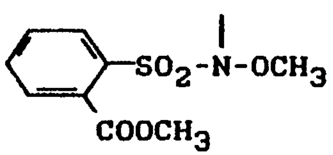 | 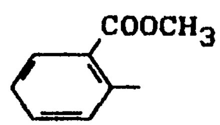 | $OCH_3$ | N | N | C-$OCH_3$ |

**<u>Tabelle 4</u> - Fortsetzung**

| $Q^4$ | $R^1$ | $R^4$ | X | Y | Z |
|---|---|---|---|---|---|
| 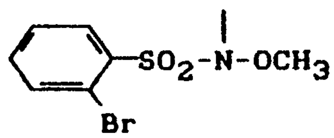 | 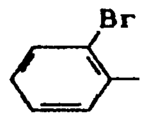 | $CH_3$ | N | N | $C-CH_3$ |
| 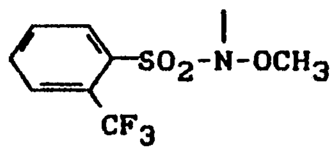 | 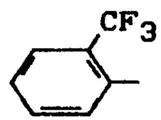 | $CH_3$ | N | N | C-Cl |
| 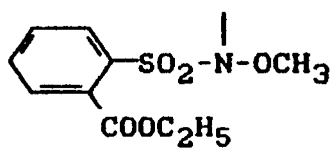 | 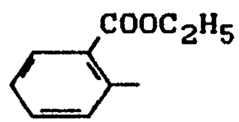 | $OCH_3$ | N | N | $C-OCH_3$ |
| 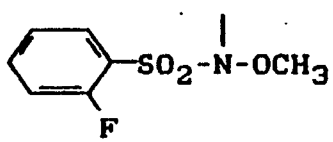 | 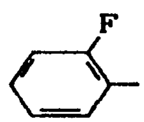 | $OCH_3$ | N | N | $C-OCH_3$ |
| 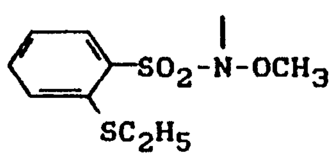 | 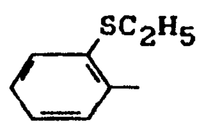 | $OCH_3$ | N | N | $C-OCH_3$ |

**Tabelle 4** - Fortsetzung

| $Q^4$ | $R^1$ | $R^4$ | X | Y | Z |
|---|---|---|---|---|---|
| 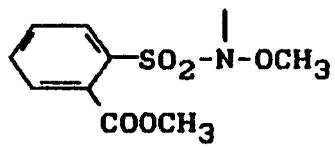 | 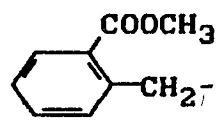 | $OCH_3$ | N | CH | $C-OCH_3$ |
| 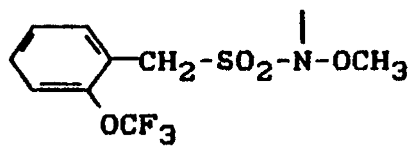 | 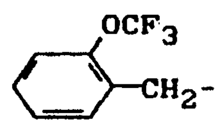 | $OCH_3$ | N | CH | $C-OCH_3$ |
| $-OC_6H_5$ | 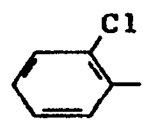 | $CH_3$ | N | CH | $C-CH_3$ |
| $-OCH_3$ | 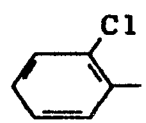 | $CH_3$ | N | CH | $C-OCH_3$ |
| $-SCH_3$ | 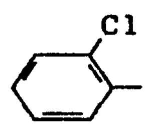 | $OCH_3$ | N | CH | $C-OCH_3$ |

Tabelle 4 - Fortsetzung

| $Q^4$ | $R^1$ | $R^4$ | X | Y | Z |
|---|---|---|---|---|---|
| $-SC_6H_5$ | 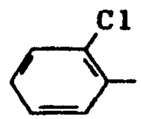 | $OCH_3$ | N | CH | $C-OCH_3$ |
| 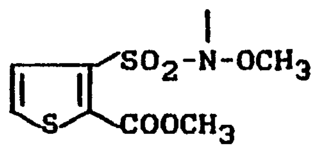 | 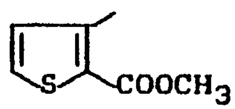 | $CH_3$ | N | N | $C-OCH_3$ |
| 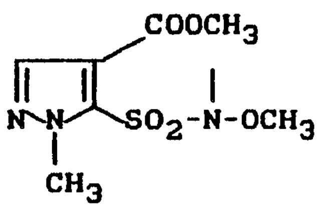 | 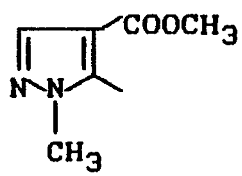 | $OCH_3$ | N | CH | $C-OCH_3$ |
| 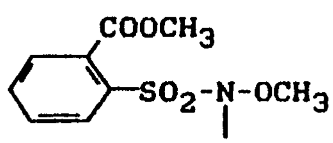 | 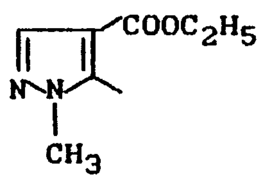 | $OCH_3$ | N | CH | $C-OCH_3$ |

<u>**Tabelle 4**</u> **- Fortsetzung**

| $Q^4$ | $R^1$ | $R^4$ | X | Y | Z |
|---|---|---|---|---|---|
| Cl | 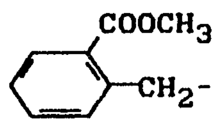 | $OCH_3$ | N | CH | $C-OCH_3$ |
| $-OC_6H_5$ | 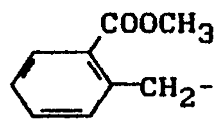 | $OCH_3$ | N | CH | $C-OCH_3$ |
| $-SC_6H_5$ | 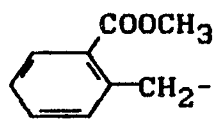 | $OCH_3$ | N | CH | $C-OCH_3$ |
| Cl | 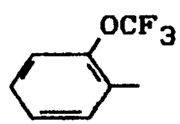 | $CH_3$ | N | N | $C-OCH_3$ |
| 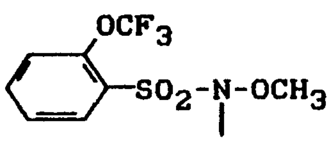 | 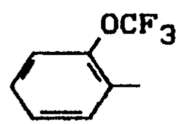 | $CH_3$ | N | N | $C-OCH_3$ |
| $O-C_6H_5$ | 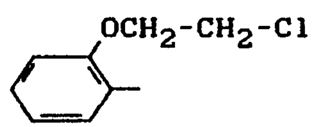 | $CH_3$ | N | N | $C-OCH_3$ |

**Tabelle 4** - Fortsetzung

| $Q^4$ | $R^1$ | $R^4$ | X | Y | Z |
|---|---|---|---|---|---|
| 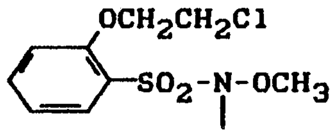 | 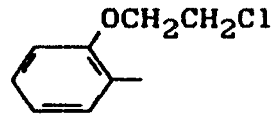 | $CH_3$ | N | N | $C-OCH_3$ |
| $S-C_6H_5$ | 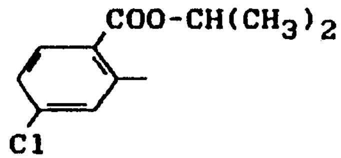 | $CH_3$ | N | N | $C-OCH_3$ |
| $S-CH_3$ | 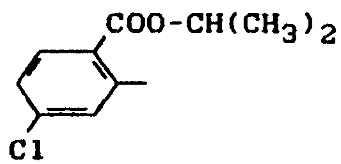 | $CH_3$ | N | N | $O-CH_3$ |
| 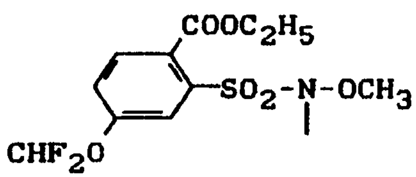 | 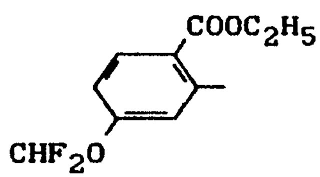 | $OCH_3$ | N | CH | $O-CH_3$ |
| $OCH_3$ | 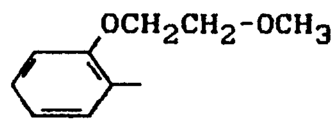 | $OCH_3$ | N | N | $O-CH_3$ |

Die Verbindungen der Formel (XIII) sind bekannt und/oder können nach an sich bekannten Verfahren hergestellt werden (vgl. EP-A 5 986, EP-A 24 215, EP-A 121 082, EP-A 172 957, EP-A 173 321, EP-A 173 956, EP-A 224 078, DE-OS 36 34 928, DE-OS 36 34 929).

Die sulfonylierten Aminoguanidine der Formel (IVB) sind noch nicht aus der Literatur bekannt und Gegenstand der vorliegenden Erfindung. Man erhält die neuen sulfonylierten Aminoguanidine der allgemeinen Formel (IVB), wenn man die dazu isomeren Verbindungen der Formel (IVA) - oben - durch Verrühren mit einem polaren Lösungsmittel, wie z. B. Methanol, Ethanol, Propanol, Isopropanol und/oder Wasser, bei Temperaturen zwischen 0 °C und 150 °C, umlagert.

Zur Herstellung der Verbindungen der Formel (IVB) können auch die Isomeren der Formel (IVA) wie oben beschrieben erzeugt und in situ, d. h. ohne Zwischenisolierung, umgelagert werden.

Die beim erfindungsgemäßen Verfahren (b) zur Herstellung von Verbindungen der Formel (I) weiter als Ausgangsstoffe zu verwendenden Ester(amide) sind durch die Formel (V) allgemein definiert.

In Formel (V) hat $R^3$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^3$ angegeben wurde, R steht vorzugsweise für $C_1$-$C_4$- Alkyl, insbesondere für Methyl oder Ethyl und $Q^1$ steht vorzugsweise für $C_1$-$C_4$-Alkoxy oder Di-($C_1$-$C_2$-alkyl)-amino, insbesondere für Methoxy, Ethoxy oder Dimethylamino.

Als Beispiele für die Ausgangsstoffe der Formel (V) seien genannt:

N,N-Dimethylformamid-dimethylacetal und -diethylacetal, Orthoameisensäure-trimethylester und -triethylester, Orthoessigsäure-trimethylester und -triethylester.

Die Verbindungen der Formel (V) sind bekannte Synthesechemikalien.

Das erfindungsgemäße Verfahren (b) wird vorzugsweise in Gegenwart eines Verdünnungsmittels durchgeführt. Neben den oben für Verfahren (a) angegebenen Verdünnungsmitteln kommen insbesondere auch Alkohole, wie Methanol, Ethanol, Propanol oder Isopropanol für Verfahren (b) als Verdünnungmittel in Betracht.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden substituierten Aminopyrazoline sind durch die Formel (VI) allgemein definiert.

In Formel (VI) haben $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$, X, Y und Z angegeben wurden.

Beispiele für die Ausgangsstoffe der Formel (VI) sind in der nachstehenden Tabelle 5 aufgeführt.

Tabelle 5

| Beispiele für die Verbindungen der Formel (VI) | | | |
|---|---|---|---|
| $R^4$ | X | Y | Z |
| $CH_3$ | H | CH | C-$CH_3$ |
| $CH_3$ | N | CH | C-$OCH_3$ |
| $CH_3$ | N | CH | C-$OC_2H_5$ |
| $OCH_3$ | N | CH | C-$OCH_3$ |
| $OCH_3$ | N | CH | C-Cl |
| H | N | CH | C-$CH_3$ |
| $CF_3$ | N | CH | C-$OCH_3$ |
| $OCH_3$ | N | CH | C-$OCHF_2$ |
| $CH_3$ | N | CH | C-$OCHF_2$ |
| $OCHF_2$ | N | CH | C-$OCHF_2$ |
| $CH_3$ | N | N | C-$CH_3$ |
| $CH_3$ | N | N | C-$OCH_3$ |
| $OCH_3$ | N | N | C-$OCH_3$ |
| $C_2H_5$ | N | CH | C-$OCH_3$ |
| $C_2H_5$ | N | N | C-$OCH_3$ |

Die Ausgangsstoffe der Formel (VI) sind noch nicht aus der Literatur bekannt.

Man erhält die Verbindungen der Formel (VI), wenn man Hydrazinoazine der Formel (IX)

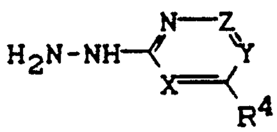

(IX)

in welcher

$R^4$, X, Y und Z die oben angegebenen Bedeutungen haben,

mit Acrylnitril in Gegenwart einer Base, wie z. B. Natrium- oder Kalium-methylat und/oder -ethylat, und in Gegenwart eines Verdünnungsmittels, wie z. B. Methanol und/oder Ethanol, bei Temperaturen zwischen 0 °C und 100 °C umsetzt.

Das erfindungsgemäße Verfahren (c) wird weiter unter Einsatz von Sulfonsäure-halogeniden bzw. -anhydriden (III) durchgeführt. Bezüglich dieser Ausgangsstoffe für Verfahren (c) gelten die oben bei der Beschreibung der Ausgangsstoffe für Verfahren (a) zu den Verbindungen der Formel (III) gemachten Angaben.

Das erfindungsgemäße Verfahren (c) wird vorzugsweise unter Verwendung von Verdünnungsmitteln durchgeführt.

Als Verdünnungsmittel kommen praktisch alle inerten organischen Lösungsmittel in Betracht, wie sie oben für das erfindungsgemäße Verfahren (a) angegeben sind.

Verfahren (c) wird gegebenenfalls in Gegenwart eines Säureakzeptors durchgeführt. Es kommen vor allem diejenigen Säurebindemittel in Betracht, die oben für Verfahren (a) angegeben sind.

Die Reaktionstemperaturen können bei dem erfindungsgemäßen Verfahren (c) in einem größeren Bereich variiert werden. Im allgemeinen arbeitet man bei Temperaturen zwischen -50 °C und +50 °C, vorzugsweise bei Temperaturen zwischen -40 °C und +40 °C.

Zur Durchführung des erfindungsgemäßen Verfahrens (c) setzt man je Mol Aminopyrazolin der Formel (VI) im allgemeinen zwischen 1 und 5 Mol, vorzugsweise zwischen 1 und 4 Mol, Sulfonsäurehalogenid bzw. Sulfonsäureanhydrid der Formel (III) ein. Falls doppelt sulfonylierte Verbindungen der Formel (I, $R^2$ = -$SO_2$-$R^1$) in einer Eintopfreaktion hergestellt werden sollen, sind je Mol Aminopyrazolin (VI) mindestens 2 Mol Sulfonsäurehalogenid bzw. -anhydrid (III) einzusetzen.

Die Reaktionskomponenten können in beliebiger Reihenfolge zusammengegeben werden.

In einer bevorzugten Ausführungsform des erfindungsgemäßen Verfahrens (c) werden die Ausgangsstoffe der Formeln (VI) und (III) bei Raumtemperatur mit einem Verdünnungsmittel verrührt, und in diese

Mischung wird - gegebenenfalls nach Abkühlen - der Säureakzeptor langsam eindosiert. Das Reaktionsgemisch wird dann - in sauerstoffhaltiger Atmosphäre - bis zum Ende der Umsetzung gerührt.

Die Aufarbeitung und (gegebenenfalls) die Desulfonylierung können wie oben für Verfahren (a) beschrieben durchgeführt werden.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z. B. Wasser, Methanol, Ethanol oder Aceton, und Zugabe einer geeigneten Säure bzw. Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als

Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser. Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); 4-Amino-benzolsulfonyl-methylcarbamat (ASULAM); 2-Chlor-4-ethylamino-6-iso propylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2,6-Dichlorbenzonitril (DICHLOBENIL); 2-[(2-Chlorphenyl)-methyl]-4,4-dimethylisoxazolidin-3-on (DIMETHAZONE); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); N,N-Dimethyl-N′-(3-trifluormethylphenyl)-harnstoff (FLUOMETURON); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); N-Phosphonomethyl-glycin (GLYPHOSATE); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N′-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2- yloxy)-acetanilid (MEFENACET); 2-Ethyl-6-methyl-N-(1-methyl-2-methoxyethyl)-chloracetanilid (METOLACHLOR); N-(1-Ethylpropyl)-3,4-dimethyl-2,6-dinitroanilin (PENDIMETHALIN); 4-Amino-3,5,6-trichlorpyridin-2-carbonsäure (PICLORAM); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); Methyl-2-{[(4,6-dimethyl-2-pyrimidinyl)-aminocarbonyl]-aminosulfonyl}-benzoat (SULFOMETURON) und 2,6-Dinitro-4-trifluormethyl-N.N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1 (IA-1)

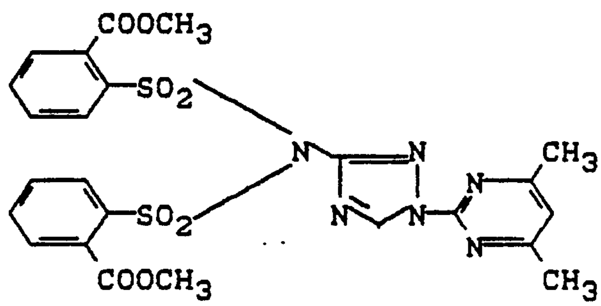

(Verfahren (a))

Zu einer Lösung von 4,75 g (0,025 Mol) 1-(4,6-Dimethylpyrimidin-2-yl)-3-amino-triazol in 100 ml Methylenchlorid werden 26,1 g (0,1 Mol) 2-Chlorsulfonylbenzoesäure-methylester zugegeben und nach dem Abkühlen auf -5 °C, bei -5 °C bis 0 °C, werden 11,2 g (0,1 Mol) Diazabicyclo[2.2.2]octan, gelöst in 40 ml Methylenchlorid, zugetropft. Das Reaktionsgemisch wird 2 Tage bei Raumtemperatur (20 °C) gerührt, dann mit Wasser gewaschen; die Methylenchloridphase wird abgetrennt, über Natriumsulfat getrocknet, filtriert und eingeengt. Der ölige Rückstand läßt sich mit Essigester auskristallisieren.

Nach dem Absaugen und Trocknen verbleiben 6,6 g (45 % der Theorie) 1-(4,6-Dimethyl-pyrimidin-2-yl)-3-(N,N-bis-(2-methoxycarbonyl-phenylsulfonyl))-aminotriazol als farblose Kristalle vom Schmelzpunkt 209 °C - 212 °C.

### Beispiel 2 (IA-2)

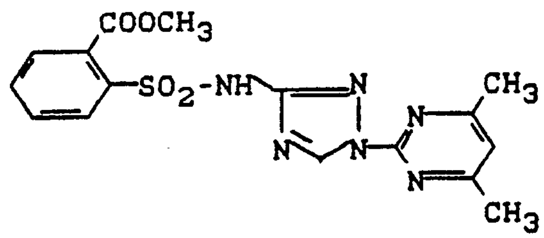

(Verfahren (a))

1,17 g (0,002 Mol) 1-(4,6-Dimethyl-pyrimidin-2-yl)-3-(N,N-bis-(2-methoxycarbonyl-phenylsulfonyl))-aminotriazol werden in einer Mischung aus 10 ml 25 %igem Ammoniakwasser und 10 ml Ethanol über Nacht bei Rückflußtemperatur (ca. 70 °C) gerührt. Dabei geht die Substanz vollständig in Lösung. Der Ansatz wird eingeengt und der Rückstand mit 30 ml Wasser verdünnt. Man stellt mit Salzsäure auf pH 3, saugt das Produkt ab, wäscht und trocknet es.

Man erhält 0,7 g (90 % der Theorie) 1-(4,6-Dimethyl-pyrimidin-2-yl)-3-(2-methoxycarbonyl-phenylsulfonyl)aminotriazol in Form farbloser Kristalle vom Schmelzpunkt 215 °C - 217 °C.

Analog zu den Beispielen 1 und 2 und entsprechend der allgemeinen Beschreibung der erfindungsgemäßen Verfahren können die in der nachstehenden Tabelle 6 aufgeführten Verbindungen der Formel (I) - welche durch die Formeln (IA), (IB) und (IC) genauer spezifiziert ist - hergestellt werden:

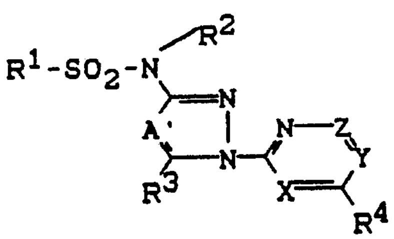

(IA)

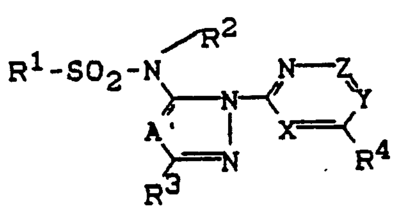

(IB)

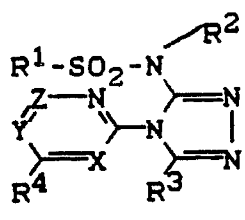

(IC)

(In der nachfolgenden Tabelle 6 wird an Stelle von A′ jeweils A angegeben).

**Tabelle 6**: Beispiele für die Verbindungen der Formel (I)

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-3 | N | 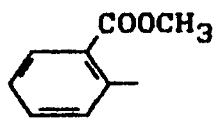 | Na | H | $CH_3$ | N | CH | $C-CH_3$ | 111 |
| IA-4 | N | 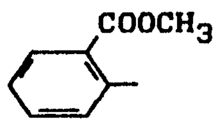 | K | H | $CH_3$ | N | CH | $C-CH_3$ | |
| IA-5 | N | 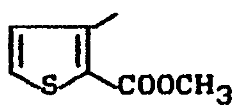 | 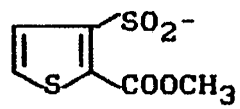 | H | $CH_3$ | N | CH | $C-CH_3$ | 165 |
| IA-6 | N | 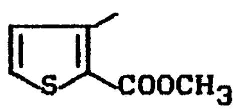 | H | H | $CH_3$ | N | CH | $C-CH_3$ | 202 |
| IA-7 | N | 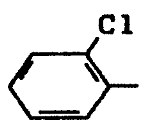 | 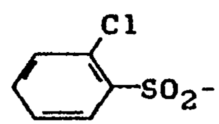 | H | $CH_3$ | N | CH | $C-CH_3$ | 229 |
| IA-8 | N | 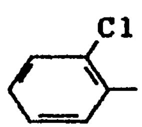 | H | H | $CH_3$ | N | CH | $C-CH_3$ | 240 |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-9 | N | 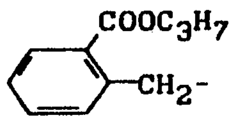 | 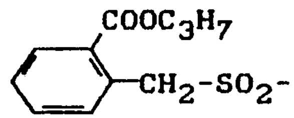 | H | $CH_3$ | N | CH | $C-CH_3$ | 152 |
| IA-10 | N | 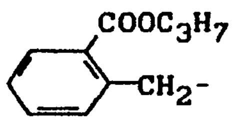 | H | H | $CH_3$ | N | CH | $C-CH_3$ | 190 |
| IB-11 | N | 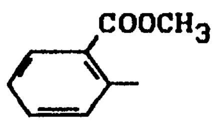 | H | H | $CH_3$ | N | CH | $C-CH_3$ | 204 |
| IA-12 | N | 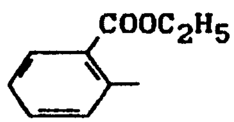 | 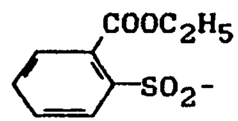 | H | $CH_3$ | N | CH | $C-CH_3$ | |
| IA-13 | N | 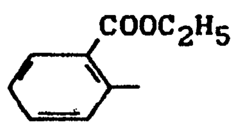 | H | H | $CH_3$ | N | CH | $C-CH_3$ | 89 |
| IA-14 | N | 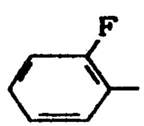 | 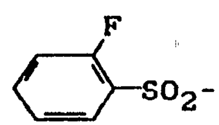 | H | $CH_3$ | N | CH | $C-CH_3$ | 246 |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-15 | N | 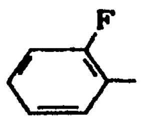 | H | H | $CH_3$ | N | CH | $C-CH_3$ | 269 |
| IA-16 | N | 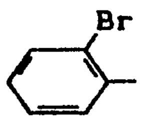 | 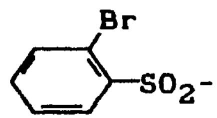 | H | $CH_3$ | N | CH | $C-CH_3$ | |
| IA-17 | N | 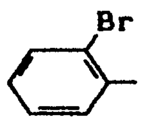 | H | H | $CH_3$ | N | CH | $C-CH_3$ | |
| IA-18 | N | 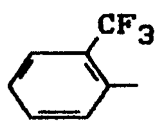 | 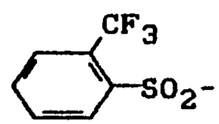 | H | $CH_3$ | N | CH | $C-CH_3$ | |
| IA-19 | N | 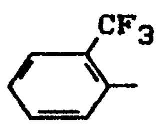 | H | H | $CH_3$ | N | CH | $C-CH_3$ | |
| IA-20 | N | 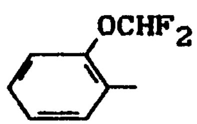 | 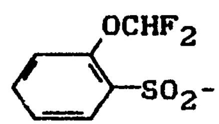 | H | $CH_3$ | N | CH | $C-CH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-21 | N | 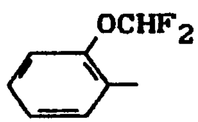 | 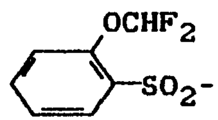 | H | $CH_3$ | N | N | $C-CH_3$ | |
| IA-22 | N | 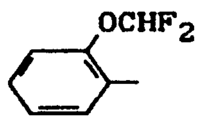 | H | H | $CH_3$ | N | CH | $C-CH_3$ | 230 |
| IA-23 | N | 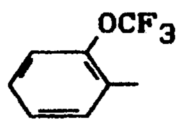 | 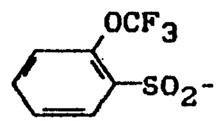 | H | $CH_3$ | N | CH | $C-CH_3$ | 202 |
| IA-24 | N | 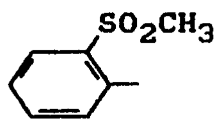 | 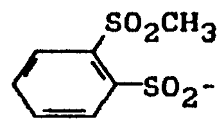 | H | $CH_3$ | N | CH | $C-CH_3$ | |
| IA-25 | N | 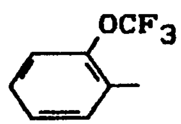 | H | H | $CH_3$ | N | CH | $C-CH_3$ | 211 |
| IA-26 | N | 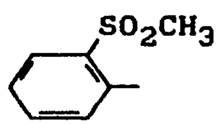 | H | H | $CH_3$ | N | CH | $C-CH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-27 | N | 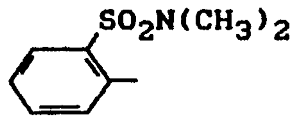 | H | H | $CH_3$ | N | CH | $C-CH_3$ | 203 |
| IA-28 | N | 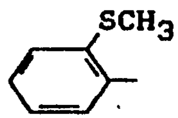 | H | H | $CH_3$ | N | CH | $C-CH_3$ | |
| IA-29 | N | 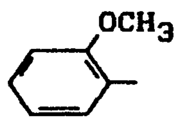 | H | H | $CH_3$ | N | CH | $C-CH_3$ | |
| IA-30 | N | 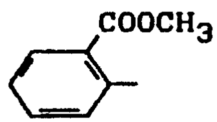 | 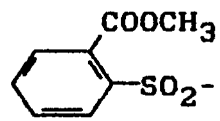 | H | $CH_3$ | N | CH | $C-OCH_3$ | |
| IA-31 | N | 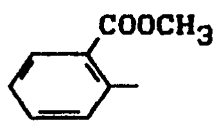 | H | H | $CH_3$ | N | CH | $C-OCH_3$ | 225 |
| IA-32 | N | 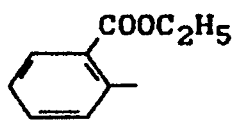 | 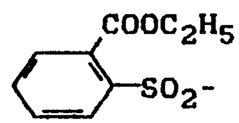 | H | $CH_3$ | N | CH | $C-OCH_3$ | 214 |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-33 | N | 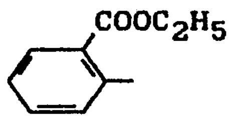 | H | H | $CH_3$ | N | CH | $C-OCH_3$ | 167 |
| IA-34 | N | 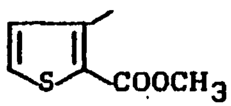 | 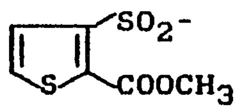 | H | $CH_3$ | N | CH | $C-OCH_3$ | |
| IA-35 | N | 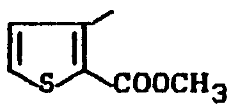 | H | H | $CH_3$ | N | CH | $C-OCH_3$ | |
| IA-36 | N | 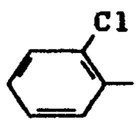 | H | H | $CH_3$ | N | CH | $C-OCH_3$ | |
| IA-37 | N | 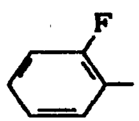 | H | H | $CH_3$ | N | CH | $C-OCH_3$ | |
| IA-38 | N | 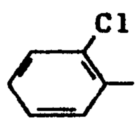 | 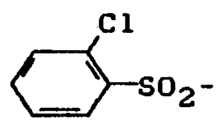 | H | $CH_3$ | N | CH | $C-OCH_3$ | |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-39 | N | 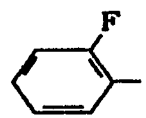 | 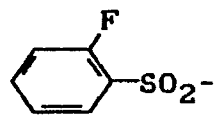 | H | $CH_3$ | N | CH | $C-OCH_3$ | |
| IA-40 | N | 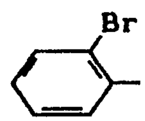 | 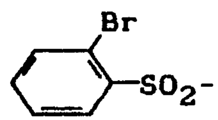 | H | $CH_3$ | N | CH | $C-OCH_3$ | |
| IA-41 | N | 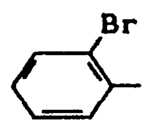 | H | H | $CH_3$ | N | CH | $C-OCH_3$ | |
| IA-42 | N | 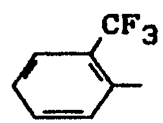 | 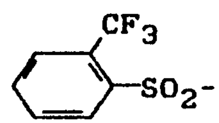 | H | $CH_3$ | N | CH | $C-OCH_3$ | |
| IA-43 | N | 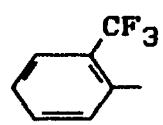 | H | H | $CH_3$ | N | CH | $C-OCH_3$ | |
| IA-44 | N | 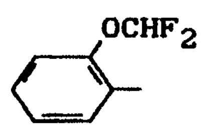 | 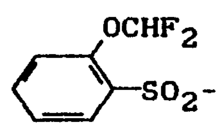 | H | $CH_3$ | N | CH | $C-OCH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-45 | N | 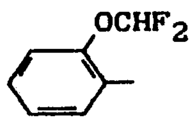 | H | H | $CH_3$ | N | CH | $C-OCH_3$ | 223 |
| IA-46 | N | 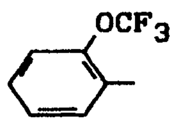 | 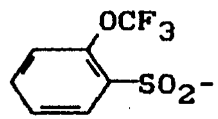 | H | $CH_3$ | N | CH | $C-OCH_3$ | |
| IA-47 | N | 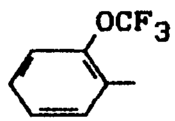 | H | H | $CH_3$ | N | CH | $C-OCH_3$ | 244 |
| IA-48 | N | 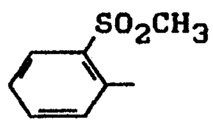 | H | H | $CH_3$ | N | CH | $C-OCH_3$ | |
| IA-49 | N | 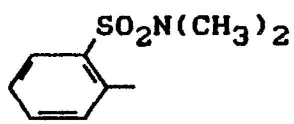 | H | H | $CH_3$ | N | CH | $C-OCH_3$ | 168 |
| IA-50 | N | 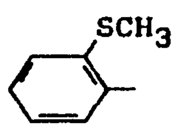 | H | H | $CH_3$ | N | CH | $C-OCH_3$ | |

<u>**Tabelle 6**</u> **- Fortsetzung**

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-51 | N | 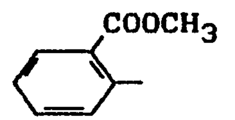 | 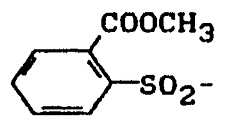 | H | $OCH_3$ | N | CH | $C-OCH_3$ | 208 |
| IA-52 | N | 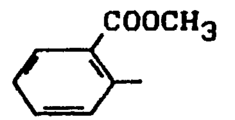 | H | H | $OCH_3$ | N | CH | $C-OCH_3$ | 203 |
| IA-53 | N | 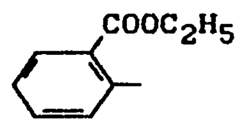 | 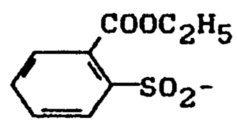 | H | $OCH_3$ | N | CH | $C-OCH_3$ | |
| IA-54 | N | 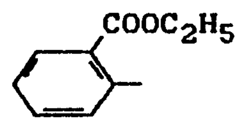 | H | H | $OCH_3$ | N | CH | $C-OCH_3$ | |
| IA-55 | N | 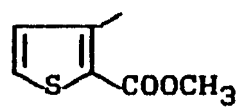 | 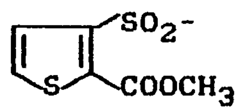 | H | $OCH_3$ | N | CH | $C-OCH_3$ | 195 |
| IA-56 | N | 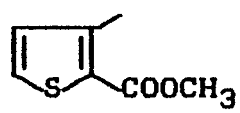 | H | H | $OCH_3$ | N | CH | $C-OCH_3$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-57 | N | 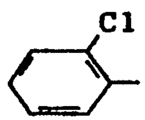 | 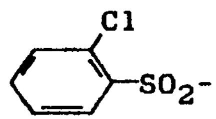 | H | $OCH_3$ | N | CH | $C-OCH_3$ | |
| IA-58 | N | 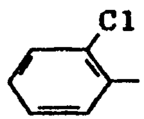 | H | H | $OCH_3$ | N | CH | $C-OCH_3$ | |
| IA-59 | N | 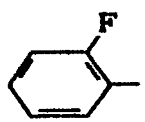 | 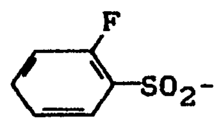 | H | $OCH_3$ | N | CH | $C-OCH_3$ | |
| IA-60 | N | 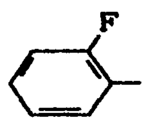 | H | H | $OCH_3$ | N | CH | $C-OCH_3$ | |
| IA-61 | N | 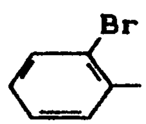 | 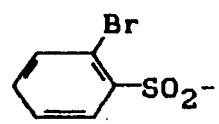 | H | $OCH_3$ | N | CH | $C-OCH_3$ | |
| IA-62 | N | 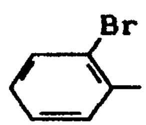 | H | H | $OCH_3$ | N | CH | $C-OCH_3$ | |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-63 | N | 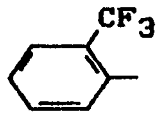 | 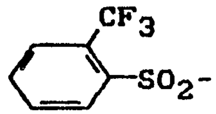 | H | $OCH_3$ | N | CH | $C-OCH_3$ | |
| IA-64 | N | 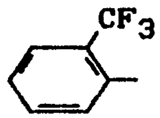 | H | H | $OCH_3$ | N | CH | $C-OCH_3$ | |
| IA-65 | N | 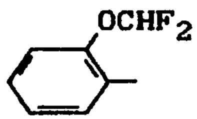 | 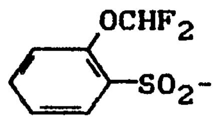 | H | $OCH_3$ | N | CH | $C-OCH_3$ | |
| IA-66 | N | 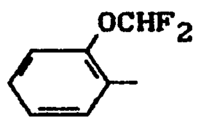 | H | H | $OCH_3$ | N | CH | $C-OCH_3$ | 194 |
| IA-67 | N | 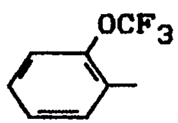 | 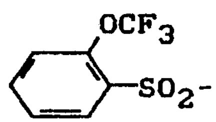 | H | $OCH_3$ | N | CH | $C-OCH_3$ | 190 |
| IA-68 | N | 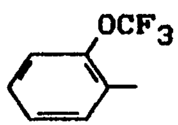 | H | H | $OCH_3$ | N | CH | $C-OCH_3$ | 233 |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-69 | N | 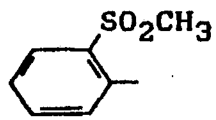 | H | H | $OCH_3$ | N | CH | $C-OCH_3$ | |
| IA-70 | N | 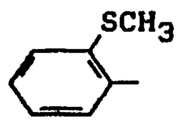 | H | H | $OCH_3$ | N | CH | $C-OCH_3$ | |
| IA-71 | N | 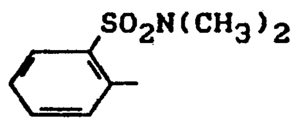 | H | H | $OCH_3$ | N | CH | $C-OCH_3$ | 203 |
| IA-72 | N | 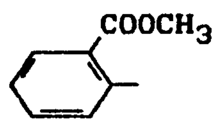 | 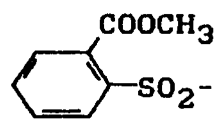 | H | $CF_3$ | N | CH | $C-OCH_3$ | |
| IA-73 | N | 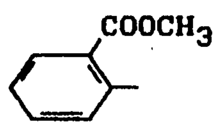 | 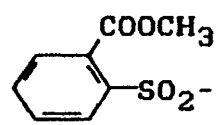 | H | $OCH_3$ | N | CH | C-Cl | |
| IA-74 | N | 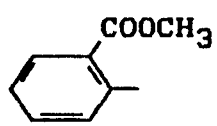 | H | H | $CF_3$ | N | CH | $C-OCH_3$ | |

<u>**Tabelle 6**</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-75 | N | 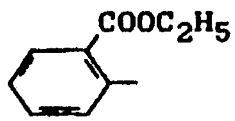 | 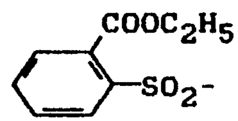 | H | $CF_3$ | N | CH | $C-OCH_3$ | |
| IA-76 | N | 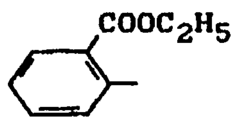 | H | H | $CF_3$ | N | CH | $C-OCH_3$ | |
| IA-77 | N | 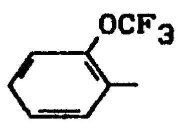 | H | H | $CF_3$ | N | CH | $C-OCH_3$ | |
| IA-78 | N | 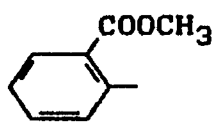 | H | H | $OCH_3$ | N | CH | C-Cl | |
| IA-79 | N | 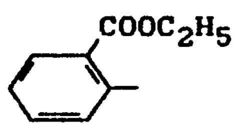 | 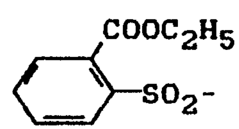 | H | $OCH_3$ | N | CH | C-Cl | |
| IA-80 | N | 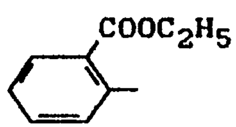 | H | H | $OCH_3$ | N | CH | C-Cl | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-81 | N | 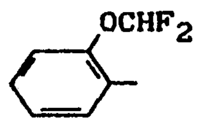 | H | H | $OCH_3$ | N | CH | C-Cl | 226 |
| IA-82 | N | 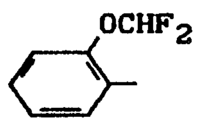 | 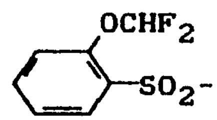 | H | $OCH_3$ | N | CH | C-Cl | |
| IA-83 | N | 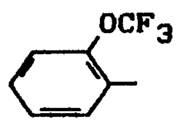 | 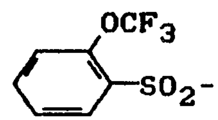 | H | $OCH_3$ | N | CH | C-Cl | |
| IA-84 | N | 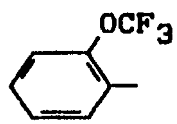 | H | H | $OCH_3$ | N | CH | C-Cl | |
| IA-85 | N | 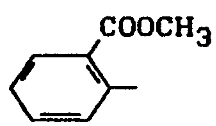 | 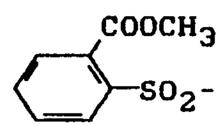 | H | $OCH_3$ | N | CH | $C-OCHF_2$ | |
| IA-86 | N | 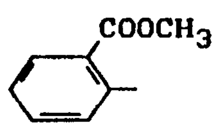 | H | H | $OCH_3$ | N | CH | $C-OCHF_2$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-87 | N | 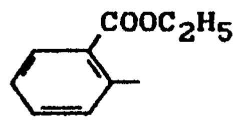 | 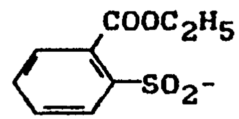 | H | $OCH_3$ | N | CH | C-$OCHF_2$ | |
| IA-88 | N | 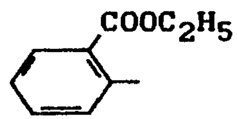 | H | H | $OCH_3$ | N | CH | C-$OCHF_2$ | |
| IA-89 | N | 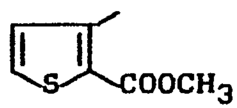 | 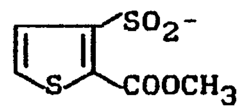 | H | $OCH_3$ | N | CH | C-$OCHF_2$ | |
| IA-90 | N | 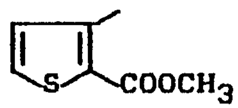 | H | H | $OCH_3$ | N | CH | C-$OCHF_2$ | |
| IA-91 | N | 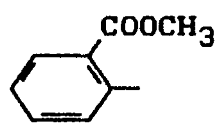 | 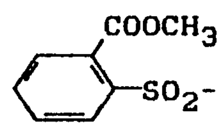 | H | $OCHF_2$ | N | CH | C-$OCHF_2$ | |
| IA-92 | N | 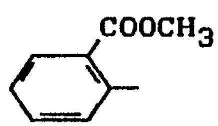 | H | H | $OCHF_2$ | N | CH | C-$OCHF_2$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-93 | N | 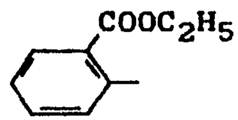 | 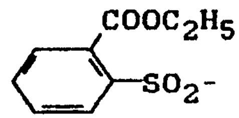 | H | $OCHF_2$ | N | CH | $C-OCHF_2$ | |
| IA-94 | N | 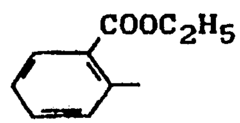 | H | H | $OCHF_2$ | N | CH | $C-OCHF_2$ | |
| IA-95 | N | 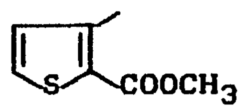 | 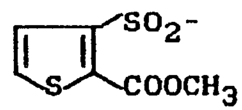 | H | $OCHF_2$ | N | CH | $C-OCHF_2$ | |
| IA-96 | N | 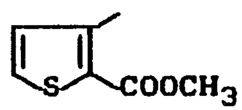 | H | H | $OCHF_2$ | N | CH | $C-OCHF_2$ | |
| IA-97 | N | 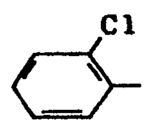 | 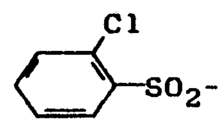 | H | $OCHF_2$ | N | CH | $C-OCHF_2$ | |
| IA-98 | N | 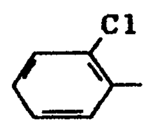 | H | H | $OCHF_2$ | N | CH | $C-OCHF_2$ | |

Tabelle 6 - Fortsetzung

| Bsp.-Nr. | A | $R^1$ | $R^2$ | $R^3$ | $R^4$ | X | Y | Z | Schmelzpunkt (° C) |
|---|---|---|---|---|---|---|---|---|---|
| IA-99 | N | 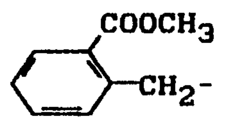 | 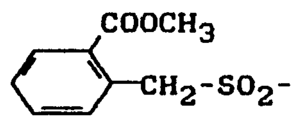 | H | $OCH_3$ | N | CH | $C-OCH_3$ | |
| IA-100 | N | 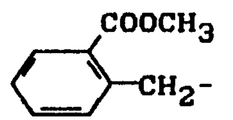 | H | H | $OCH_3$ | N | CH | $C-OCH_3$ | |
| IA-101 | N | 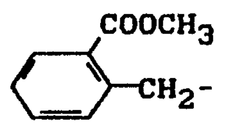 | 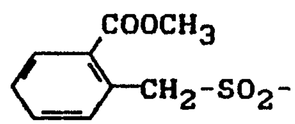 | H | $OCH_3$ | N | CH | C-Cl | |
| IA-102 | N | 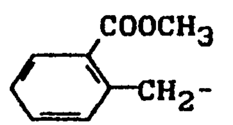 | H | H | $OCH_3$ | N | CH | C-Cl | |
| IA-103 | N | 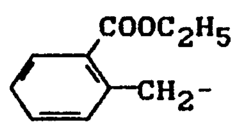 | 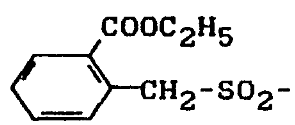 | H | $OCH_3$ | N | CH | $C-OCH_3$ | |
| IA-104 | N | 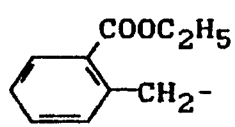 | H | H | $OCH_3$ | N | CH | $C-OCH_3$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-105 | N | 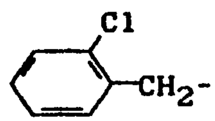 | 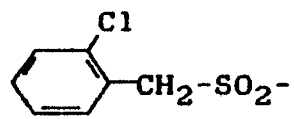 | H | $OCH_3$ | N | CH | $C-OCH_3$ | |
| IA-106 | N | 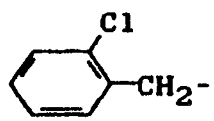 | H | H | $OCH_3$ | N | CH | $C-OCH_3$ | |
| IA-107 | N | 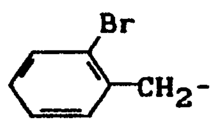 | 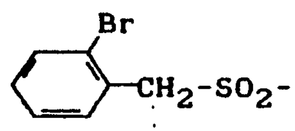 | H | $OCH_3$ | N | CH | $C-OCH_3$ | |
| IA-108 | N | 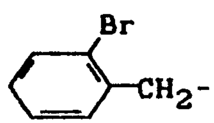 | 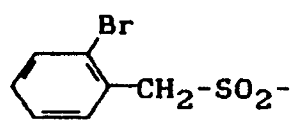 | H | $OCH_3$ | N | CH | $C-OCHF_2$ | |
| IA-109 | N | 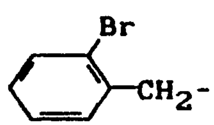 | H | H | $OCH_3$ | N | CH | $C-OCH_3$ | |
| IA-110 | N | 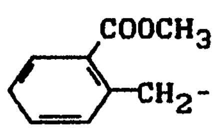 | 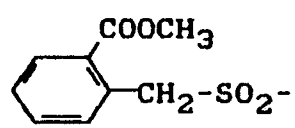 | H | $OCH_3$ | N | CH | $C-OCHF_2$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-111 | N | 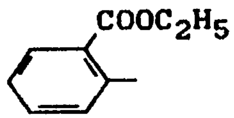 | H | H | $CH_3$ | N | N | $C-CH_3$ | |
| IA-112 | N | 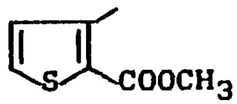 | 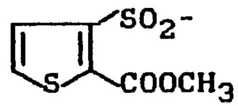 | H | $CH_3$ | N | N | $C-CH_3$ | |
| IA-113 | N | 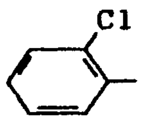 | 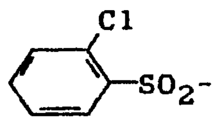 | H | $CH_3$ | N | N | $C-CH_3$ | |
| IA-114 | N | 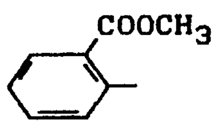 | 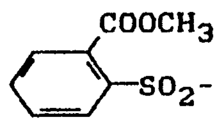 | H | $CH_3$ | N | N | $C-OCH_3$ | |
| IA-115 | N | 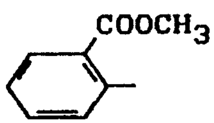 | H | H | $CH_3$ | N | N | $C-OCH_3$ | |
| IA-116 | N | 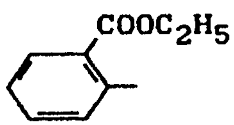 | 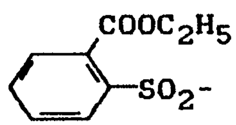 | H | $CH_3$ | N | N | $C-OCH_3$ | |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-117 | N | 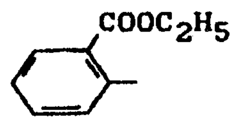 | H | H | $CH_3$ | N | N | $C-OCH_3$ | |
| IA-118 | N | 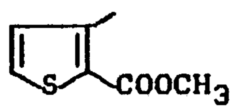 | 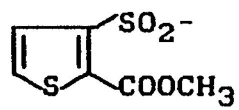 | H | $CH_3$ | N | N | $C-OCH_3$ | |
| IA-119 | N | 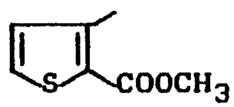 | H | H | $CH_3$ | N | N | $C-OCH_3$ | |
| IA-120 | N | 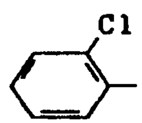 | Na | H | $CH_3$ | N | N | $C-OCH_3$ | |
| IA-121 | N | 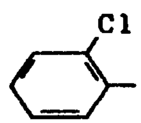 | 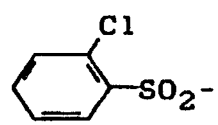 | H | $CH_3$ | N | N | $C-OCH_3$ | |
| IA-122 | N | 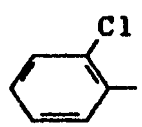 | H | H | $CH_3$ | N | N | $C-OCH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-123 | N | 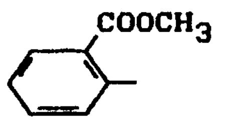 | 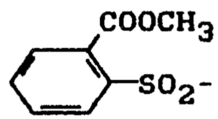 | H | $OCH_3$ | N | N | $C-OCH_3$ | 200 |
| IA-124 | N | 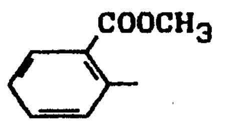 | H | H | $OCH_3$ | N | N | $C-OCH_3$ | |
| IA-125 | N | 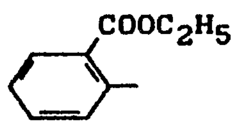 | 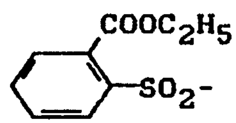 | H | $OCH_3$ | N | N | $C-OCH_3$ | |
| IA-126 | N | 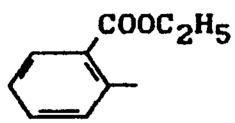 | H | H | $OCH_3$ | N | N | $C-OCH_3$ | |
| IA-127 | N | 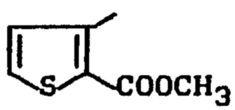 | 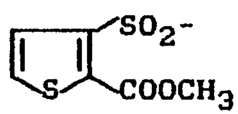 | H | $OCH_3$ | N | N | $C-OCH_3$ | |
| IA-128 | N | 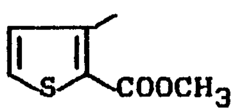 | H | H | $OCH_3$ | N | N | $C-OCH_3$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-129 | N | 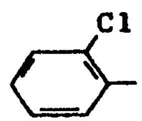 | 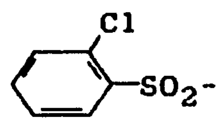 | H | $OCH_3$ | N | N | $C-OCH_3$ | 184 |
| IA-130 | N | 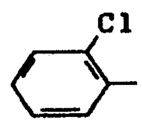 | H | H | $OCH_3$ | N | N | $C-OCH_3$ | 225 |
| IA-131 | N | 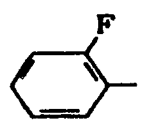 | 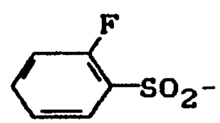 | H | $OCH_3$ | N | N | $C-OCH_3$ | |
| IA-132 | N | 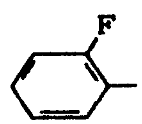 | H | H | $OCH_3$ | N | N | $C-OCH_3$ | |
| IA-133 | N | 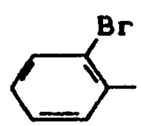 | 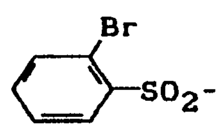 | H | $OCH_3$ | N | N | $C-OCH_3$ | 232 |
| IA-134 | N | 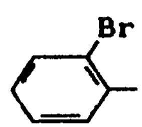 | H | H | $OCH_3$ | N | N | $C-OCH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-135 | N | 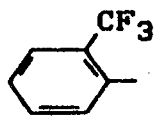 | 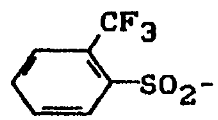 | H | $OCH_3$ | N | N | $C-OCH_3$ | |
| IA-136 | N | 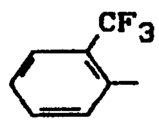 | H | H | $OCH_3$ | N | N | $C-OCH_3$ | |
| IA-137 | N | 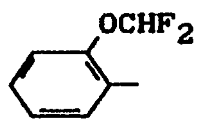 | 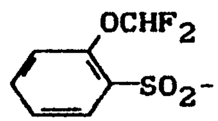 | H | $OCH_3$ | N | N | $C-OCH_3$ | 198 |
| IA-138 | N | 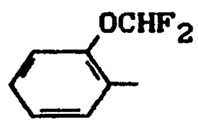 | H | H | $OCH_3$ | N | N | $C-OCH_3$ | |
| IA-139 | N | 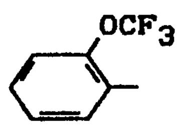 | 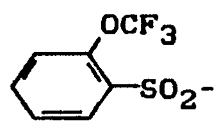 | H | $OCH_3$ | N | N | $C-OCH_3$ | 250 |
| IA-140 | N | 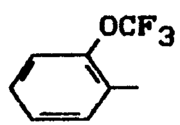 | H | H | $OCH_3$ | N | N | $C-OCH_3$ | 205 |

Tabelle 6 - Fortsetzung

| Bsp.-Nr. | A | $R^1$ | $R^2$ | $R^3$ | $R^4$ | X | Y | Z | Schmelz-punkt (°C) |
|---|---|---|---|---|---|---|---|---|---|
| IA-141 | N | 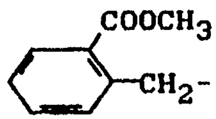 | 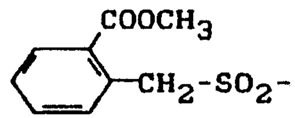 | H | $OCHF_2$ | N | CH | $C-OCHF_2$ | |
| IA-142 | N | 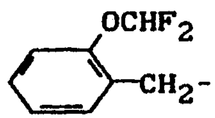 | 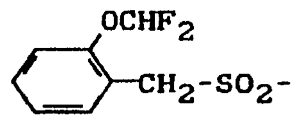 | H | $OCH_3$ | N | CH | $C-OCH_3$ | |
| IA-143 | N | 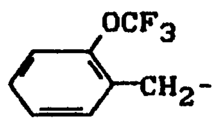 | 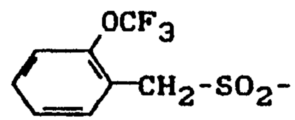 | H | $OCH_3$ | N | CH | $C-OCH_3$ | |
| IA-144 | N | 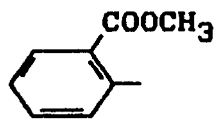 | 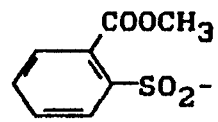 | H | $CH_3$ | N | N | $C-CH_3$ | |
| IA-145 | N | 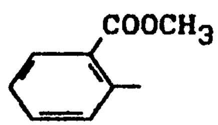 | H | H | $CH_3$ | N | N | $C-CH_3$ | |
| IA-146 | N | 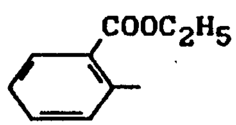 | 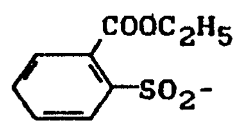 | H | $CH_3$ | N | N | $C-CH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-147 | N | 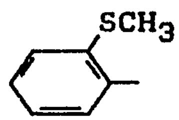 | 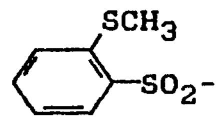 | H | $OCH_3$ | N | N | $C-OCH_3$ | |
| IA-148 | N | 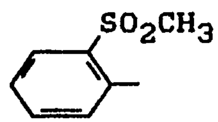 | 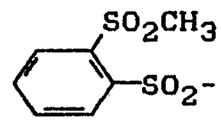 | H | $OCH_3$ | N | N | $C-OCH_3$ | |
| IA-149 | N | 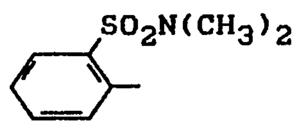 | 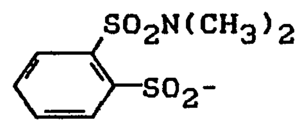 | H | $OCH_3$ | N | N | $C-OCH_3$ | |
| IA-150 | N | 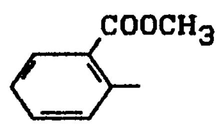 | 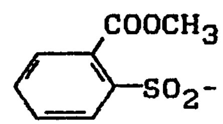 | H | $OCH_3$ | N | N | C-Cl | |
| IA-151 | N | 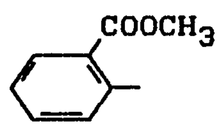 | 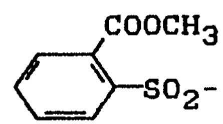 | H | $C_2H_5$ | N | N | $C-OCH_3$ | |
| IA-152 | N | 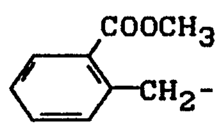 | 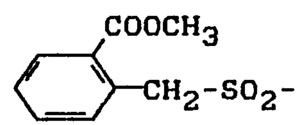 | H | $OCH_3$ | N | N | $C-OCH_3$ | |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-153 | N | 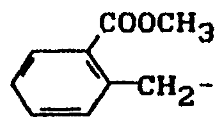 | H | H | $OCH_3$ | N | N | $C-OCH_3$ | |
| IA-154 | N | 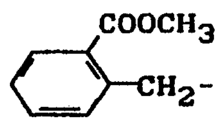 | H | H | $OCH_3$ | N | N | $C-CH_3$ | |
| IA-155 | N | 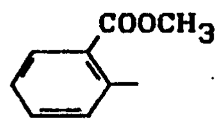 | 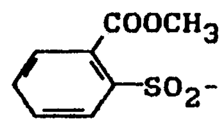 | H | $OCH_3$ | N | N | $C-NHC_2H_5$ | |
| IA-156 | N | 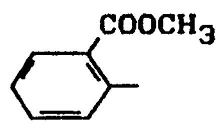 | 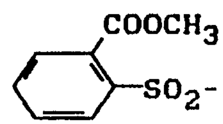 | H | $OC_2H_5$ | N | N | $C-NHCH_3$ | |
| IA-157 | N | 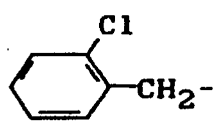 | 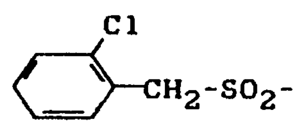 | H | $OCH_3$ | N | N | $C-OCH_3$ | |
| IA-158 | N | 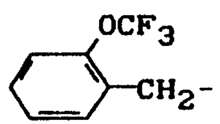 | 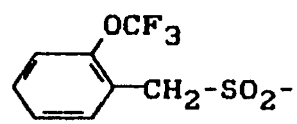 | H | $OCH_3$ | N | N | $C-OCH_3$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-159 | N | 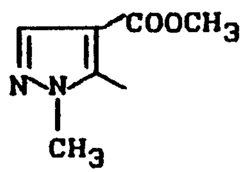 | 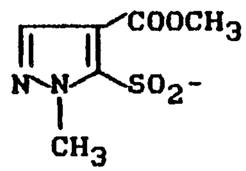 | H | $OCH_3$ | N | CH | $C-OCH_3$ | |
| IA-160 | N | 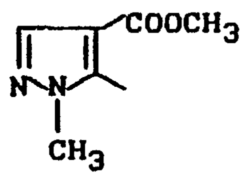 | H | H | $OCH_3$ | N | CH | $C-OCH_3$ | |
| IA-161 | N | 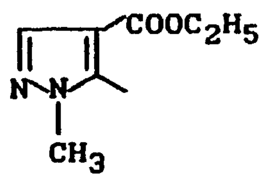 | 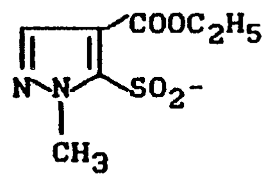 | H | $OCH_3$ | N | CH | $C-OCH_3$ | |
| IA-162 | N | 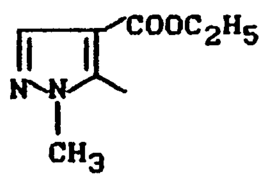 | H | H | $OCH_3$ | N | CH | $C-OCH_3$ | |
| IA-163 | N | 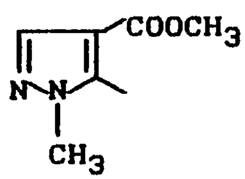 | 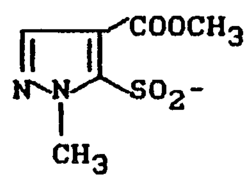 | H | $OCH_3$ | N | N | $C-OCH_3$ | |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-164 | N | 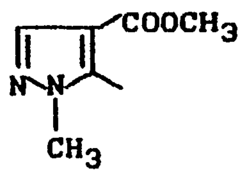 | H | H | $OCH_3$ | N | N | $C-OCH_3$ | |
| IA-165 | N | 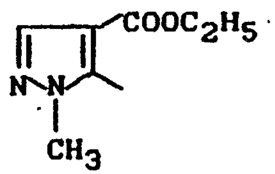 | 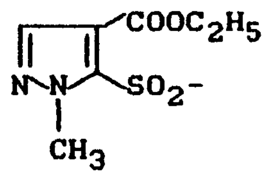 | H | $OCH_3$ | N | N | $C-OCH_3$ | |
| IA-166 | N | 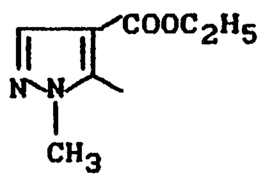 | H | H | $OCH_3$ | N | N | $C-OCH_3$ | |
| IA-167 | N | 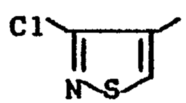 | 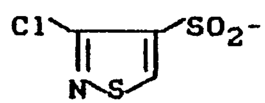 | H | $OCH_3$ | N | N | $C-OCH_3$ | |
| IA-168 | N | 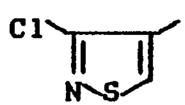 | 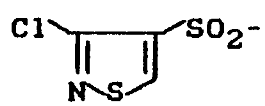 | H | $CH_3$ | N | CH | $C-CH_3$ | 217 |
| IA-169 | N | 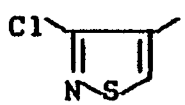 | H | H | $CH_3$ | N | CH | $C-CH_3$ | 252 |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-170 | N | 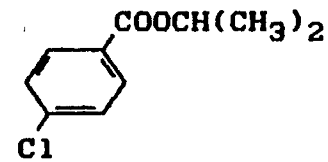 | H | H | $OCH_3$ | N | N | $C-OCH_3$ | |
| IA-171 | N | 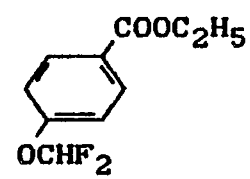 | H | H | $OCH_3$ | N | CH | $C-OCH_3$ | |
| IA-172 | N | 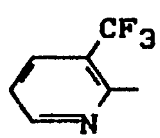 | H | H | $OCH_3$ | N | CH | $C-OCH_3$ | |
| IA-173 | N | 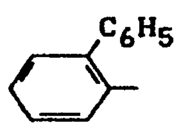 | H | H | $OCH_3$ | N | CH | $C-OCH_3$ | |
| IA-174 | N | 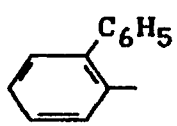 | H | H | $OCH_3$ | N | N | $C-OCH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-175 | N | 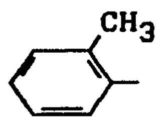 | H | H | $OCH_3$ | N | N | $C-OCH_3$ | |
| IA-176 | N | 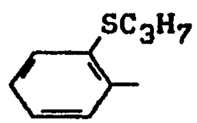 | H | H | $OCH_3$ | N | N | $C-OCH_3$ | |
| IA-177 | N | 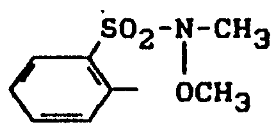 | H | H | $OCH_3$ | N | N | $C-OCH_3$ | |
| IA-178 | N | 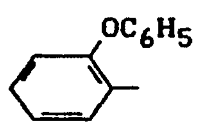 | H | H | $OCH_3$ | N | N | $C-OCH_3$ | |
| IA-179 | N | 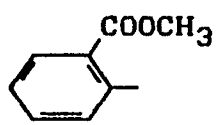 | H | $CH_3$ | $CH_3$ | N | CH | $C-CH_3$ | 208 |
| IA-180 | N | 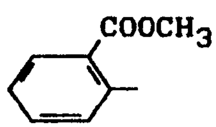 | H | $OCH_3$ | $CH_3$ | N | CH | $C-CH_3$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-181 | N | 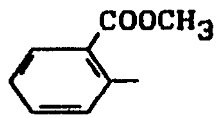 | H | $SCH_3$ | $CH_3$ | N | CH | $C-CH_3$ | |
| IB-182 | N | 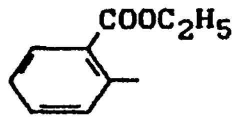 | H | H | $CH_3$ | N | CH | $C-CH_3$ | |
| IB-183 | N | 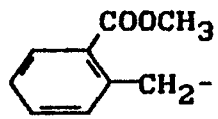 | H | H | $OCH_3$ | N | CH | $C-OCH_3$ | |
| IB-184 | N | 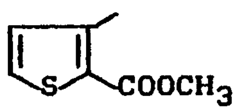 | H | H | $OCH_3$ | N | N | $C-OCH_3$ | |
| IB-185 | N | 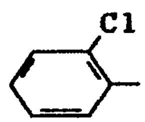 | H | H | $CH_3$ | N | N | $C-OCH_3$ | |
| IB-186 | N | 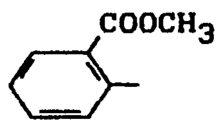 | H | H | $CH_3$ | N | N | $C-OCH_3$ | |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IB-187 | N | 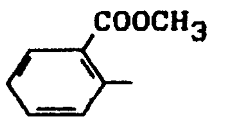 | H | H | $OCH_3$ | N | N | $C-OCH_3$ | |
| IB-188 | N | 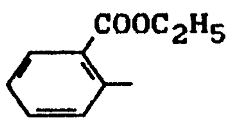 | H | H | $OCH_3$ | N | CH | C-Cl | |
| IB-189 | N | 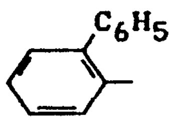 | H | H | $OCH_3$ | N | CH | $C-OCH_3$ | |
| IB-190 | N | 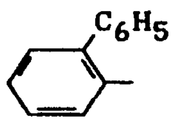 | H | H | $OCH_3$ | N | N | $C-OCH_3$ | |
| IB-191 | N | 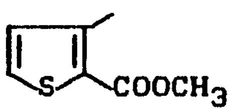 | H | H | $CH_3$ | N | N | $C-OCH_3$ | |

Tabelle 6 - Fortsetzung

| Bsp.- Nr. | A | $R^1$ | $R^2$ | $R^3$ | $R^4$ | X | Y | Z | Schmelz- punkt (° C) |
|---|---|---|---|---|---|---|---|---|---|
| IC-192 | N | 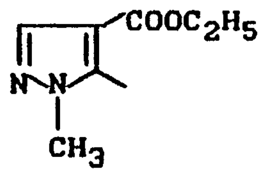 | H | H | $CH_3$ | N | CH | $C-CH_3$ | 206 |
| IA-193 | N | 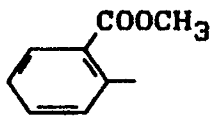 | 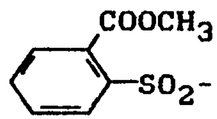 | H | $OC_2H_5$ | N | N | $C-OC_2H_5$ | 161 |
| IA-194 | N | 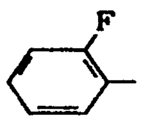 | 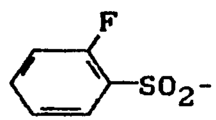 | H | $OC_2H_5$ | N | N | $C-OC_2H_5$ | 204 |
| IA-195 | N | 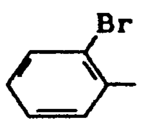 | 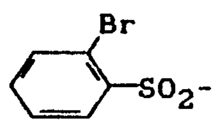 | H | $OC_2H_5$ | N | N | $C-OC_2H_5$ | 206 |
| IA-196 | N | 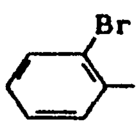 | H | H | $OC_2H_5$ | N | N | $C-OC_2H_5$ | 190 |

<u>Tabelle 6</u> - Fortsetzung

| Bsp.-Nr. | A | $R^1$ | $R^2$ | $R^3$ | $R^4$ | X | Y | Z | Schmelzpunkt (° C) |
|---|---|---|---|---|---|---|---|---|---|
| IA-197 | N | 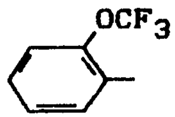 | 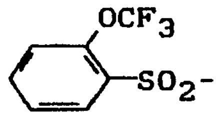 | H | $OC_2H_5$ | N | N | $C-OC_2H_5$ | 134 |
| IA-198 | N | 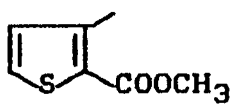 | 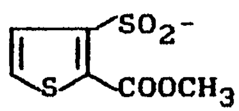 | H | $OC_2H_5$ | N | N | $C-OC_2H_5$ | 160 |
| IA-199 | N | 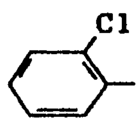 | 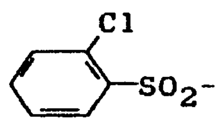 | H | $OC_2H_5$ | N | N | $C-OC_2H_5$ | 237 |
| IA-200 | N | 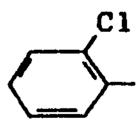 | 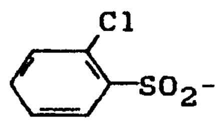 | H | $OCH_3$ | N | N | $C-OC_2H_5$ | 200 |
| IA-201 | N | 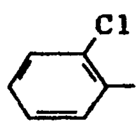 | H | H | $OCH_3$ | N | N | $C-OC_2H_5$ | 189 |
| IA-202 | N | 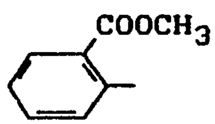 | 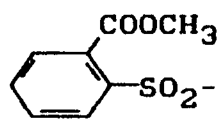 | H | $OCH_3$ | N | N | $C-OC_2H_5$ | 183 |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-203 | N | 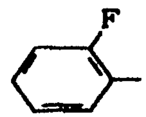 | 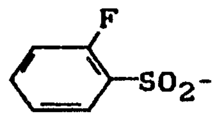 | H | $OCH_3$ | N | N | $C-OC_2H_5$ | 190 |
| IA-204 | N | 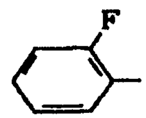 | H | H | $OCH_3$ | N | N | $C-OC_2H_5$ | 152 |
| IB-205 | N | 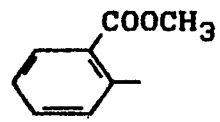 | 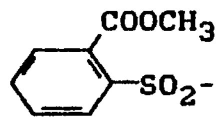 | $CH_3$ | H | N | CH | CH | 155 |
| IB-206 | N | 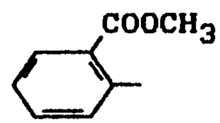 | 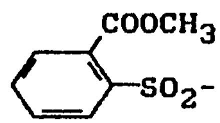 | $CH_3$ | $CH_3$ | N | CH | $C-CH_3$ | 195 |
| IB-207 | N | 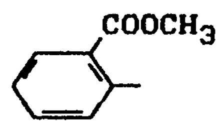 | H | $CH_3$ | H | N | CH | CH | 185 |
| IB-208 | N | 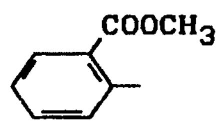 | H | $CH_3$ | $CH_3$ | N | CH | $C-CH_3$ | 202 |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-209 | CH | 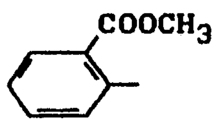 | 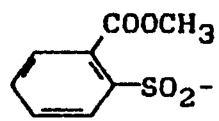 | H | $CH_3$ | N | CH | $C-CH_3$ | 177 |
| IA-210 | N | 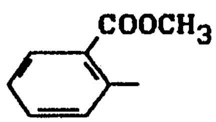 | 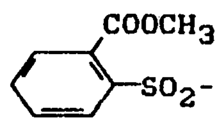 | H | H | N | CH | CH | 210 |
| IA-211 | N | 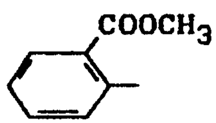 | H | H | H | N | CH | CH | 213 |
| IA-212 | CH | 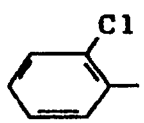 | 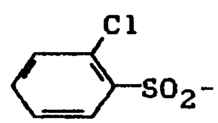 | H | $CH_3$ | N | CH | $C-CH_3$ | 340 |
| IA-213 | CH | 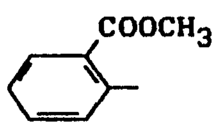 | H | H | $CH_3$ | N | CH | $C-CH_3$ | 191 |
| IC-214 | N | 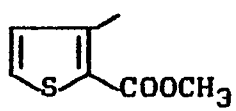 | H | H | $CH_3$ | N | CH | $C-CH_3$ | 217 |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-215 | N | 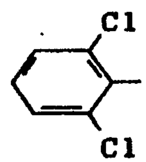 | 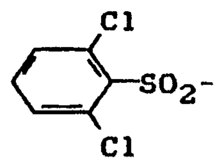 | H | $CH_3$ | N | CH | $C-CH_3$ | 195 |
| IA-216 | N | 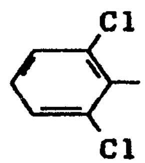 | H | H | $CH_3$ | N | CH | $C-CH_3$ | 240 |
| IA-217 | N | 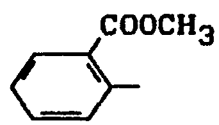 | H | H | $CH_3$ | N | CH | $C-CH_3$ | 152 |
| IC-218 | N | 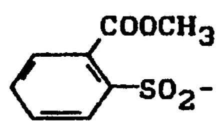 | H | H | $OCH_3$ | N | CH | $C-OCH_3$ | 213 |
| IC-219 | N | 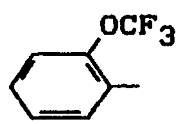 | H | H | $OCH_3$ | N | CH | $C-OCH_3$ | 225 |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IC-220 | N | 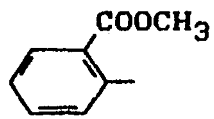 | H | H | $OCH_3$ | N | CH | $C-OCH_3$ | 210 |
| IC-221 | N | 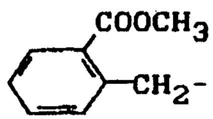 | H | $CH_3$ | $OCH_3$ | N | CH | $C-OCH_3$ | 213 |
| IC-222 | N | 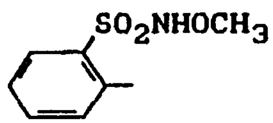 | H | H | $OCH_3$ | N | CH | $C-OCH_3$ | 215 |
| IA-223 | N | 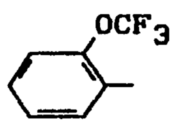 | H | H | $CH_3$ | N | N | $C-OCH_3$ | 212 |
| IC-224 | N | 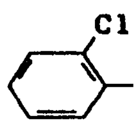 | H | H | $CH_3$ | N | CH | $C-OCH_3$ | 139 |
| IA-225 | N | 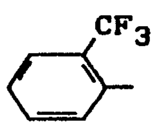 | 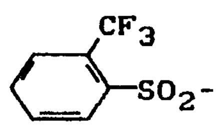 | H | $OC_2H_5$ | N | N | $C-OC_2H_5$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-226 | N | 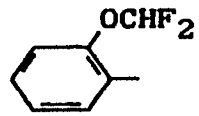 | 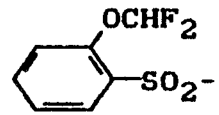 | H | $OC_2H_5$ | N | N | $C-OC_2H_5$ | 144 |
| IA-227 | N | 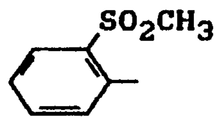 | H | H | $OC_2H_5$ | N | N | $C-OC_2H_5$ | 192 |
| IA-228 | N | 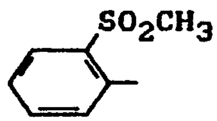 | 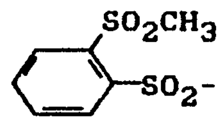 | H | $OC_2H_5$ | N | N | $C-OC_2H_5$ | 227 |
| IA-229 | N | 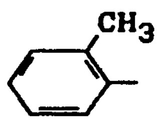 | 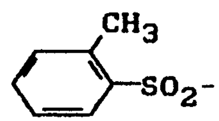 | H | $OC_2H_5$ | N | N | $C-OC_2H_5$ | 205 |
| IA-230 | N | 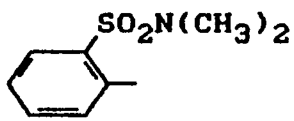 | 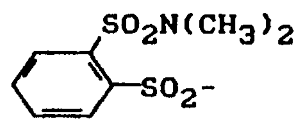 | H | $OC_2H_5$ | N | N | $C-OC_2H_5$ | 234 |
| IA-231 | N | 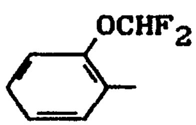 | H | H | $OC_2H_5$ | N | N | $C-OC_2H_5$ | 162 |

<u>Tabelle 6</u> - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-232 | N | 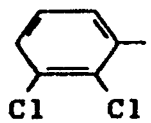 | 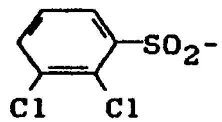 | H | $OC_2H_5$ | N | N | $C-OC_2H_5$ | 203 |
| IA-233 | N | 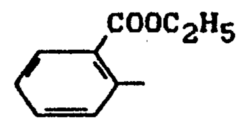 | 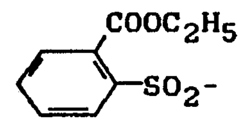 | H | $OC_2H_5$ | N | N | $C-OC_2H_5$ | 72 |
| IA-234 | N | 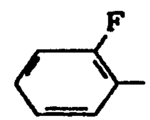 | 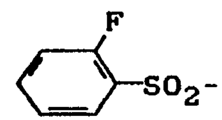 | H | $OCH_3$ | N | N | $C-OCH_3$ | 228 |
| IA-235 | N | 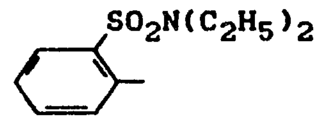 | 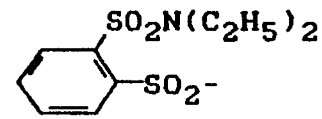 | H | $OC_2H_5$ | N | N | $C-OC_2H_5$ | 180 |
| IA-236 | N | 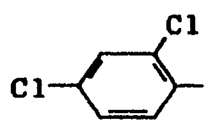 | 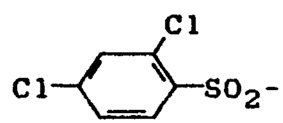 | H | $OC_2H_5$ | N | N | $C-OC_2H_5$ | 170 |
| IA-237 | N | 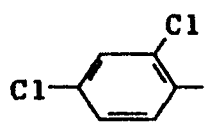 | H | H | $OC_2H_5$ | N | N | $C-OC_2H_5$ | 215 |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-238 | N | 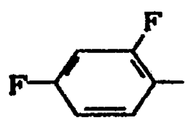 | 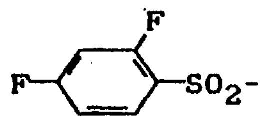 | H | $OC_2H_5$ | N | N | $C-OC_2H_5$ | 188 |
| IA-239 | N | 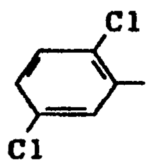 | H | H | $OC_2H_5$ | N | N | $C-OC_2H_5$ | 210 |
| IA-240 | N | 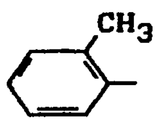 | 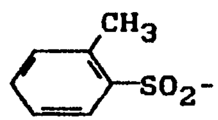 | H | $OCH_3$ | N | N | $C-OCH_3$ | 219 |
| IA-241 | CH | 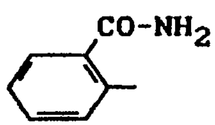 | H | H | $CH_3$ | N | CH | $C-CH_3$ | 231 |
| IA-242 | N | 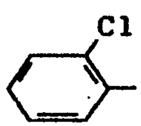 | H | H | $OC_2H_5$ | N | N | $C-OC_2H_5$ | 186 |

Tabelle 6 - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IC-243 | N | 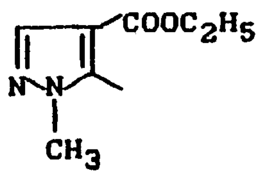 | H | H | $CH_3$ | N | CH | $C-OCH_3$ | 190 |
| IA-244 | N | 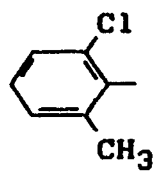 | H | H | $OCH_3$ | N | CH | $C-OCH_3$ | 275 |
| IA-245 | N | 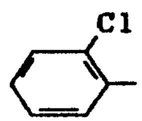 | H | H | $OC_2H_5$ | N | N | $C-OC_2H_5$ | |
| IA-246 | N | 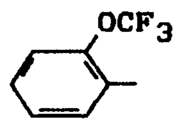 | H | H | $OC_2H_5$ | N | N | $C-OC_2H_5$ | |
| IA-247 | N | 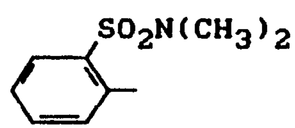 | H | H | $OC_2H_5$ | N | N | $C-OC_2H_5$ | |

**Tabelle 6** - Fortsetzung

| Bsp.-Nr. | A | $R^1$ | $R^2$ | $R^3$ | $R^4$ | X | Y | Z | Schmelzpunkt (°C) |
|---|---|---|---|---|---|---|---|---|---|
| IA-248 | N | 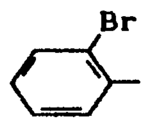 | 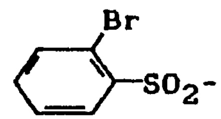 | H | $OCH_3$ | N | N | $C-OC_2H_5$ | |
| IA-249 | N | 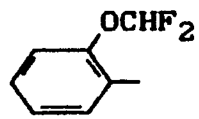 | 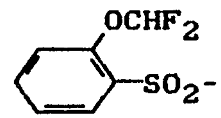 | H | $OCH_3$ | N | N | $C-OC_2H_5$ | |
| IA-250 | N | 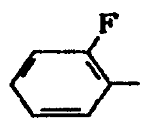 | H | H | $OC_2H_5$ | N | N | $C-OC_2H_5$ | |
| IA-251 | N | 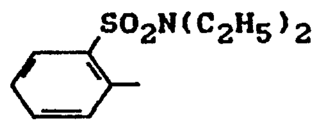 | H | H | $OC_2H_5$ | N | N | $C-OC_2H_5$ | |
| IA-252 | N | 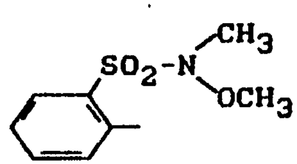 | H | H | $CH_3$ | N | CH | $C-CH_3$ | 192 |

Nachstehend wird die Herstellung einiger weiterer erfindungsgemäßer Verbindungen exemplarisch beschrieben.

Herstellung der in Tabelle 6 als Beispiel (IC-224) aufgeführten Verbindung:

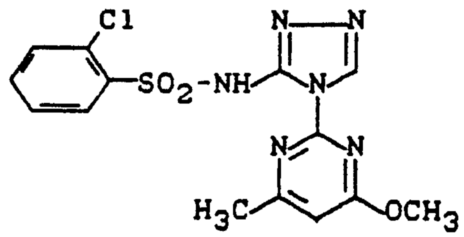

(IC-224)

(Verfahren (b))

3,0 g (0,025 Mol) Dimethylformamid-dimethylacetal werden zu einer Lösung von 9,3 g ((0,025 Mol) $N'$-(4-Methyl-6-methoxy-pyrimidin-2-yl)-$N''$-amino-$N'''$-(2-chlor-phenylsulfonyl)-guanidin in 100 ml Methanol gegeben und das Reaktionsgemisch wird 2 Stunden bei 20 °C gerührt. Das hierbei kristallin angefallene Produkt wird durch Absaugen isoliert.

Man erhält 8,0 g (84 % der Theorie) 4-(4-Methyl-6-methoxy-pyrimidin-2-yl)-3-(2-chlor-phenylsulfonylamino)-triazol vom Schmelzpunkt 139 °C.

Herstellung der in Tabelle 6 als Beispiel (IA-223) aufgeführten Verbindung:

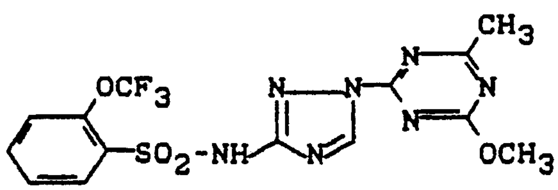

(IA-223)

(Verfahren (b))

2,7 g (0,023 Mol) Dimethylformamid-dimethylacetal werden zu einer Lösung von 6,3 g (0,015 Mol) $N'$-(4-Methyl-6-methoxy-s-triazin-2-yl-amino)-$N'$-(2-trifluormethoxy-phenylsulfonyl)-guanidin in 20 ml Methanol gegeben und das Reaktionsgemisch wird 30 Minuten unter Rückfluß zum Sieden erhitzt. Nach Abkühlen auf Raumtemperatur (ca. 20 °C) wird durch Absaugen isoliert und aus Methanol umkristallisiert.

Man erhält 4,5 g (70 % der Theorie) 1-(4-Methyl-6-methoxy-s-triazin-2-yl)-3-(2-trifluormethoxy-phenylsulfonylamino)-triazol vom Schmelzpunkt 212 °C.

Herstellung der in Tabelle 6 als Beispiel (IA-209) aufgeführten Verbindung:

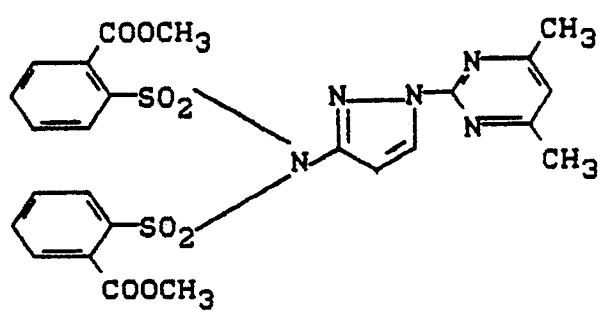

Verfahren (c))

20,2 g (0,18 Mol) 1,4-Diazabicyclo-[2,2,2]-octan (DABCO) werden unter Rühren zu einer auf 0 °C abgekühlten Mischung aus 5,73 g (0,03 Mol) 1-(4,6-Dimethyl-pyrimidin-2-yl)-3-amino-2-pyrazolin, 31,3 g (0,12 Mol) 2-Methoxycarbonyl-benzolsulfonsäurechlorid und 200 ml Methylenchlorid gegeben und das Reaktionsgemisch wird 18 Stunden, unter Zutritt von Luftsauerstoff bei Normaldruck, bei 20 °C gerührt. Dann wäscht man die Reaktionslösung mit Wasser unt trocknet sie mit Natriumsulfat. Nach Filtration wird eingeengt und der Rückstand mit Essigsäureethylester zur Kristallisation gebracht. Das kristalline Produkt wird durch Absaugen isoliert.

Man erhält 5,9 g (33 % der Theorie) 1-(4,6-Dimethylpyrimidin-2-yl)-3-(N,N-bis-(2-methoxycarbonyl-phenylsulfonyl)-amino)-pyrazol vom Schmelzpunkt 177 °C.

Herstellung der in Tabelle 6 als Beispiel (IA-242) aufgeführten Verbindung:

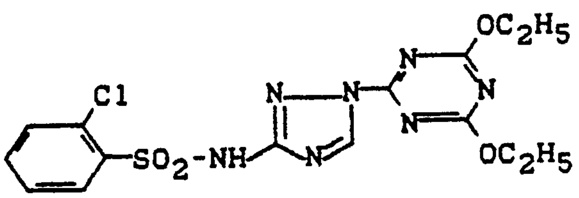

. (IA-242)

Verfahren (a))

2,93 g (0,022 Mol) Aluminiumchlorid werden bei Raumtemperatur zu einer Mischung aus 5,02 g (0,02 Mol) 1-(4,6-Diethoxy-s-triazin-2-yl)-3-amino-triazol und 60 ml Pyridin gegeben. Dabei kommt es zu einer exothermen Reaktion und die Temperatur steigt auf über 40 °C. Nach Kühlen auf 10 °C bis 20 °C werden unter Rühren 4,2 g (0,02 Mol) 2-Chlor-benzolsulfonsäurechlorid dazu gegeben und das Reaktionsgemisch wird anschließend 4 Stunden bei 50 °C gerührt. Nach weiteren 12 Stunden Rühren bei 20 °C wird das Gemisch in 480 ml 3N-Salzsäure eingerührt, 30 Minuten gerührt, abgesaugt und mit Wasser gewaschen. Das Rohprodukt wird aus Ethanol umkristallisiert.

Man erhält 6,1 g (72 % der Theorie) 1-(4,6-Diethoxy-s-triazin-2-yl)-3-(2-chlor-phenylsulfonylamino)-triazol vom Schmelzpunkt 186 °C.

Herstellung des Dimethylammoniumsalzes der in Tabelle 6 als Beispiel (IA-130) aufgeführten Verbindung:

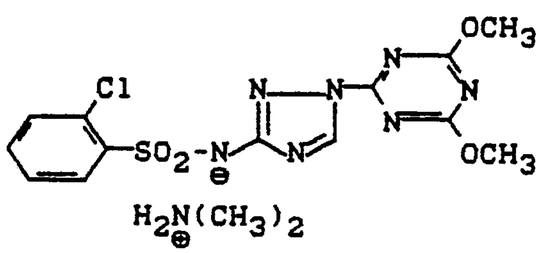

(IA-130)

Eine Mischung aus 8,0 g (0,02 Mol) N′-(4,6-Dimethoxy-s-triazin-2-yl-amino)-N″-(2-chlor-phenylsulfonyl)-guanidin, 4,5 g (0,0375 Mol) Dimethylformamid-dimethylacetal und 30 ml Methanol wird 10 Minuten bei 60 °C gerührt. Nach einer weiteren Stunde Rühren bei 20 °C wird das kristallin angefallene Produkt durch Absaugen isoliert.

Man erhält 4,6 g (52 % der Theorie) 1-(4,6-Dimethoxy-s-triazin-2-yl)-3-(2-chlor-phenylsulfonylamino)-triazol-Dimethylammoniumsalz vom Schmelzpunkt 145 °C.

Ausgangsstoffe der Formel (II)

Beispiel (II-1)

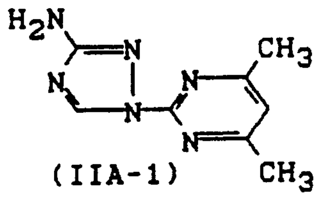

(IIA-1)

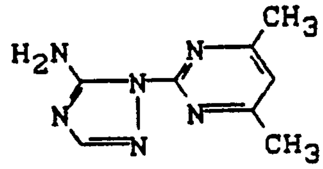

(IIB-1)

Eine Mischung aus 37,2 g (0,2 Mol) 2-Methylsulfonyl-4,6-dimethyl-pyrimidin, 33,6 g (0,4 Mol) 3-Amino-1,2,4-triazol, 27,6 g (0,2 Mol) Kaliumcarbonat und 300 ml Acetonitril wird 3 Stunden unter Rückfluß zum Sieden erhitzt. Dann wird im Wasserstrahlvakuum eingeengt, der Rückstand mit 300 ml Wasser verrührt, mit Salzsäure pH 7 eingestellt und das kristalline Produkt durch Absaugen isoliert. Das so erhaltene Rohprodukt wird durch Chromatographie über eine Kieselgelsäule mit Methylenchlorid/Methanol (Vol. 9 : 1) als Laufmittel gereinigt. Von den zwei erhaltenen Eluatfraktionen wird jeweils das Lösungsmittel im Wasserstrahlvakuum sorgfältig abgezogen.

Man erhält als erste Fraktion 10,2 g (27 % der Theorie) 3-Amino-1-(4,6-dimethyl-pyrimidin-2-yl)-1,2,4-triazol (IIA-1) vom Schmelzpunkt 235 °C - 238 °C.

Als zweite Fraktion erhält man 9,8 g (26 % der Theorie) 5-Amino-1-(4,6-dimethyl-pyrimidin-2-yl)-1,2,4-triazol (IIB-1) vom Schmelzpunkt 230 °C - 232 °C.

Beispiel (II-2)

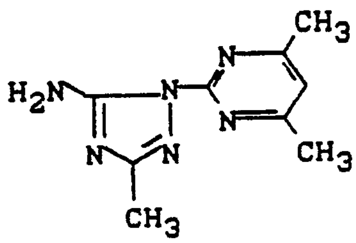

(IIB-2)

Eine Mischung aus 11,2 g (0,06 Mol) 4,6-Dimethyl-2-methylsulfonyl-pyrimidin, 5,9 g (0,06 Mol) 3-Amino-5-methyl-1,2,4-triazol, 8,3 g (0,06 Mol) Kaliumcarbonat und 100 ml Acetonitril wird 5 Stunden unter Rückfluß zum Sieden erhitzt. Nach Einengen wird der Rückstand in 100 ml Wasser eingerührt, das hierbei kristallin angefallene Rohprodukt durch Absaugen isoliert, mit Wasser gewaschen und aus Acetonitril umkristallisiert.

Man erhält 4,6 g (37 % der Theorie) 1-(4,6-Dimethyl-pyrimidin-2-yl)-5-amino-3-methyl-1,2,4-triazol vom Schmelzpunkt 237 °C.

Beispiel (II-3)

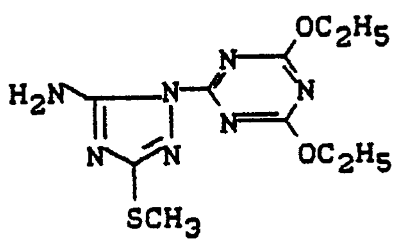

(IIB-3)

Eine Mischung aus 60 g (0,3 Mol) 4,6-Diethoxy-2-hydrazino-s-triazin, 44 g (0,3 Mol) Cyanimino-dithiokohlensäure-S,S-dimethylester und 300 ml Ethanol wird 12 Stunden unter Rückfluß zum Sieden erhitzt. Nach Abkühlen auf Raumtemperatur (ca. 20 °C) wird das kristallin angefallene Produkt durch

Absaugen isoliert.

Man erhält 73,8 g (83 % der Theorie) 1-(4,6-Diethoxy-s-triazin-2-yl)-5-amino-3-methylthio-1,2,4-triazol vom Schmelzpunkt 201 °C.

Beispiel (II-4)

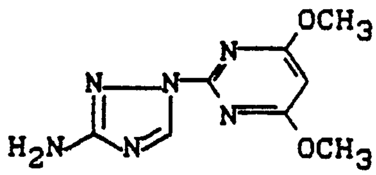

(IIA-4)

Eine Mischung aus 22,4 g (0,09 Mol) (4,6-Dimethoxy-pyrimidin-2-yl-amino)-guanidin-Hydrochlorid, 12,6 g (0,11 Mol) Dimethylformamid-dimethylacetal und 150 ml Acetonitril wird 5 Stunden unter Rückfluß zum Sieden erhitzt. Nach Abkühlen auf -10 °C wird das kristallin angefallene Produkt durch Absaugen isoliert.

Man erhält 10,9 g (54 % der Theorie) 1-(4,6-Dimethoxy-pyrimidin-2-yl)-3-amino-1,2,4-triazol vom Schmelzpunkt 219 °C.

Analog zu den Beispielen (II-1) bis (II-4) können die in der nachstehenden Tabelle 7 aufgeführten Verbindungen der Formel (II) - welche durch die Formeln (IIA), (IIB) und (IIC) genauer spezifiziert ist - hergestellt werden:

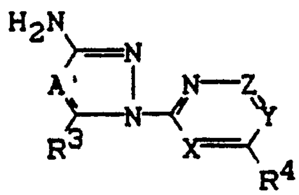

(IIA)

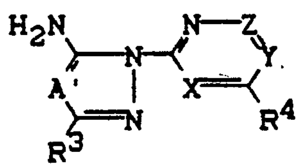

(IIB)

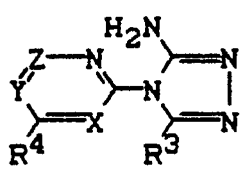

(IIC)

(In der folgenden Tabelle 7 wird an Stelle von A′ jeweils A angegeben).

Tabelle 7

| Beispiele für die Verbindungen der Formel (II) | | | | | | | |
|---|---|---|---|---|---|---|---|
| Bsp.-Nr. | A | $R^3$ | $R^4$ | X | Y | Z | Schmelzpunkt (°C) |
| IIB-5 | N | $CH_3$ | H | N | CH | CH | 223 |
| IIA-6 | N | $CH_3$ | $CH_3$ | N | CH | $C-CH_3$ | 211 |
| IIB-7 | N | $SCH_3$ | $OCH_3$ | N | CH | $C-OCH_3$ | 204 |
| IIB-8 | N | $SCH_3$ | $CH_3$ | N | CH | $C-OCH_3$ | 220 |
| IIB-9 | N | $SCH_3$ | $OCH_3$ | N | N | $C-OC_2H_5$ | 188 |
| IIB-10 | N | $SCH_3$ | $OCH_3$ | N | N | $C-OCH_3$ | 198 |
| IIA-11 | N | H | $OCH_3$ | N | N | $C-OCH_3$ | 233 |
| IIA-12 | N | H | $OC_2H_5$ | N | N | $C-OC_2H_5$ | 226 |
| IIA-13 | N | H | $OCH_3$ | N | N | $C-OC_2H_5$ | 211 |
| IIA-14 | N | H | H | N | H | C-H | 206 |
| IIB-15 | N | $SCH_3$ | H | N | CH | CH | 235 |
| IIB-16 | N | $SO_2CH_3$ | H | N | CH | CH | 276 |
| IIA-17 | N | H | $CH_3$ | N | CH | $C-OCH_3$ | 229 |

Ausgangsstoffe der Formeln (IVA) und (IVB)

Beispiel (IV-1)

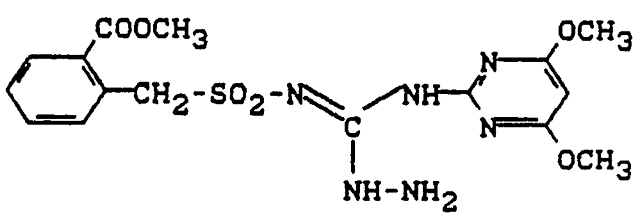

(IVA-1)

1,3 g (0,025 Mol) Hydrazinhydrat werden bei anfangs 20 °C zu einer Suspension von 15,9 g (0,025 Mol) N'-(4,6-Dimethoxy-pyrimidin-2-yl)-N''-methoxy-N''-(2-methoxycarbonyl-phenylsulfonyl)-N'''-(2-methoxycarbonyl-benzylsulfonyl)-guanidin in 100 ml Methanol unter Rühren gegeben, wobei sich die Reaktionsmischung auf 30 °C erwärmt und eine klare Lösung entsteht. Das nach vierstündigem Rühren bei 20 °C bis 30 °C kristallin abgeschiedene Produkt wird durch Absaugen isoliert.

Man erhält 9,5 g (89 % der Theorie) N'-(4,6-Dimethoxy-pyrimidin-2-yl)-N''-amino-N'''-(2-methoxycarbonyl-benzylsulfonyl)-guanidin vom Schmelzpunkt 166 °C.

Beispiel (IV-2)

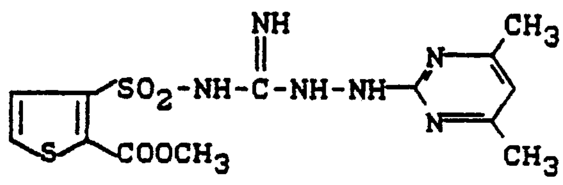

(IVB-2)

Eine Lösung von 3,8 g (0,01 Mol) N'-(4,6-Dimethyl-pyrimidin-2-yl)-N''-amino-N'''-(2-methoxycarbonyl-thien-3-yl- sulfonyl)-guanidin in 100 ml Ethanol wird 15 Stunden unter Rückfluß zum Sieden erhitzt. Nach

Abkühlen des Reaktionsgemisches auf 0 °C wird das kristallin angefallene Produkt durch Absaugen isoliert.

Man erhält 2,2 g (58 % der Theorie) N′-(4,6-Dimethyl-pyrimidin-2-yl-amino)-N″-(2-methoxy-carbonyl-thien-3-yl-sulfonyl)-guanidin vom Schmelzpunkt 213 °C.

Beispiel (IV-3)

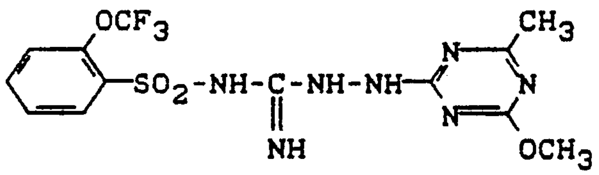

(IVB-3)

Eine Mischung aus 8,8 g (0,02 Mol) N′-(4-Methyl-6-methoxy-s-triazin-2-yl)-N″-(2-trifluormethoxy-phenylsulfonyl)-S-methyl-isothioharnstoff, 1,0 g (0,02 Mol) Hydrazinhydrat und 100 ml Ethanol wird 60 Minuten bei 20 °C gerührt. Das hierbei kristallin angefallene Produkt wird durch Absaugen isoliert.

Man erhält 6,4 g (76 % der Theorie) (N′-(4-Methyl-6-methoxy-s-triazin-2-yl-amino)-N″-(2-trifluormethoxy-phenylsulfonyl)-guanidin vom Schmelzpunkt 245 °C.

Analog zu den Beispielen (IV-1) bis (IV-3) können die in der nachstehenden Tabelle 8 aufgeführten Verbindungen der Formeln (IVA) und (IVB) hergestellt werden:

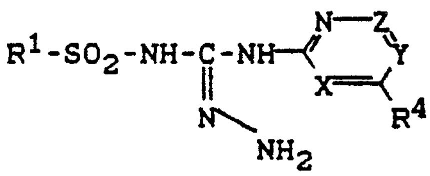

(IVA)

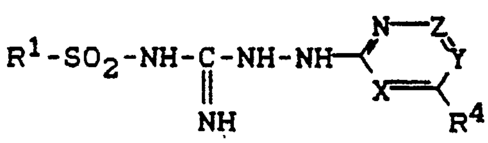

(IVB)

**Tabelle 8: Beispiele für die Verbindungen der Formeln (IVA) und (IVB)**

| Bsp.-Nr. | $R^1$ | $R^4$ | X | Y | Z | Schmelzpunkt (°C) |
|---|---|---|---|---|---|---|
| IVA-4 | 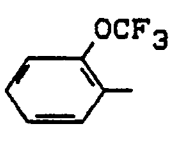 | $OCH_3$ | N | CH | C-$OCH_3$ | 134 |
| IVA-5 | 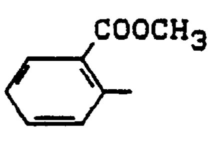 | $OCH_3$ | N | CH | C-$OCH_3$ | 159 |
| IVA-6 | 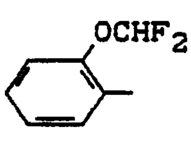 | $CH_3$ | N | CH | C-$CH_3$ | 98 |

Tabelle 8 - Fortsetzung

| Bsp.-Nr. | $R^1$ | $R^4$ | X | Y | Z | Schmelzpunkt (°C) |
|---|---|---|---|---|---|---|
| IVA-7 | 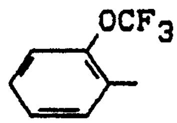 | $CH_3$ | N | N | C-$OCH_3$ | amorph |
| IVA-8 | 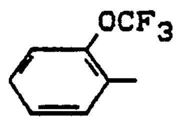 | $OCH_3$ | N | N | C-$OCH_3$ | amorph |
| IVA-9 | 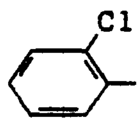 | $CH_3$ | N | CH | C-$OCH_3$ | 166 |
| IVA-10 | 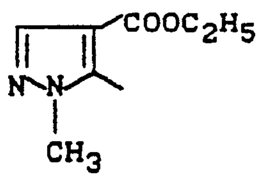 | $CH_3$ | N | CH | C-$CH_3$ | 105 |
| IVA-11 | 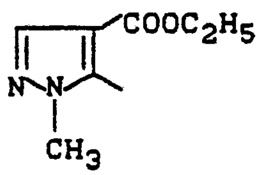 | $CH_3$ | N | N | C-$CH_3$ | 102 |
| IVB-12 | 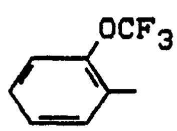 | $OCH_3$ | N | N | C-$OCH_3$ | 222 |
| IVB-13 | 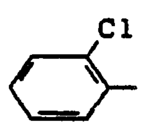 | $OCH_3$ | N | N | C-$OCH_3$ | 138 |
| IVB-14 | 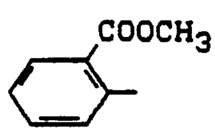 | $CH_3$ | N | N | C-$OCH_3$ | 230 |

<u>Tabelle 8</u> - Fortsetzung

| Bsp.-Nr. | $R^1$ | $R^4$ | X | Y | Z | Schmelzpunkt (°C) |
|---|---|---|---|---|---|---|
| IVB-15 | 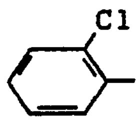 | $CH_3$ | N | N | $C-OCH_3$ | 208 |
| IVB-16 | 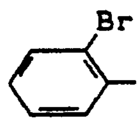 | $CH_3$ | N | N | $C-OCH_3$ | 195 |
| IVB-17 | 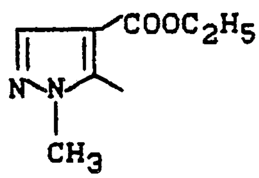 | $CH_3$ | N | CH | $C-OCH_3$ | 262 |
| IVB-18 | 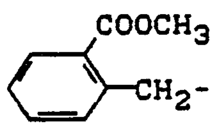 | $OCH_3$ | N | CH | $C-OCH_3$ | 195 |
| IVB-19 | 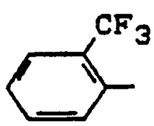 | $OCH_3$ | N | N | $C-OCH_3$ | 215 |
| IVB-20 | 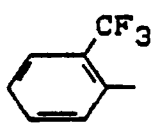 | $CH_3$ | N | N | $C-SCH_3$ | 202 |
| IVB-21 | 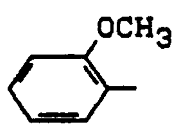 | $CH_3$ | N | N | $C-OCH_3$ | 235 |
| IVB-22 | 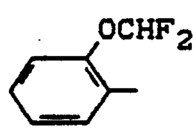 | $OCH_3$ | N | N | $C-OCH_3$ | 199 |

<u>Ausgangsstoffe der Formel (VI)</u>

<u>Beispiel (VI-1)</u>

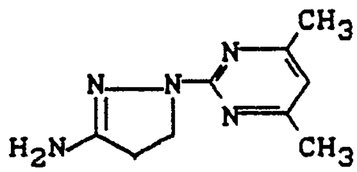

Eine aus 0,6 g (0,026 Mol) Natrium und 30 ml Ethanol erzeugte Lösung und 5,3 g (0,1 Mol) Acrylnitril werden nacheinander zu einer Mischung aus 13,8 g (0,1 Mol) 4,6-Dimethyl-2-hydrazino-pyrimidin, 27 g (0,1 Mol) Natriummethanolat und 80 ml Methanol gegeben. Das Reaktionsgemisch wird 18 Stunden unter Rückfluß zum Sieden erhitzt und anschließend eingeengt. Der Rückstand wird in 200 ml Wasser aufgenommen und diese Lösung wird mit Salzsäure auf pH 6 eingestellt. Dann gibt man 20 g Natriumchlorid dazu und kühlt auf 10 °C ab. Das hierbei kristallin angefallene Produkt wird durch Absaugen isoliert.

Man erhält 10,8 g (57 % der Theorie) 1-(4,6-Dimethyl-pyrimidin-2-yl)-3-amino-2-pyrazolin vom Schmelzpunkt 205 °C.

Ausgangsstoffe der Formel (IX)

Beispiel (IX-1)

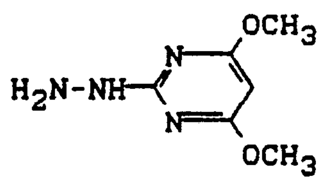

Eine Mischung aus 32,7 g (0,15 Mol) 4,6-Dimethoxy-2-methylsulfonyl-pyrimidin, 7,3 g (0,15 Mol) Hydrazinhydrat, 21 g (0,15 Mol) Triethylamin und 200 ml Ethanol wird 24 Stunden bei 25 °C gerührt. Nach Zugabe von weiteren 3,7 ml (0,076 Mol) Hydrazinhydrat wird das Reaktionsgemisch weitere 24 Stunden bei 25 °C gerührt und dann mit 200 ml Petrolether verdünnt. Das kristallin angefallene Produkt wird durch Absaugen isoliert.

Man erhält 18,9 g (74 % der Theorie) (4,6-Dimethoxy-2-hydrazino-pyrimidin vom Schmelzpunkt 94 °C.

Ausgangsstoffe der Formel (XIB)

Beispiel (XI-1)

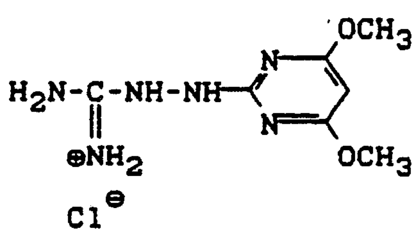

(XIB-1)

Eine Mischung aus 17,0 g (0,1 Mol) 4,6-Dimethoxy-2-hydrazino-pyrimidin, 8,3 ml konz. Salzsäure, 4,2 g (0,1 Mol) Cyanamid und 100 ml Ethanol wird 18 Stunden unter Rückfluß zum Sieden erhitzt. Dann wird eingeengt, der Rückstand mit Diethylether zur Kristallisation gebracht und das kristalline Produkt durch Absaugen isoliert.

Man erhält 22,9 g (92 % der Theorie) (4,6-Dimethoxy-pyrimidin-2-yl-amino)-guanidin-Hydrochlorid vom

Schmelzpunkt 214 °C.

Analog Beispiel (XI-1) können die in der nachstehenden Tabelle 9 aufgeführten Verbindungen der Formel (XIB) hergestellt werden:

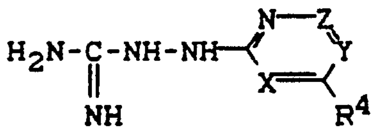

(XIB)

**Tabelle 9: Beispiele für die Verbindungen der Formel (XIB)**

| Bsp.-Nr. | $R^4$ | X | Y | Z | Schmelzpunkt (°C) |
|---|---|---|---|---|---|
| XIB-2 | $CH_3$ | N | CH | $C-CH_3$ | 218 |
| XIB-3 | $OCH_3$ | N | CH | C-Cl | 237 |
| XIB-4 | $OC_2H_5$ | N | N | $C-OC_2H_5$ | 169 |
| XIB-5 | $OCH_3$ | N | N | $C-OCH_3$ | 235 |
| XIB-6 | $OCH_3$ | N | N | $C-OC_2H_5$ | 220 |

## Anwendungsbeispiele

### Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen (IA-2), (IA-3), (IA-6), (IA-8) und (IA-223). Dies gilt in besonderem Maße auch für die Verbindungen gemäß Herstellungsbeispielen (IA-25) und (IA-68).

### Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen (IA-1), (IA-2), (IA-5), (IA-6), (IA-8), (IA-52) und (IA-3). Dies gilt insbesondere auch für die Verbindungen gemäß Herstellungsbeispielen (IA-25) und (IA-68).

## Ansprüche

1. Substituierte Sulfonylaminoazole der allgemeinen Formel (I)

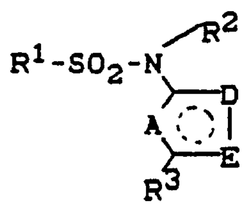

(I)

in welcher
$R^1$ für einen gegebenenfalls substituierten Rest aus der Reihe Alkyl, Aralkyl, Aryl und Heteroaryl steht,
$R^2$ für Wasserstoff, ein Metalläquivalent oder die Gruppierung $-SO_2-R^1$ steht, worin
$R^1$ die oben angegebene Bedeutung hat,
$R^3$ für Wasserstoff, Halogen, Amino oder einen gegebenenfalls substituierten Rest aus der Reihe Alkyl, Alkoxy, Alkylthio, Alkylamino und Dialkylamino steht,
A für eine CH-Gruppierung, für Stickstoff oder die Gruppierung

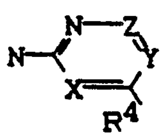

steht,
D für Stickstoff oder die Gruppierung

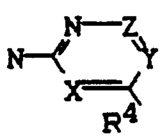

steht,
E für Stickstoff oder die Gruppierung

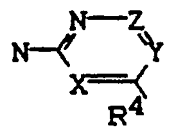

steht,
wobei jeweils immer einer der Reste A, D oder E für die Gruppierung

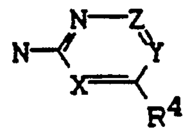

steht. worin
$R^4$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
X für Stickstoff oder eine CH-Gruppierung steht,
Y für Stickstoff oder eine $CR^5$-Gruppierung steht, worin
$R^5$ für Wasserstoff, Halogen, Cyano, Alkyl, Formyl, Alkylcarbonyl oder Alkoxycarbonyl steht, und
Z für Stickstoff oder eine $CR^6$-Gruppierung steht, worin
$R^6$ für Wasserstoff, Halogen, Hydroxy, Amino oder einen gegebenenfalls substituierten Rest aus der Reihe Alkyl, Alkoxy, Alkylthio, Alkylamino und Dialkylamino steht,
sowie Salze von Verbindungen der Formel (I).

2. Substituierte Sulfonylaminoazole gemäß Anspruch 1, entsprechend einer der folgenden Formeln (IA), (IB) bzw. (IC):

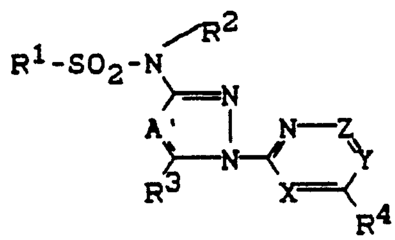

(IA)

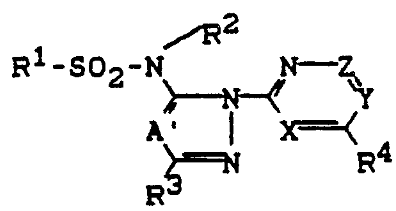

(IB)

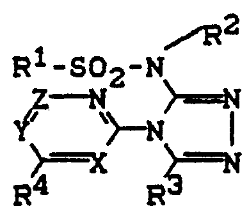

(IC)

in welchen
$R^1$, $R^2$, $R^3$, $R^4$, X, Y und Z die in Anspruch 1 angegebenen Bedeutungen haben und
A' für Stickstoff oder eine CH-Gruppierung steht.

3. Verfahren zur Herstellung von substituierten Sulfonylaminoazolen der allgemeinen Formel (I) gemäß Anspruch 1, dadurch gekennzeichnet, daß man

(a) substituierte Aminoazole der allgemeinen Formel (II)

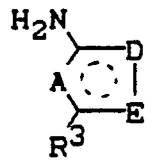

(II)

in welcher
A, D, E und $R^3$ die in Anspruch 1 angegebenen Bedeutungen haben,
mit Sulfonsäurehalogeniden oder Sulfonsäureanhydriden der allgemeinen Formel (III)

$R^1 - SO_2 - Q$ (III)

in welcher
$R^1$ die in Anspruch 1 angegebene Bedeutung hat und
Q für Fluor, Chlor, Brom oder die Gruppierung $-O-SO_2-R^1$ steht,
gegebenenfalls in Gegenwart eines Säureakzeptors, gegebenenfalls in Gegenwart eines Katalysators und gegebenenfalls in Gegenwart eines Verdünnungsmittels umsetzt,
gegebenenfalls die so erhältlichen Verbindungen der Formel (I), in welcher
$R^2$ für die Gruppierung $-SO_2-R^1$ steht,
mit Desulfonylierungsmitteln,
gegebenenfalls in Gegenwart von Verdünnungsmitteln, zu Verbindungen der Formel (I), in welcher
$R^2$ für Wasserstoff steht,
umsetzt, oder daß man

(b) sulfonylierte Aminoguanidine der allgemeinen Formeln (IVA) oder (IVB)

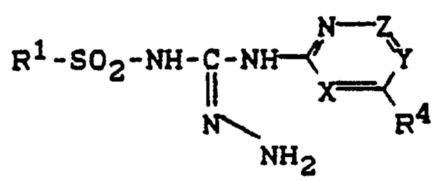

(IVA)

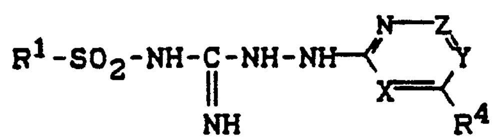

(IVB)

in welchen
$R^1$, $R^4$, X, Y und Z die in Anspruch 1 angegebenen Bedeutungen haben,
oder Tautomere zu den Verbindungen der Formeln (IVA) und (IVB) oder Gemische aus den Verbindungen der Formeln (IVA) bzw. (IVB) und den jeweils möglichen Tautomeren mit Ester(amide)n der allgemeinen Formel (V) (d.h. Orthoestern bzw. Amid-acetalen)

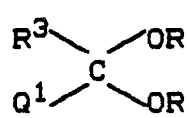

(V)

in welcher
$R^3$ die in Anspruch 1 angegebene Bedeutung hat,
R für Alkyl steht und
$Q^1$ für Alkoxy oder Dialkylamino steht,
gegebenenfalls in Gegenwart eines Verdünningsmittels umsetzt, oder daß man

(c) substituierte Aminopyrazoline der allgemeinen Formel (VI)

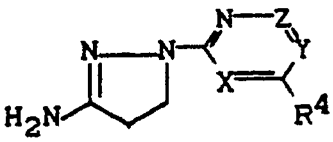

(VI)

in welcher
$R^4$, X, Y und Z die in Anspruch 1 angegebenen Bedeutungen haben,
mit Sulfonsäurehalogeniden oder Sulfonsäureanhydriden der allgemeinen Formel (III)
$R^1 - SO_2 - Q$ (III)
in welcher
$R^1$ die in Anspruch 1 angegebene Bedeutung hat und
Q für Fluor, Chlor, Brom oder die Gruppierung $-O-SO_2-R^1$ steht, worin
$R^1$ die oben angegebene Bedeutung hat,
in Genenwart von (Luft)Sauerstoff, gegebenenfalls in Gegenwart eines Säureakzeptors und gegebenenfalls in Gegenwart eines Verdünnungsmittels umsetzt, und
gegebenenfalls die so erhältlichen Verbindungen der Formel (I), in welcher
$R^2$ für die Gruppierung $-SO_2-R^1$ steht,
mit Desulfonylierungsmitteln, gegebenenfalls in Gegenwart von Verdünnungsmitteln, zu Verbindungen der Formel (I), in welcher
$R^2$ für Wasserstoff steht,
umsetzt und gegebenenfalls die nach den Verfahren (a), (b) oder (c) erhaltenen Produkte nach üblichen Methoden in Salze überführt.

4. Herbizide Mittel, gekennzeichnet durch einen Gehalt an mindestens einem substituierten Sulfonylaminoazol der Formel (I) gemäß Anspruch 1.

5. Verfahren zur Bekämpfung von Unkräutern, dadurch gekennzeichnet, daß man substituierte Sulfonylaminoazole der Formel (I) gemäß Anspruch 1 auf die Unkräuter und/oder ihren Lebensraum einwirken läßt.

6. Verwendung von substituierten Sulfonylaminoazolen der Formel (I) gemäß Anspruch 1 zur Bekämpfung von Unkräutern.

7. Verfahren zur Herstellung von herbiziden Mitteln, dadurch gekennzeichnet, daß man substituierte Sulfonylaminoazole der Formel (I) gemäß Anspruch 1 mit Streckmitteln und/oder oberflächenaktiven Mitteln vermischt.